# EUROPEAN PATENT APPLICATION

(11) **EP 4 273 139 A1**
(43) Date of publication of application: **08.11.2023**
(21) Application number: 21914310.4
(22) Date of filing: 28.12.2021
(51) Int. Cl.: C07D 401/12, C07D 401/10, C07D 213/69, A61K 31/53, A61P 5/14, A61P 3/04, A61P 9/06

(54) **2-PYRIDONE DERIVATIVE, AND PREPARATION METHOD THEREFOR AND PHARMACEUTICAL APPLICATION THEREOF**

(30) Priority: 30.12.2020 CN 202011609510
(71) Applicant: KPC Pharmaceuticals, Inc., Kunming, Yunnan 650106 (CN); Shanghai Kunheng Pharma-Tech Co., Ltd., Shanghai 201210 (CN)
(72) Inventor: LV, Hejun, Kunming, Yunnan 650106 (CN); WANG, Peng, Kunming, Yunnan 650106 (CN); GUO, Fei, Kunming, Yunnan 650106 (CN); CHEN, Shaoqing, Kunming, Yunnan 650106 (CN); LIU, Junfeng, Kunming, Yunnan 650106 (CN)
(74) Representative: V.O.
(86) International application number: PCT/CN2021/141829
(87) International publication number: WO 2022/143574

(57) **Abstract**

The present invention provides a 2-pyridone derivative having a structure represented by formula I, or a stereoisomer or pharmaceutically acceptable salt thereof. Activity experiment results show that the 2-pyridone derivative provided in the present invention has higher activity and selectivity, and can be used to treat thyroid hormone receptor-related diseases.

## Description

### CROSS REFERENCE TO RELATED APPLICATIONS

This application claims the priority of Chinese Patent Application No. 202011609510.0, filed with the China National Intellectual Property Administration on December 30, 2020, and titled with "2-PYRIDONE DERIVATIVE, AND PREPARATION METHOD THEREFOR AND PHARMACEUTICAL APPLICATION THEREOF", which is hereby incorporated by reference in its entirety.

### FIELD

The present invention relates to the field of pharmaceutical technology, in particular to a compound that can be used as a novel agonist of the THR β receptor and its preparation method and application, specifically, to a 2-pyridone derivative, its preparation method and its application in medicine.

### BACKGROUND

Thyroid hormones are essential for normal growth and development and for maintaining metabolic balance (Physiological Reviews 2001, 81(3), 1097-1126.). Thyroid hormones are produced by the thyroid and secreted into the circulatory system (hypothalamus/pituitary/thyroid system) in two different forms, T4 and T3, where T4 is the predominant form secreted by the thyroid, and T3 is the more physiologically active form. T4 is converted to T3 by tissue-specific deiodinases, which are present in all tissues but mainly in liver and kidney.

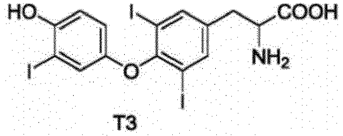

Circulating levels of thyroid hormones are tightly regulated by feedback mechanisms in the hypothalamus/pituitary/thyroid axis. Thyroid dysfunction leading to hypothyroidism or hyperthyroidism has profound impact on heart/weight/metabolism/metabolic rate/body temperature/cholesterol/bone/muscle and behavior.

The biological activity of thyroid hormones is mediated by the thyroid hormone receptor (THR) (Endocrine Reviews (1993) 14 348-399). THR, a member of the nuclear receptor family, is encoded by different genes expressing α and β located on human chromosomes 17 and 3. Selective splicing of primary transcripts generates different protein subtypes, and each gene generates two subtypes, namely THRα1, THRα2, THPβ1, and THPβ2. THRβ1 and THRβ2 are generated from differential expression of promoters, and the two subtypes only differ at the amino terminals. THRα1 and THRα2 arise from differential splicing of pre-mRNAs, and the two subtypes mainly differ at the carboxyl terminals. THRα1, THRβ1, and THRβ2 can bind thyroid hormones. THRβ is mainly distributed in the liver/kidney/pituitary gland and brain tissue, and plays an important role in regulating TRH and thyroid hormones behavior in the liver. THRα is widely distributed throughout the body, and is mainly associated with extrahepatic cardiovascular and skeletal/muscular adverse reactions (Drugs (2017) 77 1613-1621). Therefore, if a thyroid hormone analog can avoid the adverse effects of hyperthyroidism and hypothyroidism while maintaining the beneficial effects of thyroid hormone, it may be applied to the treatment of responsive diseases, such as metabolic diseases including obesity, hyperlipidemia, hypercholesterolemia, diabetes and other conditions such as hepatic steatosis and non-alcoholic steatohepatitis (NASH), atherosclerosis, cardiovascular disease, hypothyroidism, thyroid cancer and thyroid disease.

The therapeutic use of thyroid hormone itself is limited by adverse side effects associated with hyperthyroidism, especially cardiovascular toxicity. A series of thyroid hormone agonists have been developed in the prior art. These structural agonists are almost all designed and developed based on the structure of T3, a natural ligand of the THR receptor. For example, thyroid hormone analogues that are structurally different from the compounds of the present invention have been disclosed (Agricultural and Biol. Chem. 1974, 38(6), 1169; J. Med. Chem. 1989, 32, 320; J. Med. Chem. 2014, 57(10), 3912; WO2007009913; WO2010122980). Among them, Example 8 (compound 31) disclosed in WO2007009913 is MGL-3196, currently the first-in-class orally administered small molecule selective agonist of hepatic thyroid hormone receptor β subtype (THR-β) in clinical. Preclinical toxicology and data of phase I suggest that, as a potential treatment for non-alcoholic steatohepatitis (NASH) and dyslipidemia, MGL-3196 can significantly reduce LDL cholesterol, triglycerides and lipoproteins, causing it to be an ideal candidate for reducing cardiovascular risk in NASH patients and for patients with dyslipidemia who are on moderate statin doses or intolerant to statins. Data of phase II suggest that adverse effects (AEs) were predominantly mild (85 %) and moderate (15%), and there were 3 severe cases not related to treatment. Furthermore, in WO2009037172A1 and CN 110938094 A, MGL3196 was structurally modified, where the nitrogen atom in the pyridazine ring was substituted to form a prodrug of MGL3196 with no or low activity, which was metabolized in the body to form the original drug, so as to achieve technical effects of improving the absorption, distribution, transportation and metabolism of the drug in the body, improving bioavailability, improving the selectivity of the drug on the target site, reducing the toxic and side effects of the drug, prolonging the action time, and reducing the impact of food.

Afterwards, many documents such as WO2020073974, CN 111320609 A and WO2019240938 disclose a series of structural modifications are conducted in different directions on the pyridazinone ring based on the structure of MGL-3196, but the core structures thereof always have a pyridazinone structure similar to the structure of MGL-3196. WO2020169069 discloses similar structures of pyridine and pyridone, which result in reduced biological activity of THRβ and reduced selectivity of THRβ/THRα. In particular, when the nitrogen atom on pyridone is replaced, the pharmacological activity is almost lost.

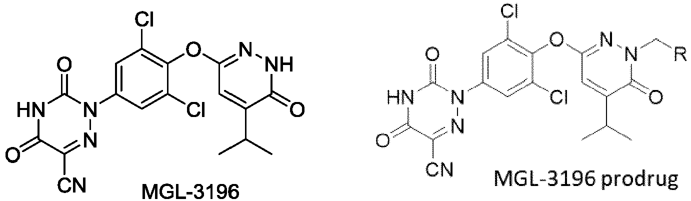

There are still problems in the effectiveness, safety or selectivity of the compounds and experimental drugs disclosed in the prior art. Therefore, it is necessary to continue to discover and develop highly active and highly selective new compounds that have the beneficial effects of thyroid hormones and can avoid adverse effects, for treating diseases associated with thyroid hormone receptors.

### SUMMARY

In view of this, the technical problem to be solved by the present invention is to provide a 2-pyridone derivative, a preparation method therefor and pharmaceutical use thereof, wherein the 2-pyridone derivative has high activity and selectivity.

In order to achieve the above purpose, the present invention provides a 2-pyridone derivative having a structure represented by formula I, or a stereoisomer or pharmaceutically acceptable salt thereof: 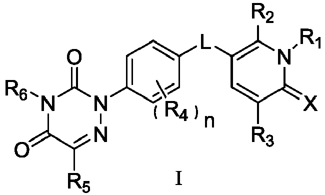
wherein, R₁ is selected from the group consisting of C₁₋₆ alkyl, saturated or unsaturated C₃₋₆ cycloalkyl, C₅₋₁₀ aryl and C₅₋₁₀ heteroaryl; wherein, the C₁₋₆ alkyl can be optionally substituted with one or more of halogen, deuterium, hydroxyl, alkoxy, oxo, amino, C₃₋₆ cycloalkyl, 5-10 membered aryl or 5-10 membered heteroaryl; the saturated or unsaturated C₃₋₆ cycloalkyl can be optionally substituted with one or more of halogen, deuterium, hydroxyl, alkoxy, oxo or amino; the C₅₋₁₀ aryl, C₅₋₁₀ heteroaryl, 5-10 membered aryl or 5-10 membered heteroaryl can be optionally substituted with one or more of halogen, substituted or unsubstituted C₁₋₆ alkyl, substituted or unsubstituted C₃₋₆ cycloalkyl, substituted or unsubstituted C₁₋₆ alkoxy, cyano, hydroxyl, or amino;
R₂ is selected from the group consisting of hydrogen and C₁₋₆ alkyl;
R₃ is selected from the group consisting of hydrogen, halogen, C₁₋₆ alkyl and C₃₋₆ cycloalkyl;
R₄ is independently selected from the group consisting of hydrogen, hydroxyl, C₁₋₆ alkyl, halogen, C₁₋₆ alkoxy, and C₃₋₆ cycloalkyl, or two adjacent R₄ form C₃₋₆ cycloalkyl, wherein the C₁₋₆ alkyl, C₃₋₆ cycloalkyl or C₁₋₆ alkoxy can be optionally further substituted with one or more of hydroxyl or halogen;
n is 1, 2 or 3;
R₅ is selected from the group consisting of hydrogen, cyano, carboxyl, C₁₋₆ alkyl, and C₃₋₆ cycloalkyl, wherein the C₁₋₆ alkyl or C₃₋₆ cycloalkyl can be optionally substituted with one or more of halogen, hydroxyl, or C₁₋₆ alkoxy;
R₆ is selected from the group consisting of hydrogen and C₁₋₆ alkyl;
L is selected from the group consisting of -CH₂-, -O-, -CF₂- and -S-;
X is selected from the group consisting of O and S;
or alternatively, R₁ and R₂ together with the atoms to which they are attached form a 4-10 membered heterocycloalkyl, wherein the heterocycloalkyl can further contains 0, 1 or 2 optional oxygen, sulfur and nitrogen atoms in addition to the original nitrogen atom connected to R₁, and the heterocycloalkyl can be optionally further substituted with one or more of C₁₋C₆ alkyl, hydroxyl, halogen or cycloalkyl;
when R₃ is C₁₋₆ alkyl and/or C₃₋C₆ cycloalkyl, R₁ is not C₁₋₆ alkyl, saturated or unsaturated C₃₋₆ cycloalkyl.

Preferably in the present invention, the C₅₋₁₀ aryl, C₅₋₁₀ heteroaryl, 5-10 membered aryl or 5-10 membered heteroaryl can be optionally substituted with one or more of halogen, substituted or unsubstituted C₁₋₆ alkyl, substituted or unsubstituted C₃₋₆ cycloalkyl, substituted or unsubstituted C₁₋₆ alkoxy, or cyano;
the C₁₋₆ alkyl, C₃₋₆ cycloalkyl, or C₁₋₆ alkoxy can be optionally substituted with one or more F atoms.

Preferably in the present invention, the substituted or unsubstituted C₁-C₆ alkyl, substituted or unsubstituted C₃-C₆ cycloalkyl, or substituted or unsubstituted C₁-C₆ alkoxy is preferably substituted with 1, 2 or 3 fluorine atoms.

Preferably in the present invention, R₁ is selected from the group consisting of methyl, ethyl, n-propyl, isopropyl, n-butyl, isobutyl, tert-butyl, n-pentyl, isopentyl, neopentyl, -CH(CH₂CH₃)₂, cyclopropyl, cyclobutyl, cyclopentyl, cyclohexyl, cyclopentenyl, cyclohexenyl, phenyl, thienyl, and thiazolyl.

Preferably, the methyl, ethyl, n-propyl, isopropyl, n-butyl, isobutyl, tert-butyl, n-pentyl, isopentyl, neopentyl, or -CH(CH₂CH₃) can be optionally substituted with one or more of halogen, deuterium, hydroxyl, C₁₋₆ alkoxy, oxo, amino, C₃₋₆ cycloalkyl, phenyl, naphthyl, pyridyl or pyrrolyl.

Preferably, the phenyl, naphthyl, pyridyl or pyrrolyl can be optionally further substituted with one or more of halogen, substituted or unsubstituted C₁₋₆ alkyl, substituted or unsubstituted C₃₋₆ cycloalkyl , substituted or unsubstituted C₁₋₆ alkoxy, cyano, hydroxyl, or amino.

The above alkoxy is preferably C₁₋₆ alkoxy.

The C₁₋₆ alkoxy in the present invention is preferably selected from the group consisting of methoxy, ethoxy, propoxy, isopropoxy, butoxy and pentyloxy.

The above C₃₋₆ cycloalkyl is preferably selected from the group consisting of cyclopropyl, cyclobutyl, cyclopentyl and cyclohexyl.

Preferably, the cyclopropyl, cyclobutyl, cyclopentyl, cyclohexyl, cyclopentenyl, or cyclohexenyl can be optionally substituted with one or more of halogen, deuterium, hydroxyl, C₁₋₆ alkoxy, oxo or amino.

The above C₁₋₆ alkoxy is preferably selected from the group consisting of methoxy, ethoxy, propoxy, isopropoxy, butoxy and pentyloxy.

The phenyl, thienyl, or thiazolyl can be optionally substituted with one or more of halogen, substituted or unsubstituted C₁₋₆ alkyl, substituted or unsubstituted C₃₋₆ cycloalkyl, substituted or unsubstituted C₁₋₆ alkoxy, cyano, hydroxyl, or amino.

The above C₁₋₆ alkyl is preferably selected from the group consisting of methyl, ethyl, propyl, isopropyl, butyl, isobutyl and tert-butyl.

The above C₁₋₆ alkoxy is preferably selected from the group consisting of methoxy, ethoxy, propoxy, isopropoxy, butoxy and pentyloxy.

The above C₃₋₆ cycloalkyl is preferably selected from the group consisting of cyclopropyl, cyclobutyl, cyclopentyl and cyclohexyl.

The above C₁₋₆ alkyl, C₁₋₆ alkoxy, or C₃₋₆ cycloalkyl can be optionally further substituted with 1-3 fluorine atoms.

Or alternatively, R₁ and R₂ together with the atoms to which they are attached form a 4-10 membered heterocycloalkyl, preferably a five-membered or six-membered monocyclic heterocyclyl, more preferably a tetrahydropyrrolyl.

The heterocycloalkyl can optionally further contains 0, 1 or 2 oxygen, sulfur and nitrogen atoms in addition to the original nitrogen atom connected to R₁, and the heterocycloalkyl can be optionally further substituted with one or more of C₁-C₆ alkyl, hydroxyl, halogen or cycloalkyl.

More preferably, the heterocycloalkyl is substituted with one or more of C₁₋₃ alkyl, halogen or C₃₋₆ cycloalkyl.

Further preferably, the heterocycloalkyl is substituted with one or more of fluorine, chlorine, bromine, methyl, ethyl, propyl, isopropyl, cyclopropyl, cyclobutyl, cyclopentyl, or cyclohexyl.

Preferably in the present invention, R₂ is selected from the group consisting of hydrogen, methyl, ethyl, n-propyl, isopropyl, n-butyl, isobutyl, tert-butyl, n-pentyl, isopentyl, neopentyl and -CH(CH₂CH₃)₂. More preferably, R₂ is hydrogen.

Preferably in the present invention, R₃ is selected from the group consisting of hydrogen, fluorine, chlorine, bromine, iodine, methyl, ethyl, n-propyl, isopropyl, n-butyl, isobutyl, tert-butyl, n-pentyl, isopentyl, neopentyl, -CH(CH₂CH₃)₂, cyclopropyl, cyclobutyl, cyclopentyl and cyclohexyl. More preferably, R₃ is selected from the group consisting of hydrogen, fluorine, chlorine, methyl and cyclopropyl.

Preferably in the present invention, R₄ is selected from the group consisting of fluorine, chlorine, bromine and iodine.

More preferably, R₄ is ortho-dihalo at the L position. Specifically, it is selected from the group consisting of ortho-difluoro, ortho-dichloro and ortho-dibromo at the L position.

Further preferably, R₄ is chlorine, which is substituted on the ortho positions of the L position, i.e., 2,6-disubstitution, as shown in the following structure (taking L being O as an example):

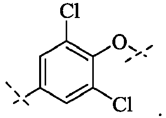

Preferably in the present invention, R₅ is selected from the group consisting of cyano and C₁₋₆ alkyl.

The C₁₋₆ alkyl can be optionally substituted with one or more of halogen, hydroxyl or amino.

Further preferably, R₅ is selected from the group consisting of hydrogen, cyano, methyl, ethyl, n-propyl, isopropyl, n-butyl, isobutyl, tert-butyl, n-pentyl, isopentyl, neopentyl and -CH(CH₂CH₃)₂.

Preferably, the methyl, ethyl, n-propyl, isopropyl, n-butyl, isobutyl, tert-butyl, n-pentyl, isopentyl, neopentyl or -CH(CH₂CH₃)₂ can be optionally substituted with 1 to 3 fluorine atoms.

In some specific embodiments of the present invention, R₅ is selected from the group consisting of cyano, methyl, monofluoromethyl, difluoromethyl and trifluoromethyl.

Preferably in the present invention, R₆ is selected from the group consisting of hydrogen, methyl, ethyl, n-propyl, isopropyl, n-butyl, isobutyl, tert-butyl, n-pentyl, isopentyl, neopentyl and -CH(CH₂CH₃)₂. More preferably, R₆ is hydrogen.

n is preferably 1, 2 or 3, more preferably 2.

L is preferably selected from the group consisting of -CH₂-, -O-, -CF₂- and -S-, more preferably -O-.

X is preferably selected from the group consisting of O and S, more preferably O.

Preferably in the present invention, the 2-pyridone derivative has any one of the structures represented by formula IIa to formula IIc: 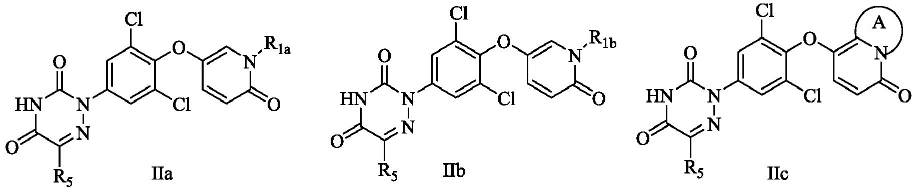
wherein, R₁ₐ is selected from the group consisting of C₁₋₆ alkyl and saturated or unsaturated C₃₋₆ cycloalkyl; wherein the C₁₋₆ alkyl can be optionally further substituted with one or more of halogen, deuterium, hydroxyl, alkoxy, oxo, amino, C₃₋₆ cycloalkyl, 5-10 membered aryl or 5-10 membered heteroaryl; the saturated or unsaturated C₃₋₆ cycloalkyl can be optionally further substituted with one or more of halogen, deuterium, hydroxy, alkoxy, oxo or amino;
the 5-10 membered aryl or 5-10-membered heteroaryl can be optionally substituted with one or more of halogen, substituted or unsubstituted C₁₋₆ alkyl, substituted or unsubstituted C₃₋₆ cycloalkyl, substituted or unsubstituted C₁₋₆ alkoxy, cyano, hydroxyl, or amino;
R_{1b} is selected from the group consisting of C₅₋₁₀ aryl and C₅₋₁₀ heteroaryl, wherein the C₅₋₁₀ aryl or C₅₋₁₀ heteroaryl can be optionally further substituted with one or more of halogen, substituted or unsubstituted C₁₋₆ alkyl, substituted or unsubstituted C₃₋₆ cycloalkyl, substituted or unsubstituted C₁₋₆ alkoxy, cyano, hydroxyl, or amino;
ring A in formula IIc is a 4-10 membered heterocycloalkyl containing attached nitrogen, wherein the 4-10 membered heterocycloalkyl can further contains optional 0, 1 or 2 oxygen, sulfur and nitrogen atoms in addition to the attached nitrogen atom, and the heterocycloalkyl can be optionally further substituted with one or more of C₁-C₆ alkyl, hydroxyl, halogen, or cycloalkyl;
R₅ is selected from the group consisting of hydrogen, cyano, carboxyl, C₁₋₆ alkyl, and C₃₋₆ cycloalkyl, wherein the C₁₋₆ alkyl or C₃₋₆ cycloalkyl can be optionally substituted with one or more of halogen, hydroxyl, or C₁₋₆ alkoxy.

The above alkoxy is preferably C₁₋₆ alkoxy.

Preferably in the present invention, R₁ₐ is selected from the group consisting of methyl, ethyl, n-propyl, isopropyl, n-butyl, isobutyl, tert-butyl, n-pentyl, isopentyl, neopentyl, -CH(CH₂CH₃)₂, cyclopropyl, cyclobutyl, cyclopentyl, cyclohexyl, cyclopentenyl, and cyclohexenyl.

The methyl, ethyl, n-propyl, isopropyl, n-butyl, isobutyl, tert-butyl, n-pentyl, isopentyl, neopentyl, or -CH(CH₂CH₃)₂ can be optionally substituted with one or more of halogen, deuterium, hydroxyl, C₁₋₆ alkoxy, oxo, amino, C₃₋₆ cycloalkyl, phenyl, naphthyl, pyridyl, or pyrrolyl.

Preferably in the present invention, the phenyl, naphthyl, pyridyl, or pyrrolyl can be optionally substituted with one or more of halogen, substituted or unsubstituted C₁₋₆ alkyl, substituted or unsubstituted C₃₋₆ cycloalkyl, substituted or unsubstituted C₁₋₆ alkoxy, cyano, hydroxyl, or amino.

Preferably in the present invention, the C₁₋₆ alkyl, C₃₋₆ cycloalkyl, or C₁₋₆ alkoxy can be optionally further substituted with one or more F atoms.

The cyclopropyl, cyclobutyl, cyclopentyl, cyclohexyl, cyclopentenyl, or cyclohexenyl can be optionally substituted with one or more of halogen, deuterium, hydroxyl, C₁₋₆ alkoxy, oxo or amino.

R_{1b} is preferably selected from the group consisting of C₅₋₁₀ aryl or C₅₋₁₀ heteroaryl.

Preferably in the present invention, the C₅₋₁₀ aryl or C₅₋₁₀ heteroaryl can be optionally substituted with one or more of halogen, substituted or unsubstituted C₁₋₆ alkyl, substituted or unsubstituted C₃₋₆ cycloalkyl, substituted or unsubstituted C₁₋₆ alkoxy, or cyano.

Preferably in the present invention, the C₁₋₆ alkyl, C₃₋₆ cycloalkyl, or C₁₋₆ alkoxy can be optionally further substituted with one or more F atoms.

Further preferably, R_{1b} is selected from the group consisting of substituted or unsubstituted phenyl, thienyl and thiazolyl.

The phenyl, thienyl, or thiazolyl can be optionally substituted with one or more of halogen, substituted or unsubstituted C₁₋₆ alkyl, substituted or unsubstituted C₃₋₆ cycloalkyl, substituted or unsubstituted C₁₋₆ alkoxy, cyano, hydroxyl, or amino.

The above C₁₋₆ alkyl is preferably selected from the group consisting of methyl, ethyl, propyl, isopropyl, butyl, isobutyl and tert-butyl.

The above C₁₋₆ alkoxy is preferably selected from the group consisting of methoxy, ethoxy, propoxy, isopropoxy, butoxy and pentyloxy.

The above C₃₋₆ cycloalkyl is preferably selected from the group consisting of cyclopropyl, cyclobutyl, cyclopentyl and cyclohexyl.

The above C₁₋₆ alkyl, C₁₋₆ alkoxy, or C₃₋₆ cycloalkyl can be optionally further substituted with 1-3 fluorine atoms.

Preferably in the present invention, in the formula IIc, ring A is selected from the group consisting of a 5-6 membered monocyclic heterocyclyl and a 7-10 membered spiro heterocyclyl.

Preferably in the present invention, in the formula IIc, ring A and/or R₁ and R₂ together with the atoms connected to the originally attached atoms form a 4-10-membered ring, preferably a 5-membered monocyclic ring or a 6-membered monocyclic ring.

Preferably in the present invention, in the formula IIc, ring A is a five-membered or six-membered monocyclic heterocyclyl; more preferably, ring A is a tetrahydropyrrole ring.

The five-membered or six-membered monocyclic heterocyclyl can be optionally substituted with one or more of halogen, C₁₋₃ alkyl or C₃₋₆ cycloalkyl.

In particular, the five-membered or six-membered monocyclic heterocyclyl can be optionally substituted with one or more of fluorine, chlorine, bromine, methyl, ethyl, propyl, isopropyl, cyclopropyl, cyclobutyl, cyclopentyl, or cyclohexyl.

R₅ in the formula IIa to formula IIc is selected from the same group as above, which will not be elaborated here.

Preferably in the present invention, R₂ is H;
R₃ is H;
R₄ is selected from the group consisting of fluorine, chlorine, bromine and iodine; further R₄ is selected from the group consisting of ortho-difluoro, ortho-dichloro and ortho-dibromo at the L position;
R₆ is H;
Lis -O-;
X is O.

Preferably in the present invention, the 2-pyridone derivative has any one of the following structures:

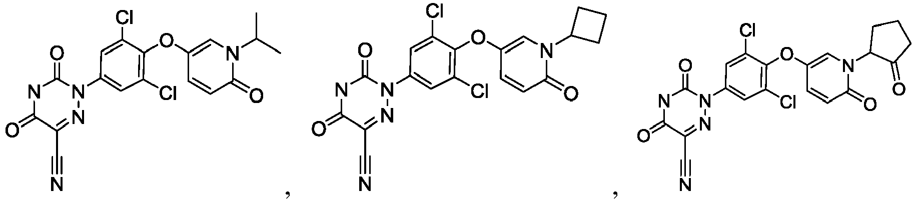

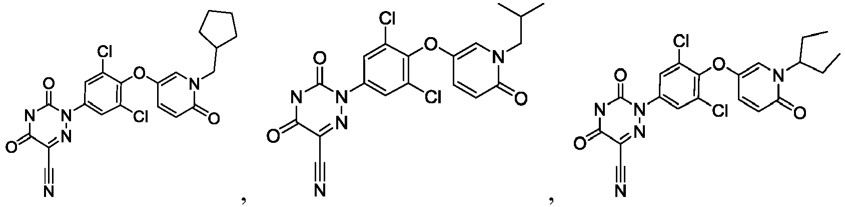

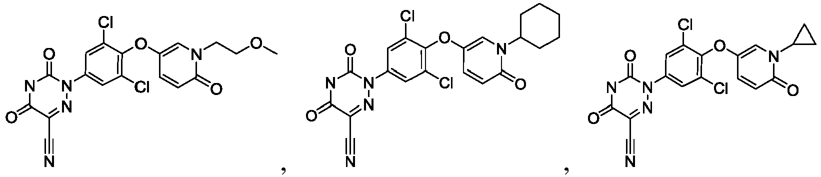

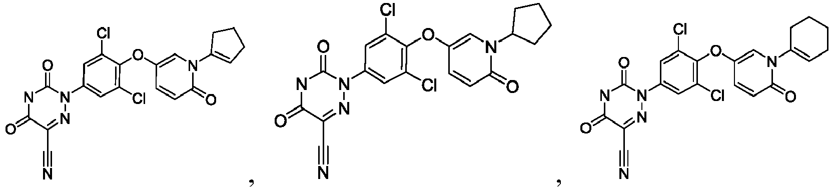

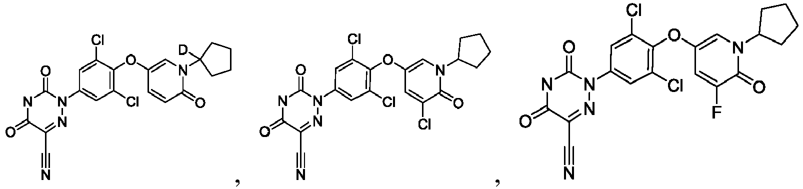

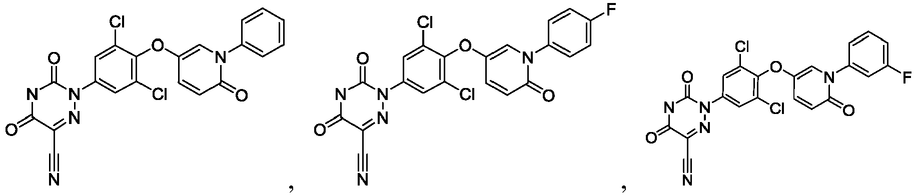

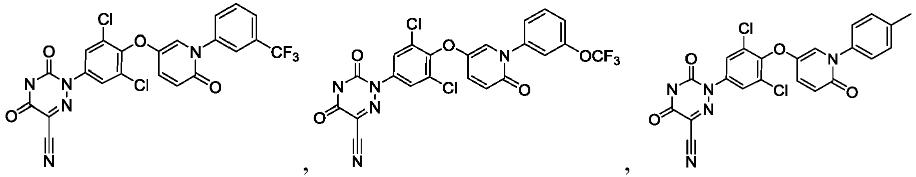

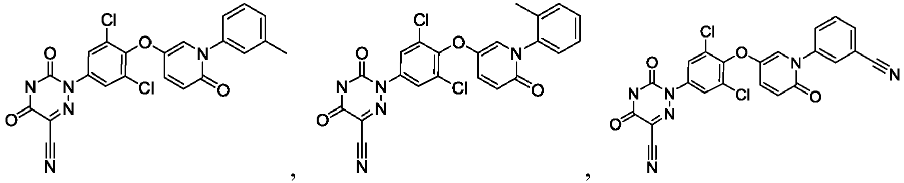

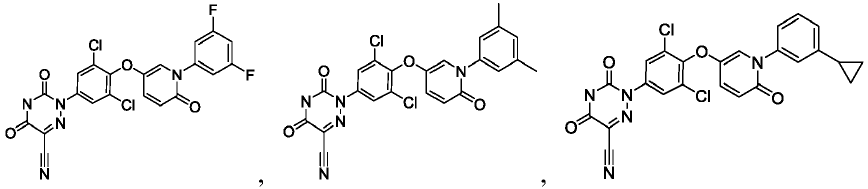

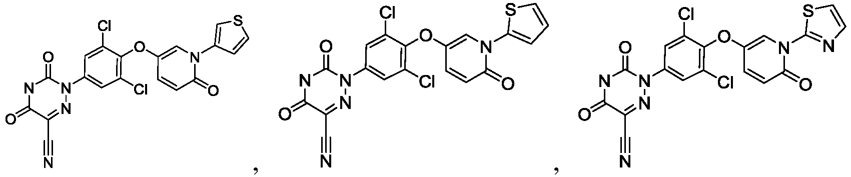

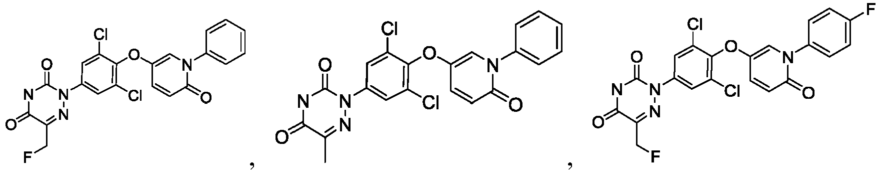

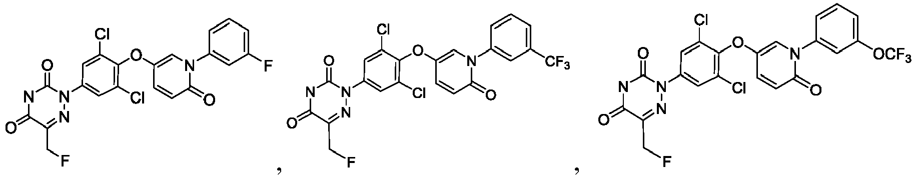

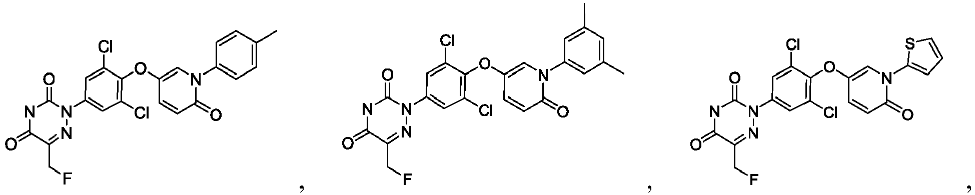

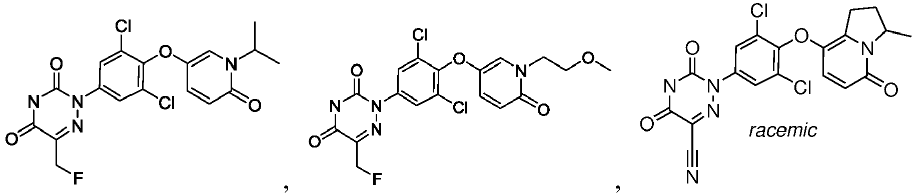

Some embodiments of the compounds of the present invention can be realized by the following reaction scheme:

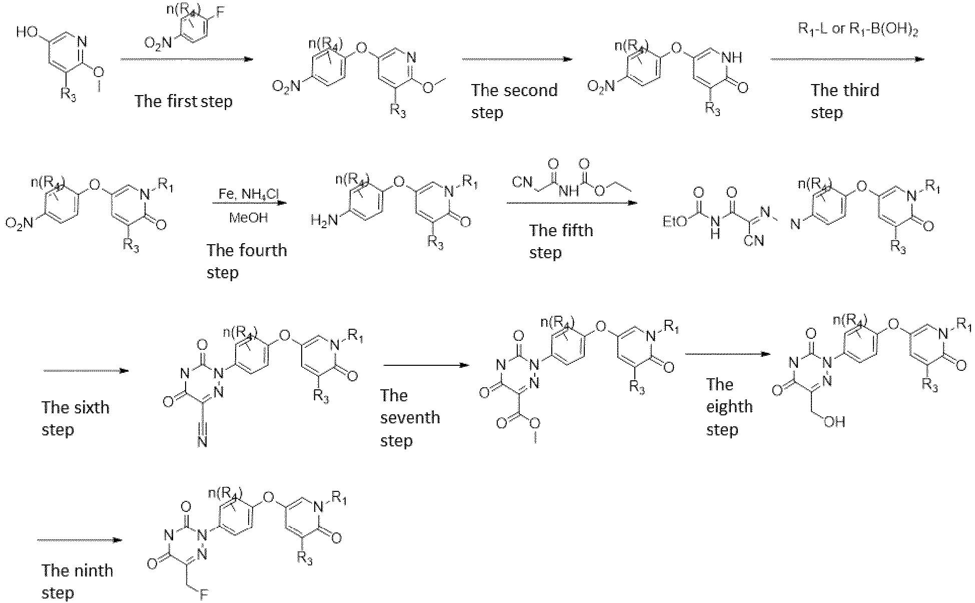

The present invention further provides use of the above 2-pyridone derivative in the manufacture of a medicament for preventing, treating and/or alleviating a disease caused by the regulation of thyroid hormone analogs.

Preferably in the present invention, the disease caused by the regulation of thyroid hormone analogs is selected from the group consisting of obesity, hyperlipidemia, hypercholesterolemia, diabetes, non-alcoholic steatohepatitis (NASH), atherosclerosis, cardiovascular disease, hypothyroidism, thyroid cancer and a combination thereof.

The present invention further provides a pharmaceutical preparation, comprising the above 2-pyridone derivative, and a pharmaceutically acceptable excipient.

In the present invention, the pharmaceutical preparation has a dosage form selected from the group consisting of tablet, hard capsule, soft capsule, dry suspension, drop pill or micropill.

"Optional" or "optionally" means that the subsequently described event or circumstance may or may not occur, and the description includes instances where the event or circumstance occurs and instances where the event or circumstance does not occur. For example, "optionally substituted alkyl" includes "alkyl" and "substituted alkyl" as defined herein. Those skilled in the art will appreciate that any group containing one or more substituents is not intended to introduce any substitution or substitution pattern that is sterically unavailable, synthetically infeasible, and/or inherently unstable.

"Alkyl" includes straight and branched chains having the indicated number of carbon atoms (typically 1-20 carbon atoms, for example 1-8 carbon atoms, such as 1-6 carbon atoms). For example, C₁-C₆ alkyl includes straight chain and branched chain alkyl of 1 to 6 carbon atoms. Examples of alkyl include, but are not limited to, methyl, ethyl, propyl, isopropyl, n-butyl, sec-butyl, tert-butyl, pentyl, 2-pentyl, isopentyl, neopentyl, hexyl, 2-hexyl, 3-hexyl, 3-methylpentyl, etc. Alkylene is another subset of alkyl and refers to the same residues as alkyl, but has two points of attachment. Alkylene generally has 2-20 carbon atoms, for example 2-8 carbon atoms, such as 2-6 carbon atoms. When naming an alkyl residue having a specific number of carbon atoms, all geometric isomers with that number of carbon atoms are intended to be included. For example, "butyl" is intended to include n-butyl, sec-butyl, iso-butyl and tert-butyl; "propyl" includes n-propyl and isopropyl. "Lower alkyl" refers to an alkyl having 1 to 4 carbon atoms.

"Alkenyl" refers to a straight-chain or branched-chain hydrocarbon group having the indicated number of carbon atoms (usually 1-8 carbon atoms, such as 2-4 carbon atoms) and at least 1 and preferably 1-2 vinyl (>C=C<) unsaturated sites. Examples of such group include, for example, vinyl, allyl and but-3-en-1-yl. This term includes the cis and trans isomers or mixtures of such isomers. "Lower alkenyl" refers to an alkenyl having 1 to 4 carbon atoms, which can be represented by C₂-C₄ alkenyl.

"Cycloalkyl" refers to a partially saturated, or fully saturated non-aromatic carbocyclic ring having the indicated number of ring carbon atoms (for example, 3-10, or 3-8, or 3-6 ring carbon atoms). Cycloalkyl can be monocyclic or polycyclic (for example, bicyclic, tricyclic). Examples of cycloalkyl include cyclopropyl, cyclobutyl, cyclopentyl, cyclopentenyl, cyclohexyl, a bridged ring group and a caged ring group (for example, bicyclo[2.2.2]octane). Lower cycloalkane generally refers to 3-6 single rings.

"Aryl" refers to an aromatic carbocyclic ring having the indicated number of carbon atoms (for example, 6-12 or 6-10 carbon atoms) in the ring. Aryl can be monocyclic or polycyclic (for example, bicyclic, tricyclic). In some cases, both rings of a polycyclic aryl are aromatic (for example, naphthyl). In other cases, a polycyclic aryl can include a non-aromatic ring fused to an aromatic ring (for example, cycloalkyl, cycloalkenyl, heterocycloalkyl, heterocycloalkenyl), as long as the polycyclic aryl is bonded to the parent structure through an atom in the aromatic ring. Therefore, 1,2,3,4-tetrahydronaphthalen-5-yl (where the moiety is bonded to the parent structure through an aromatic carbon atom) is considered as aryl, while 1,2,3,4-tetrahydronaphthalen-1-yl (where the moiety is bonded to the parent structure through a non-aromatic carbon atom) is not considered as aryl. Similarly, 1,2,3,4-tetrahydroquinolin-8-yl (where the moiety is bonded to the parent structure through an aromatic carbon atom) is considered as aryl, while 1,2,3,4-tetrahydroquinolin-1-yl (where the moiety is bonded to the parent structure through a non-aromatic nitrogen atom) is not considered as aryl. However, the term "aryl" does not include or overlap with "heteroaryl" as defined herein, regardless of point of attachment (for example, quinolin-5-yl and quinolin-2-yl are all heteroaryl). In some cases, aryl is phenyl or naphthyl. In some cases, aryl is phenyl. Other examples of aryl comprising an aromatic carbocyclic ring fused to a non-aromatic ring are described below.

"Carboxy" or "carboxyl" refers to -COOH or a salt thereof.

"Heteroaryl" refers to an aromatic ring containing the indicated number of ring atoms (for example, 5-12 or 5-10 membered heteroaryl), wherein the ring contains one or more heteroatoms selected from the group consisting of N, O and S atoms (for example, 1, 2, 3, or 4 heteroatoms), and the remaining ring atoms are carbon. 5-membered heteroaryl is a heteroaryl having 5 ring atoms. 6-membered heteroaryl is a heteroaryl having 6 ring atoms. In some embodiments, the total number of S and O atoms in heteroaryl does not exceed 2. In some embodiments, the total number of S and O atoms in heteroaryl does not exceed 1. Unless otherwise stated, heteroaryl can be bonded to the parent structure through a carbon or nitrogen atom, as valence permits. For example, "pyridyl" includes 2-pyridyl, 3-pyridyl and 4-pyridyl, and "pyrrolyl" includes 1-pyrrolyl, 2-pyrrolyl and 3-pyrrolyl. When nitrogen is present in a heteroaryl ring, the nitrogen may exist in an oxidized state (i.e., N+-O-) as the nature of the adjacent atoms and groups permits. Furthermore, when sulfur is present in a heteroaryl ring, the sulfur may exist in an oxidized state (i.e., S+-O- or SO₂) as the nature of the adjacent atoms and groups permits. Heteroaryl can be monocyclic or polycyclic (for example, bicyclic, tricyclic).

In some cases, heteroaryl is monocyclic. Examples include pyrrolyl, pyrazolyl, imidazolyl, triazolyl (for example, 1,2,3-triazolyl, 1,2,4-triazolyl, 1,2,4-triazolyl), tetrazolyl, furanyl, isoxazolyl, oxazolyl, oxadiazolyl (for example, 1,2,3-oxadiazolyl, 1,2,4-oxadiazolyl, 1,3,4-oxadiazolyl), thiophenyl, isothiazolyl, thiazolyl, thiadiazolyl (for example, 1,2,3-thiadiazolyl, 1,2,4-thiadiazolyl, 1,3,4-thiadiazolyl), pyridinyl, pyridazinyl, pyrimidinyl, pyrazinyl, triazinyl (for example, 1,2,4-triazinyl, 1,3,5-triazinyl) and tetrazinyl.

In other cases, polycyclic heteroaryl can include a non-aromatic ring fused to a heteroaryl ring (for example, cycloalkyl, cycloalkenyl, heterocycloalkyl, heterocycloalkenyl), as long as the polycyclic heteroaryl is bonded to the parent structure through an atom in the aromatic ring. For example, 4,5,6,7-tetrahydrobenzo[d]thiazol-2-yl (where the moiety is bonded to the parent structure through an aromatic carbon atom) is considered as heteroaryl, while 4,5,6,7-tetrahydrobenzo[d]thiazol-5-yl (wherein the moiety is bonded to the parent structure through a non-aromatic carbon atom) is not considered as heteroaryl. Examples of polycyclic heteroaryl formed by a heteroaryl ring fused to a non-aromatic ring are described below.

"Heterocycloalkyl" refers to a partially saturated or fully saturated non-aromatic ring having the indicated number of ring atoms (for example, 3-10 or 3-7 membered heterocycloalkyl), wherein the ring contains one or more heteroatoms selected from the group consisting of N, O and S atoms (for example, 1, 2, 3, or 4 heteroatoms), and the remaining ring atoms are carbon. 5-membered heterocycloalkyl is a heterocycloalkyl having 5 ring atoms. 6-membered heterocycloalkyl is a heterocycloalkyl having 6 ring atoms. Heterocycloalkyl can be monocyclic or polycyclic (for example, bicyclic, tricyclic). Examples of heterocycloalkyl include oxiranyl, aziridinyl, azetidinyl, pyrrolidinyl, imidazolidinyl, pyrazolidinyl, piperidinyl, piperazinyl, morpholinyl and thiomorpholinyl. When nitrogen is present in a heterocycloalkyl ring, the nitrogen may exist in an oxidized state (i.e., N+-O-) as the nature of the adjacent atoms and groups permits, for example, piperidinyl-N-oxide and morpholinyl-N-oxide. In addition, when sulfur is present in a heterocycloalkyl ring, the sulfur may exist in an oxidized state (i.e., S+-O- or -SO2-) as the nature of the adjacent atoms and groups permits, for example, thiomorpholinyl-S-oxide and thiomorpholinyl-S,S-dioxide. In addition, one ring of a polycyclic heterocycloalkyl can be aromatic (for example, aryl or heteroaryl), as long as the polycyclic heterocycloalkyl is bonded to the parent structure through a non-aromatic carbon or nitrogen atom. For example, 1,2,3,4-tetrahydroquinolin-1-yl (where the moiety is bonded to the parent structure through a non-aromatic nitrogen atom) is considered as heterocycloalkyl, while 1,2,3,4-tetrahydroquinolin-8-yl (where the moiety is bonded to the parent structure through an aromatic carbon atom) is not considered as heterocycloalkyl. Examples of polycyclic heterocycloalkyl formed by a heterocycloalkyl fused with an aromatic ring are described below.

"Alkoxy" refers to alkyl having the indicated number of carbon atoms attached through an oxygen bridge, for example methoxy, ethoxy, propoxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy, 3-methylpentyloxy, etc. Alkoxy is also intended to include cycloalkyl attached through an oxygen bridge as defined above. Alkoxy typically has 1-6 carbon atoms attached through an oxygen bridge. "Lower alkoxy" refers to alkoxy having 1 to 4 carbon atoms.

The term "halo" includes fluoro, chloro, bromo and iodo, and the term "halogen" includes fluorine, chlorine, bromine and iodine.

As used herein, the term "substituted" means that any one or more hydrogens on an indicated atom or group are selectively replaced by an indicated group, provided that the normal valence of the indicated atom permits. When a substituent is oxo (i.e., =O), 2 hydrogens on the atom are replaced. Combinations of substituents and/or variables are permissible only if such combinations result in stable compounds or useful synthetic intermediates. A stable compound or stable structure is intended to imply that the compound is stable enough to be separated from the reaction mixture and subsequently prepared into at least a practically useful reagent. Unless otherwise indicated, substituents are named to the core structure. For example, it should be understood that when (cycloalkyl)alkyl is listed as a possible substituent, the point of attachment of the substituent to the core structure is in the alkyl moiety.

Pharmaceutically acceptable salts include, but are not limited to: salts formed from inorganic acids, such as hydrochlorides, phosphates, diphosphates, hydrobromides, sulfates, sulfinates, nitrates, etc.; and salts formed from organic acids, such as malate, maleate, fumarate, tartrate, succinate, citrate, acetate, lactate, methanesulfonate, p-toluenesulfonate, 2-hydroxyethylsulfonate, benzoate, salicylate, stearate and alkanoate such as acetates, HOOC-(CH₂)n-COOH (where n is 0-4) and similar salts. Similarly, pharmaceutically acceptable cations include, but are not limited to, sodium, potassium, calcium, aluminum, lithium, and ammonium.

Furthermore, if the compound described herein is obtained as an acid addition salt, the free base can be obtained by basifying a solution of the salt. On the contrary, if the product is a free base, an addition salt, especially pharmaceutically acceptable addition salt can be produced by dissolving the free base in a suitable organic solvent and treating the solution with an acid according to conventional methods for preparing acid addition salts from base compounds. Those skilled in the art will know a variety of synthetic methods that can be used to prepare non-toxic pharmaceutically acceptable addition salts.

As used herein, the terms "group", "residue" and "fragment" are synonymous and are intended to denote a functional group or molecular fragment that can be attached to a bond or to another molecular fragment.

The term "therapeutically effective amount" or "effective amount" refers to such amount that provides therapeutic benefits such as amelioration of symptoms, delay of disease progression, or prevention of disease when administered to a human or non-human subject, or inhibits the activity of fascin *in vitro* or *in vivo.* For example, a therapeutically effective amount can be an amount sufficient to reduce symptoms of a disease in response to inhibition of fascin activity.

"Treatment" refers to any treatment of a disease in a patient, including:
a) preventing the disease, i.e., causing the clinical symptoms of the disease not to develop;
b) inhibiting the progression of the disease;
c) slowing or impeding the development of clinical symptoms; and/or
d) ameliorating the disease, i.e., causing regression of clinical symptoms.

"Subject" or "patient" refers to an animal, such as a mammal, which has been or will be the subject of treatment, observation or experimentation. The methods described herein may be useful in both human therapy and veterinary applications. In some embodiments, the subject is a mammal; in some embodiments, the subject is a human.

The compounds as described herein can be prepared into a pharmaceutical composition, which is then administered to a mammalian host (such as a human patient) in a variety of forms suitable for the chosen route of administration, i.e., oral or parenteral, intravenous, intramuscular, topical, transdermal, intrathecal, ophthalmic, intranasal, intraperitoneal, or subcutaneous routes.

The compounds described herein can be administered systemically, for example, orally or intravenously in combination with a pharmaceutically acceptable vehicle (such as an inert diluent or an assimilable edible carrier). They can be encapsulated in hard or soft shell gelatin capsules, compressed into tablets, or admixed directly with the food for the patient. For oral administration of the therapeutic agent, the active compound can be combined with one or more excipients and used in the form of ingestible tablets, buccal tablets, troches, capsules, elixirs, suspensions, syrups, wafer capsules and the like.

The starting materials for the following reactions are generally known compounds or can be prepared by known procedures or obvious improvements thereof.

In appropriate circumstances, the various starting materials, intermediates and compounds described herein can be isolated and purified using conventional techniques such as precipitation, filtration, crystallization, evaporation, distillation and chromatography. Characterization of these compounds can be performed using conventional methods such as melting point, mass spectrometry, nuclear magnetic resonance and various other spectroscopic analysis.

The present disclosure is not to be limited by the specific embodiments described in this application, which are intended as illustrations in various aspects. It is apparent to those skilled in the art that multiple modifications and changes can be made without departing from the spirit and scope of the present invention. Functionally equivalent compositions, equipment, and methods within the scope of the present disclosure in addition to those enumerated herein, will be apparent to those skilled in the art from the foregoing description. Such modifications and changes are intended to fall within the scope of the claims. The present disclosure is to be limited only by the full scope of the claims and equivalents to which such claims are entitled. It is to be understood that this disclosure is not limited to particular methods, reagents, compounds compositions or biological systems, which can surely vary. It is also to be understood that terminology used herein is only for the purpose of describing particular embodiments and is not intended to be limiting.

In addition, where features or aspects of the disclosure are described in terms of Markush groups, those skilled in the art will recognize that the disclosure is thereby also described in terms of any individual member or subgroup of members of the Markush group.

Although certain embodiments have been illustrated and described, it should be understood that changes and modifications can be made therein according to ordinary skill in the art without departing from the technology as defined in the following claims in its broader aspects.

Compared with the prior art, the present invention provides a 2-pyridone derivative having a structure represented by formula I, a stereoisomer thereof or a pharmaceutically acceptable salt thereof. Activity experiment results show that the 2-pyridone derivative provided by the present invention has high activity and high selectivity, and can be used to treat diseases related to thyroid hormone receptors.

### DETAILED DESCRIPTION

In order to further illustrate the present invention, the 2-pyridone derivative provided by the present invention, the preparation method therefor and pharmaceutical use thereof will be described in detail below in conjunction with the examples.

### Example 1

2-(3,5-Dichloro-4-((1-isopropyl-6-oxo-1,6-dihydropyridin-3-yl)oxy)phenyl)-3,5-dioxo-2,3,4,5-tetrahydro-1,2,4-triazine-6-carbonitrile

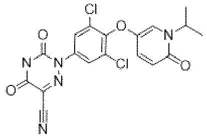

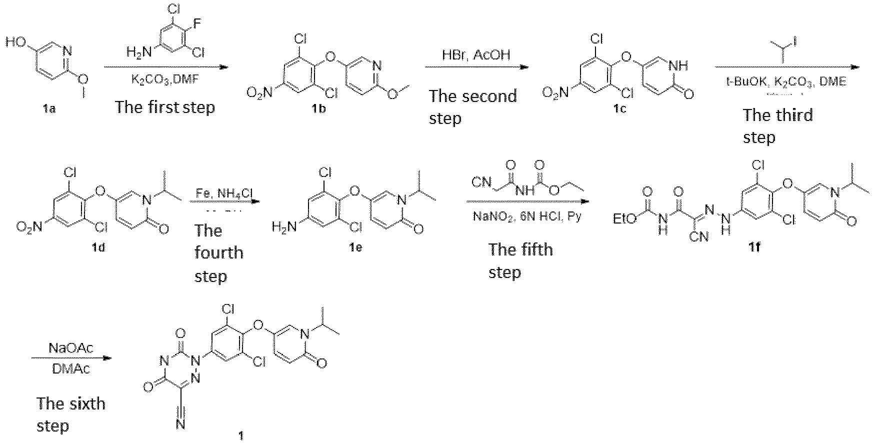

### The first step

### 5-(2,6-Dichloro-4-nitrophenoxy)-2-methoxypyridine

6-Methoxypyridin-3-ol 1a (6.3 g, 50.4 mmol) was dissolved in 80 mL of N,N-dimethylformamide, and then added with 3,5-dichloro-4-fluoroaniline (13.8 g , 65.5 mmol), potassium carbonate (20.8 g, 151.0 mmol) for 16 h of reaction at 24°C. The reaction solution was added with 70 mL of water until a large amount of white solids were precipitated. The mixture was then filtered. The filter cake was washed with water for 1 to 2 times, and then dissolved with isopropanol. The obtained solution was then concentrated. The above operation was repeated 2 times. The solvent was evaporated under reduced pressure to obtain 5-(2,6-dichloro-4-nitrophenoxy)-2-methoxypyridine 1 b (15.0 g, pale yellow solid) with a yield of 94.5%. MS m/z (ESI): 315.00 [M+1]⁺.

### The second step

### 5-(2,6-Dichloro-4-nitrophenoxy)-2-carbonylpyridine

5-(2,6-Dichloro-4-nitrophenoxy)-2-methoxypyridine 1b (15.0 g, 47.60 mmol) was dissolved in 30 mL of a mixed solution of hydrobromic acid and acetic acid in 1:1. The mixture was then heated up to 100°C for 4 h of reaction under nitrogen protection. Part of the solvent was evaporated under reduced pressure until a large amount of solids were precipitated. The mixture was filtered. The filter cake was dissolved with isopropanol, and the obtained solution was then concentrated. The above operation was repeated 2-3 times to obtain 5-(2,6-dichloro-4-nitrophenoxy)-2-hydroxypyridine 1c (13.0 g, pale yellow solid) with a yield of 90.7%. MS m/z (ESI): 300.95 [M+1]⁺. ¹H NMR (400 MHz, DMSO-*d₆*) δ 8.52 (s, 2H), 7.44 (d, *J* = 8.0 Hz, 1H), 7.27 (s, 1H), 6.49(d, *J* = 8.0 Hz,1H).

### The third step

### 5-(2,6-Dichloro-4-nitrophenoxy)-1-isopropyl-2-hydroxypyridine

5-(2,6-dichloro-4-nitrophenoxy)-2-hydroxypyridine 1c (2.5 g, 8.3 mmol) was dissolved in 30 mL of ethylene glycol dimethyl ether, and then added with potassium tert-butoxide (932.0 mg, 8.3 mmol). The mixture was stirred at room temperature for 40 minutes, and then added with potassium carbonate (803.0 mg, 5.81 mmol), 2-iodopropane (2.5 mL, 24.91 mmol). The resulting mixture was heated up to 100°C and reacted at this temperature for 4 h. The reaction solution was concentrated under reduced pressure to remove part of the solvent. Then the reaction solution was added with 30 mL of water, and extracted with ethyl acetate (30 mL×3). The organic phases were combined, dried over anhydrous sodium sulfate, and filtered. The filtrate was concentrated under reduced pressure. The residue was purified by silica gel column chromatography to obtain 5-(2,6-dichloro-4-nitrophenoxy)-1-isopropyl-2-hydroxypyridine 1d (1.1 g, yellow solid) with a yield of 38.6% . MS m/z (ESI): 343.00 [M+1]⁺. ¹H NMR (400 MHz, CDCl₃) δ 8.31 (s, 2H), 7.11 (dd, *J* = 8.0, 4.0 Hz, 1H), 7.05 (d, *J* = 4.0 Hz, 1H), 6.63(d, *J* = 8.0 Hz,1H), 5.30-5.20 (m, 1H), 1.33(d, *J* = 8.0 Hz, 6H).

### The fourth step

### 5-(4-Amino-2,6-dichlorophenoxy)-1-isopropyl-2-hydroxypyridine

5-(2,6-Dichloro-4-nitrophenoxy)-1-isopropyl-2-hydroxypyridine 1d (1.1 g, 3.2 mmol) was dissolved in 10 mL of methanol, and then added with a saturated ammonium chloride aqueous solution (1 mL) and iron powder (895.0 mg, 16 mmol) for 4 h of reaction at 100°C. The reaction solution was filtered. The filtrate was dried by rotating, added with 30 mL of water, and extracted with ethyl acetate (30 mL×3). The organic phases were combined, dried over anhydrous sodium sulfate, and filtered. The filtrate was concentrated under reduced pressure. The residue was purified by silica gel column chromatography to obtain 5-(4-amino-2,6-dichlorophenoxy)-1-isopropyl-2-hydroxypyridine 1e (760.0 mg, gray solid) with a yield of 75.7%. MS m/z (ESI): 313.05 [M+1]⁺.

### The fifth step

### Ethyl (E)-(2-cyano-2-(2-(3,5-dichloro-4-((1-isopropyl-6-oxo-1,6-dihydropyridin-3-yl) oxy)phenyl)hydrazono)acetyl carbamate

5-(4-Amino-2,6-dichlorophenoxy)-1-isopropyl-2-hydroxypyridine 1e (760.0 mg, 2.43 mmol) was dissolved in 6 N hydrochloric acid aqueous solution. The obtained solution was cooled to 0°C, and then added with an aqueous solution of sodium nitrite (251.1 mg, 3.64 mmol, 1 mL) for 30 min of reaction at 0°C. N-cyanoacetylurethane (568.3 mg, 3.64 mmol) was dissolved in 3 mL of water. The resulting mixture was cooled to 0°C, and added with pyridine (5 mL). The former reaction solution was slowly added dropwise to the latter reaction solution for 30 min of reaction at 0°C. Then the above reaction solution was added with 15 mL of water, and a large amount of solids were precipitated. The mixture was filtered. The filter cake was dissolved in isopropanol, and the obtained solution was then concentrated. The above operation was repeated 2 to 3 times to obtain ethyl (E)-(2-cyano-2-(2-(3,5-dichloro-4-((1-isopropyl-6-oxo-1,6-dihydropyridin-3-yl)oxy)phenyl)hydr azono)acetyl carbamate 1f (1.0 g, reddish-brown solid) crude. MS m/z (ESI): 482.05 [M+1]⁺.

### The sixth step

### 2-(3,5-Dichloro-4-((1-isopropyl-6-oxo-1,6-dihydropyridin-3-yl)oxy)phenyl)-3,5-dioxo-2,3,4,5-tetrahydro-1,2,4-triazine-6-carbonitrile

Ethyl (E)-(2-cyano-2-(2-(3,5-dichloro-4-((1-isopropyl-6-oxo-1,6-dihydropyridin-3-yl)oxy)phenyl)hydrazono)acetyl carbamate 1f (1.0 g) crude was dissolved in N,N-dimethylacetamide (15 mL), and added with sodium acetate (512.3 mg, 2.08 mmol) for 3 h of reaction at 120°C. The reaction solution was added with 30 mL of water, extracted with ethyl acetate (30 mL×3), and washed twice with a saturated sodium chloride aqueous solution. The organic phases were combined, dried over anhydrous sodium sulfate, and filtered. The filtrate was concentrated under reduced pressure. The obtained residue was purified by silica gel column chromatography to obtain (3,5-dichloro-4-((1-isopropyl-6-oxo-1,6-dihydropyridin-3-yl)oxy) phenyl)-3,5-dioxo-2,3,4,5-tetrahydro 1,2,4-triazine-6-carbonitrile 1 (650.0 mg, red solid) with a yield of 61.6% over two steps. MS m/z (ESI): 434.00 [M+1]⁺. ¹H NMR (400 MHz, DMSO-d*₆*) δ 7.78 (s, 2H), 7.58 (d, *J* = 4.0 Hz, 1H), 7.23 (dd, *J* = 8.0, 4.0 Hz, 1H), 6.38 (d, *J* = 8.0 Hz, 1H), 5.10 - 4.93 (m, 1H), 1.27 (d, *J* = 8.0 Hz, 8H).

### Example 2

### 2-(3,5-Dichloro-4-((6-oxo-1,6-dihydropyridin-3-yl)oxy)phenyl)-3,5-dioxo-2,3,4,5-tetra hydro-1H-1,2,4-triazine-6-carbonitrile

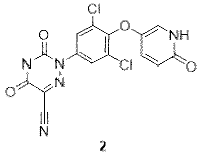

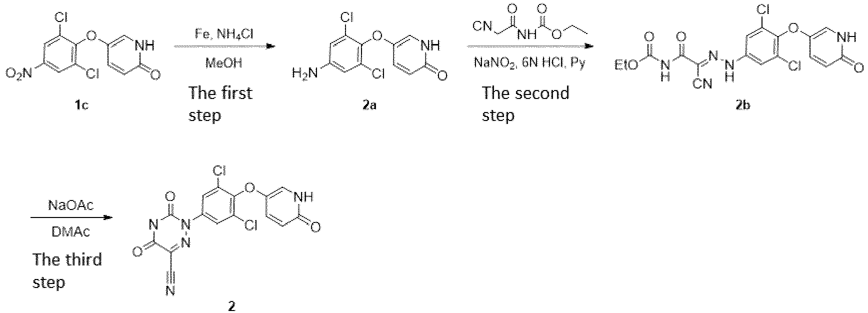

### The first step

### 5-(4-Amino-2,6-dichlorophenoxy)-2-carbonyl pyridine

5-(4-Nitro-2,6-dichlorophenoxy)-2-carbonylpyridine 1c (400.0 mg, 1.33 mmol) was dissolved in 6 mL of methanol, and added with iron powder (222.5 mg, 3.99 mmol) and 1 mL of saturated ammonium chloride aqueous solution for 6 h of reaction at 85°C. The iron powder was removed using a magnetic rod. The reaction solution was dried by rotating, added with 20 mL of ethyl acetate and 15 mL of water, extracted three times, washed once with saturated saline, dried over anhydrous sodium sulfate, and filtered. The organic phase was dried by rotating, and purified by flash column chromatography to obtain 5-(4-amino-2,6-dichlorophenoxy)-2-carbonylpyridine 2a (100.0 mg, brown solid) with a yield of 27.8%. MS m/z (ESI): 271.00 [M+1]⁺.

### The second step

### Ethyl (E)-(2-cyano-2-(2-(3,5-dichloro-4-((6-oxo-1,6-dihydropyridin-3-yl)oxy)phenyl) hydrazino)acetyl)carbamate

5-(4-Amino-2,6-dichlorophenoxy)-2-carbonylpyridine 2a (100.0 mg, 0.37 mmol) was dissolved in 4 mL (6N) of aqueous solution of hydrochloric acid. The mixture was cooled to 0°C, and then slowly added dropwise with 0.5 mL of an aqueous solution of sodium nitrite (38.1 mg, 0.55 mmol). Then the reaction solution was stirred for 20 minutes. N-cyanoacetylurethane (86.4 mg, 0.55 mmol) was dissolved in 3 mL of water. The mixture was cooled to 0°C, and then added with 2 mL of pyridine. The obtained solution was stirred at 0°C for 10 minutes. Then the latter reaction solution was slowly added dropwise to the former reaction solution for 30 min of reaction at 0°C. The resulting reaction solution was added with 20 mL of water, and a large amount of solids were precipitated. The mixture was filtered. The filter cake was dissolved in anhydrous ethanol, and the obtained solution was then concentrated. The above operation was repeated 2-3 times to obtain ethyl (E)-(2-cyano-2-(2-(3,5-dichloro-4-((6-oxo-1,6-dihydropyridin-3-yl)oxy)phenyl)hydrazino)acetyl) carbamate 2b (100.0 mg, red solid) with a yield of 61.8%. MS m/z (ESI): 437.95 [M+1]⁺.

### The third step

### 2-(3,5-Dichloro-4-((6-oxo-1,6-dihydropyridin-3-yl)oxy)phenyl)-3,5-dioxo-2,3,4,5-tetra hydro-1,2,4-triazine-6-carbonitrile

Ethyl (E)-(2-cyano-2-(2-(3,5-dichloro-4-((6-oxo-1,6-dihydropyridin-3-yl)oxy)phenyl) hydrazino)acetyl)carbamate 2b (100.0 mg, 0.23 mmol) was dissolved in 5 mL of N,N-dimethylacetamide, and then added with sodium acetate (74.9 mg, 0.91 mmol). The mixture was heated up to 120°C and reacted at this temperature for 4 h. The reaction solution was added with 20 mL of water, and extracted with ethyl acetate (30 mL×3). The organic phases were combined, dried over anhydrous sodium sulfate, and filtered. The filtrate was concentrated under reduced pressure, and subjected to Prep-HPLC to obtain 2-(3,5-dichloro-4-((6-oxo-1,6-dihydropyridin-3-yl)oxy)phenyl)-3,5-dioxo-2,3,4,5-tetrahydro-1,2, 4-triazine-6-carbonitrile 2 (4.99 mg, white solid) with a yield of 5.6%. MS m/z (ESI): 391.95 [M+1]⁺. ¹H NMR (400 MHz, DMSO-*d₆*) *δ* 13.29 (s, 1H), 7.78 (s, 2H), 7.38 (dd, *J* = 9.6 Hz, 3.2 Hz, 1H), 7.21 (s, 1H), 6.46 (d, *J* = 9.6 Hz, 1H).

### Example 3

### 2-(3,5-Dichloro-4-[(1-cyclobutyl-6-oxo-1,6-dihydropyridin-3-yl)oxy]phenyl)-3,5-dioxo -2,3,4,5-tetrahydro-1,2,4-triazine-6-carbonitrile

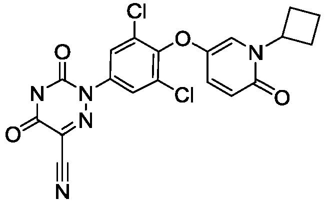

The title product 2-(3,5-dichloro-4-[(1-cyclobutyl-6-oxo-1,6-dihydropyridin-3-yl)oxy]phenyl)-3,5-dioxo-2,3,4,5-te trahydro-1,2,4-triazine-6-carbonitrile 3 (35.0 mg, white solid) was prepared using the method of Example 1 except that 2-iodopropane was replaced with bromocyclobutane. MS m/z (ESI): 446.00 [M+1]⁺. ¹H NMR (400 MHz, DMSO-*d₆*) δ 7.79 (s, 2H), 7.62 (d, *J* = 3.2 Hz, 1H), 7.25 (dd, *J* = 10.0, 3.2 Hz, 1H), 6.36 (d, *J* = 10.0 Hz, 1H), 5.07-4.95 (m, 1H), 2.30-2.21 (m, 4H), 1.79-1.67 (m, 2H).

### Example 4

### 2-(3,5-Dichloro-4-[(1-cyclobutyl-6-oxo-1,6-dihydropyridin-3-yl)oxy]phenyl)-3,5-dioxo -2,3,4,5-tetrahydro-1,2,4-triazine-6-carbonitrile

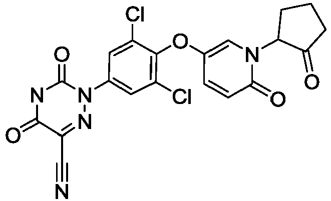

The title product 2-(3,5-dichloro-4-((6-oxo-1-(2-oxocyclopentyl)-1,6-dihydropyridin-3-yl)oxy)phenyl)-3,5-dioxo-2,3,4,5-tetrahydro-1,2,4-triazine-6-carbonitrile 4 (20.0 mg, white solid, yield 23.03%) was prepared using the method of Example 1 except that 2-iodopropane was replaced with 2-chlorocyclopentanone. MS m/z (ESI): 471.95 [M+1]⁺. ¹H NMR (400 MHz, DMSO-*d₆*) δ 13.28 (br s, 1H), 7.78 (s, 2H), 7.47 (d, *J* = 4.0 Hz, 1H), 7.43 (d, *J* = 4.0 Hz, 1H), 6.43 (d, *J* = 10.0 Hz, 1H), 4.59-4.54 (m, 1H), 2.56-2.21 (m, 5H), 1.81-1.69 (m, 1H).

### Example 5

### 2-(3,5-Dichloro-4-((1-(cyclopentylmethyl)-6-oxo-1,6-dihydropyridin-3-yl)oxy)phenyl)-3,5-dioxo-2,3,4,5-tetrahydro-1,2,4-triazine-6-carbonitrile

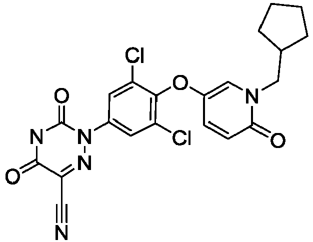

The title product 2-(3,5-dichloro-4-((1-(cyclopentylmethyl)-6-oxo-1,6-dihydropyridin-3-yl)oxy)phenyl)-3,5-dioxo-2,3,4,5-tetrahydro-1,2,4-triazine-6-carbonitrile 5 (16.0 mg, white solid, yield 41.29%) was prepared using the method of Example 1 except that 2-iodopropane was replaced with (bromomethyl)cyclopentane. MS m/z (ESI): 471.95 [M-1]⁻. ¹H NMR (400 MHz, DMSO-*d₆*) δ 13.27 (br s, 1H), 7.78 (s, 2H), 7.43-7.37 (m, 2H), 6.42 (d, *J* = 8.0 Hz, 1H), 3.78 (d, *J* = 8.0 Hz, 2H), 2.33-2.20 (m, 1H), 1.76-1.30 (m, 6H), 1.19-1.10 (m, 2H).

### Example 6

### 2-(3,5-Dichloro-4-[(1-(2-methylpropyl)-6-oxo-1,6-dihydropyridin-3-yl)oxy]phenyl)-3,5 -dioxo-2,3,4,5-tetrahydro-1,2,4-triazine-6-carbonitrile

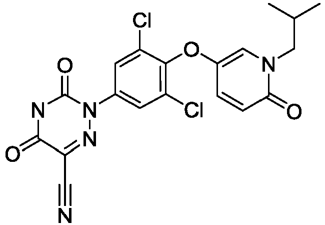

The title product 2-(3,5-dichloro-4-[(1-(2-methylpropyl)-6-oxo-1,6-dihydropyridin-3-yl)oxy]phenyl)-3,5-dioxo-2,3 ,4,5-tetrahydro-1,2,4-triazine-6-carbonitrile 6 (70.0 mg, white solid) was prepared in a yield of 77.8% using the method of Example 1 except that 2-iodopropane was replaced with 1-iodo-2-methylpropane. MS m/z (ESI): 448.00 [M+1]⁺. ¹H NMR (400 MHz, DMSO-d*₆*) δ 13.28 (s, 1H), 7.78 (s, 2H), 7.43 (dd, *J* = 10.0, 3.6 Hz, 1H), 7.38 (d, *J* = 3.2 Hz, 1H), 6.44 (d, *J* = 10.0 Hz, 1H), 3.66 (d, *J* = 7.2 Hz, 2H), 2.08 - 1.94 (m, 1H), 0.81 (d, *J* = 6.8 Hz, 6H).

### Example 7

### 2-(3,5-Dichloro-4-((6-oxo-1-(pentan-3-yl)-1,6-dihydropyridin-3-yl)oxy)phenyl)-3,5-dio xo-2,3,4,5-tetrahydro-1,2,4-triazine-6-carbonitrile

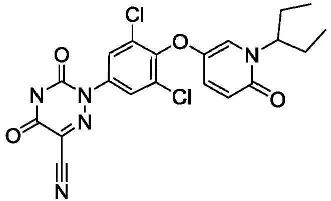

The title product 2-(3,5-dichloro-4-((6-oxo-1-(pentan-3-yl)-1,6-dihydropyridin-3-yl)oxy)phenyl)-3,5-dioxo-2,3,4,5 -tetrahydro-1,2,4-triazine-6-carbonitrile 7 (70 mg, pale yellow solid, yield 29.76%) was prepared using the method of Example 1 except that 2-iodopropane was replaced with 3-bromopentane. MS m/z (ESI): 460.00 [M-1]⁻. ¹H NMR (400 MHz, DMSO-d*₆*) δ 13.29 (s, 1H), 7.77 (s, 2H), 7.39 (s, 1H), 7.26 (dd, *J* = 10.0, 3.6 Hz, 1H), 6.41 (d, *J* = 10.0 Hz, 1H), 4.69 (m, 1H), 1.71-1.62 (m, 4H), 0.70 (t, *J* = 8.0 Hz, 6H).

### Example 8

### 2-(3,5-Dichloro-4-((1-(2-methoxyethyl)-6-oxo-1,6-dihydropyridin-3-yl)oxy)phenyl)-3,5-dioxo-2,3,4,5-tetrahydro-1,2,4-triazine-6-carbonitrile

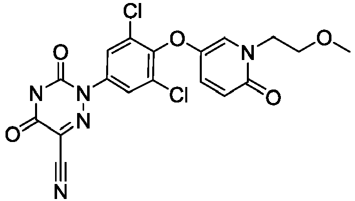

The title product 2-(3,5-dichloro-4-((1-(2-methoxyethyl)-6-oxo-1,6-dihydropyridin-3-yl)oxy)phenyl)-3,5-dioxo-2, 3,4,5-tetrahydro-1,2,4-triazine-6-carbonitrile 8 (257.0 mg, white solid, yield 15.24%) was prepared using the method of Example 1 except that 2-iodopropane was replaced with 1-bromo-2-methoxyethane. MS m/z (ESI): 449.95 [M+1]⁺.

### Example 9

### 2-(3,5-Dichloro-4-((1-cyclohexyl-6-oxo-1,6-dihydropyridin-3-yl)oxy)phenyl)-3,5-dioxo -2,3,4,5-tetrahydro-1,2,4-triazine-6-carbonitrile

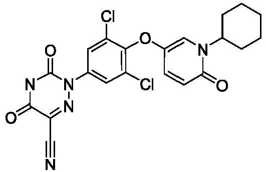

The title product 2-(3,5-dichloro-4-((1-cyclohexyl-6-oxo-1,6-dihydropyridin-3-yl)oxy)phenyl)-3,5-dioxo-2,3,4,5-t etrahydro-1,2,4-triazine-6-carbonitrile 9 (12.0 mg, white solid) was prepared in a yield of 83.8% using the method of Example 1 except that 2-iodopropane was replaced with cyclohexyl p-toluenesulfonate. MS m/z (ESI): 475.30 [M+1]⁺. ¹H NMR (400 MHz, DMSO-*d₆*) δ 13.28 (s, 1H), 7.79 (s, 2H), 7.55 (d, *J* = 4.0 Hz, 1H), 7.24 (dd, *J* = 8.0, 4.0 Hz, 1H), 6.40 (d, *J* = 8.0 Hz, 1H),4.71-4.56 (m, 1H), 1.86-1.76 (m, 2H), 1.73-1.54 (m, 5H), 1.44-1.28 (m, 2H), 1.27-1.14 (m, 1H).

### Example 10

### 2-(3,5-Dichloro-4-((1-cyclopropyl-6-oxo-1,6-dihydropyridin-3-yl)oxy)phenyl)-3,5-diox o-2,3,4,5-tetrahydro-1,2,4-triazine-6-carbonitrile

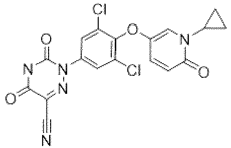

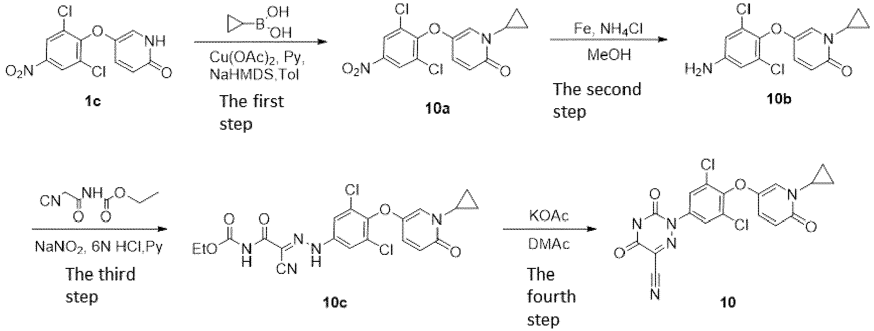

### The first step

### 1-Cyclopropyl-5-(2,6-dichloro-4-nitrophenoxy)pyridin-2(1H)-one

5-(2,6-Dichloro-4-nitrophenoxy)-2-hydroxypyridine 1c (0.6 g, 1.99 mmol) was dissolved in 10 mL of toluene, and added with cyclopropylboronic acid (342.0 mg, 3.99 mmol), copper acetate (361.9 mg, 1.99 mmol), pyridine (0.8 mL, 9.96 mmol) and sodium bis(trimethylsilyl)amide (365.0 mg, 1.99 mmol) for 4 h of reaction at 100°C. The reaction was quenched with water. The reaction solution was concentrated, added with 30 mL of water, and extracted with ethyl acetate (30 mL×3). The organic phases were combined, dried over anhydrous sodium sulfate, and filtered. The filtrate was concentrated under reduced pressure. The obtained residue was purified by silica gel column chromatography to obtain 1-cyclopropyl-5-(2,6-dichloro-4-nitrophenoxy)pyridin-2(1H)-one 10a (265.0 mg, yellow solid) with a yield of 31.2%. MS m/z (ESI): 341.00 [M+1]⁺.

### The second step

### 5-(4-Amino-2,6-dichlorophenoxy)-1-cyclopropylpyridin-2(1H)-one

1-Cyclopropyl-5-(2,6-dichloro-4-nitrophenoxy)pyridin-2(1H)-one 10a (265.0 mg, 0.78 mmol) was dissolved in 4 mL of methanol, and added with saturated ammonium chloride aqueous solution (0.5 mL) and iron powder (173.5 mg, 3.11 mmol) for 4 h of reaction at 100°C. The reaction mixture was filtered. The filtrate was dried by rotating, added with 15 mL of water, and extracted with ethyl acetate (30 mL×3). The organic phases were combined, dried over anhydrous sodium sulfate, and filtered. The filtrate was concentrated under reduced pressure. The obtained residue was purified by silica gel column chromatography to obtain 5-(4-amino-2,6-dichlorophenoxy)-1-cyclopropylpyridin-2(1H)-one 10b (150.0 mg, brown solid), with a yield of 77.6% . MS m/z (ESI): 311.00 [M+1]⁺. ¹H NMR (400 MHz, DMSO-d6) *δ* 7.20 (dd, *J* = 12.0 Hz, 4.0 Hz, 1H), 6.99 (d, *J* = 4.0 Hz, 1H), 6.68 (s, 2H), 6.36 (d, *J* = 12.0 Hz, 1H), 5.69 (s, 2H), 3.31 - 3.21 (m, 1H), 0.97-0.94 (m, 2H), 0.75-0.72 (m, 2H).

### The third step

### Ethyl (E)-(2-cyano-2-(2-(3,5-dichloro-4-((1-cyclopropyl-6-oxo-1,6-dihydropyridin-3-yl)oxy)phenyl)hydrazone) acetyl) carbamate

5-(4-Amino-2,6-dichlorophenoxy)-1-cyclopropylpyridin-2(1H)-one 10b (150.0 mg, 0.482 mmol) was dissolved in 4 mL (6 N) of aqueous solution of hydrochloric acid. The reaction solution was cooled to 0°C, and added with 0.5 mL of aqueous solution of sodium nitrite (49.8 mg, 0.723 mmol) for 30 min of reaction at 0°C. N-cyanoacetylurethane (112.9 mg, 0.723 mmol) was dissolved in 3 mL of water. The mixture was cooled to 0°C, and added with pyridine (2 mL). The former reaction solution was slowly added dropwise to the latter reaction solution for 30 min of reaction at 0°C. The resulting reaction solution was added with 15 mL of water, and a large amount of solids were precipitated. The mixture was filtered. The filter cake was dissolved with isopropanol, and the obtained solution was then concentrated. The above operations were repeated 2 to 3 times to obtain ethyl (E)-(2-cyano-2-(2-(3,5-dichloro-4-((1-cyclopropyl-6-oxo-1,6-dihydropyridin-3-yl)oxy)phenyl)hydrazone)acetyl) carbamate 10c (140.0 mg, reddish-brown solid) crude. MS m/z (ESI): 478.05 [M+1]⁺.

### The fourth step

### 2-(3,5-Dichloro-4-((1-cyclopropyl-6-oxo-1,6-dihydropyridin-3-yl)oxy)phenyl)-3,5-diox o-2,3,4,5-tetrahydro-1,2,4-triazine-6-carbonitrile

Ethyl (E)-(2-cyano-2-(2-(3,5-dichloro-4-((1-cyclopropyl-6-oxo-1,6-dihydropyridin-3-yl) oxy)phenyl)hydrazone)acetyl)carbamate 10c (140.0 mg, 0.29 mmol) was dissolved in N,N-dimethylacetamide (5 mL), and added with potassium acetate (86.1 mg, 0.87 mmol) for 3 h of reaction at 120°C. The reaction solution was added with 20 mL of water, extracted with ethyl acetate (30 mL×3), and washed twice with saturated sodium chloride aqueous solution. The organic phases were combined, dried over anhydrous sodium sulfate, and filtered. The filtrate was concentrated under reduced pressure. The obtained residue was separated by preparative HPLC to obtain 2-(3,5-dichloro-4-((1-cyclopropyl-6-oxo-1,6-dihydropyridin-3-yl)oxy)phenyl)-3,5-dioxo-2,3,4,5-tetrahydro-1,2,4-triazine-6-carbonitrile 10 (37.7 mg, white solid) with a yield of 30.0%. MS m/z (ESI): 432.00 [M+1]⁺. ¹H NMR (400 MHz, DMSO-d6) *δ* 13.29 (s, 1H), 7.78 (s, 2H), 7.31-7.28 (m, 2H), 6.38 (d, *J* = 12.0 Hz,1H), 3.33 - 3.24 (m, 1H), 0.99-0.93 (m,2H), 0.83-0.80 (m, 2H).

### Example 11

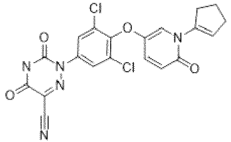

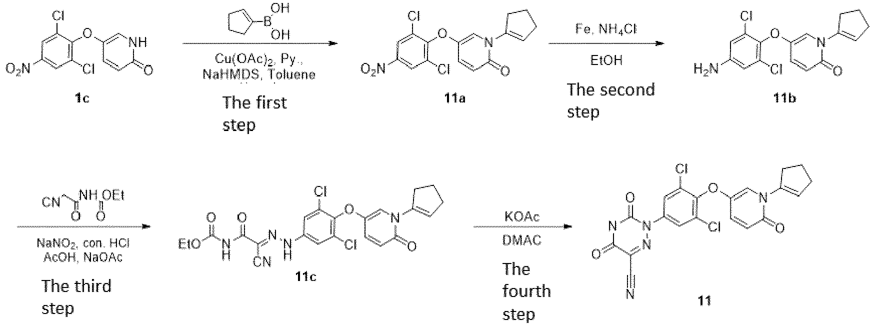

### The first step

### 1-(Cyclopentyl-1-en-1-yl)-5-(2,6-dichloro-4-nitrophenoxy)pyridin-2(1H)-one

5-(2,6-Dichloro-4-nitrophenoxy)pyridin-2(1H)-one 1c (2.00 g, 6.60 mmol) was dissolved in 20 mL of toluene, and then added with pyridine (2.61 g, 33.00 mmol), copper acetate (1.20 g, 6.60 mmol), cyclopentyl-1-en-1-yl boronic acid (2.96 g, 26.40 mmol), and sodium bis(trimethylsilyl)amide (1.22 g, 6.60 mmol) for 8 h of reaction at 100°C. The solvent was removed. The residue was purified by flash silica gel column chromatography to obtain 1-(cyclopentyl-1-en-1-yl)-5-(2,6-dichloro-4-nitrophenoxy)pyridin-2(1H)-one 11a (0.70 g, white solid) with a yield of 28.1%. MS m/z (ESI): 367.00 [M+1]⁺.

### The second step

### 5-(4-Amino-2,6-dichlorophenoxy)-1-(cyclopentyl-1-en-1-yl)pyridin-2(1H)-one

1-(Cyclopentyl-1-en-1-yl)-5-(2,6-dichloro-4-nitrophenoxy)pyridin-2(1H)-one 11a (200.0 mg, 0.54 mmol) and iron powder (152.0 mg, 2.73 mmol) were dissolved in 10.0 mL of ethanol, and added with 2.0 mL of saturated ammonium chloride aqueous solution for 4 h of reaction at 100°C. The solvent was removed. The residue was purified by flash silica gel column chromatography to obtain 5-(4-amino-2,6-dichlorophenoxy)-1-(cyclopentyl-1-en-1-yl)pyridin-2(1H)-one 11b (180.0 mg, white solid) with a yield of 98.1%. MS m/z (ESI): 337.05 [M+1]⁺.

### The third step

### (E)-Ethyl(2-cyano-2-(2-(3,5-dichloro-4-((1-(cyclopentyl-1-en-1-yl)-6-oxy-1,6-dihydrop yridin-3-yl)oxy)phenyl)hydrazino)acetyl)carbamate

5-(4-Amino-2,6-dichlorophenoxy)-1-(cyclopentyl-1-en-1-yl)pyridin-2(1H)-one 11b (180.0 mg, 0.53 mmol) was dissolved in 8.0 mL of acetic acid and 2.0 mL of water. The solution was cooled to 0°C, added with 1.0 mL of concentrated hydrochloric acid, and slowly dropwise added with 2.0 mL of aqueous solution of sodium nitrite (44.0 mg, 0.63 mmol). After the dropwise addition was completed, the reaction solution was stirred for ten minutes, and then added with sodium acetate (23.5 mg, 0.34 mmol) and N-cyanoacetylurethane (165.3 mg, 1.06 mmol). The resulting mixture was removed out of the ice bath, and warmed up to room temperature, and reacted for 12 h. The resulting mixture was added with 15 mL of water, and a solid was generated. The solid was filtered out and dried to obtain (E)-ethyl(2-cyano-2-(2-(3,5-dichloro-4-((1-(cyclopentyl-1-en-1-yl)-6-oxy-1,6-dihydropyridin-3-y 1)oxy)phenyl)hydrazino)acetyl)carbamate 11c (179.0 mg, yellow solid) with a yield of 67.3%. The crude product was used directly in the next step. MS m/z (ESI): 504.05 [M+1]⁺.

### The fourth step

### 2-(3,5-Dichloro-4-((1-(cyclopentyl-1-en-1-yl)-6-oxo-1,6-dihydropyridin-3-yl)oxy)phen yl)-3,5-dioxo-2,3,4,5-tetrahydro-1,2,4-triazine-6-carbonitrile

(E)-Ethyl(2-cyano-2-(2-(3,5-dichloro-4-((1-(cyclopentyl-1-en-1-yl)-6-oxy-1,6-dihydrop yridin-3-yl)oxy)phenyl)hydrazino)acetyl)carbamate 11c (179.0 mg, 0.35 mmol) crude product was dissolved in N,N-dimethylacetamide (5.0 mL), and added with potassium acetate (104.0 mg, 1.06 mmol) for 3 h of reaction at 120°C. The solvent was removed. The residue was purified by preparative HPLC to obtain 2-(3,5-dichloro-4-[(5-cyclopropyl-6-oxo-1-(propan-2-yl)-1,6-dihydropyridin-3-yl)oxy]phenyl)-3,5-dioxo-2,3,4,5-tetrahydro-1,2,4-triazine-6-carbonitrile 11 (22.0 mg, white solid ) with a yield of 13.1%. MS m/z (ESI): 458.00 [M+1]⁺. ¹H NMR (400 MHz, DMSO-*d₆*) δ 13.29 (s, 1H), 7.78 (s, 2H), 7.41 (dd, *J* = 10.0, 3.2 Hz, 1H), 7.27 (d, *J* = 3.2 Hz, 1H), 6.44 (d, *J* = 10.0 Hz, 1H), 6.00-5.96 (s, 1H), 2.70-2.59 (m, 2H), 2.43-2.38 (m, 2H), 2.01-1.89 (m, 2H).

### Example 12

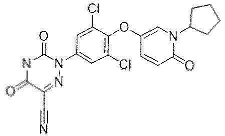

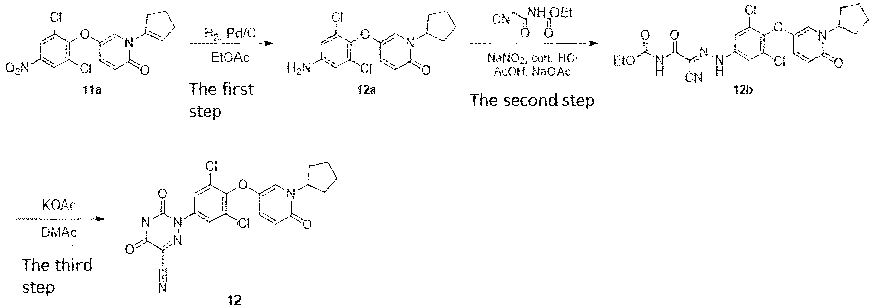

### The first step

### 5-(4-Amino-2,6-dichlorophenoxy)-1-cyclopentylpyridin-2(1H)-one

1-(Cyclopentyl-1-en-1-yl)-5-(2,6-dichloro-4-nitrophenoxy)pyridin-2(1H)-one 11a (500.0 mg, 0.54 mmol) was dissolved in ethyl acetate (10.0 mL), and then added with 10% wet palladium carbon (50.0 mg) for 12 h of reaction at room temperature under hydrogen atmosphere (15 psi). The reaction mixture was filtered. The solvent was removed. The residue was purified by flash silica gel column chromatography to obtain 5-(4-amino-2,6-dichlorophenoxy)-1-cyclopentylpyridin-2(1H)-one 12a (301.0 mg, white solid) with a yield of 60.1%. MS m/z (ESI): 339.05 [M+1]⁺.

### The second step

### Ethyl (E)-(2-cyano-2-(2-(3,5-dichloro-4-((1-cyclopentyl-6-oxo-1,6-dihydropyridine-3-oxy)phenyl)hydrazone)acetyl)carbamate

5-(4-Amino-2,6-dichlorophenoxy)-1-cyclopentylpyridin-2(1H)-one 12a (180.0 mg, 0.53 mmol) was dissolved in 8.0 mL of acetic acid and 2.0 mL of water. The solution was cooled to 0°C, then added with 1.0 mL of concentrated hydrochloric acid, and slowly dropwise added with 2.0 mL of aqueous solution of sodium nitrite (44.0 mg, 0.63 mmol). After the dropwise addition was completed, the solution was stirred for ten minutes, and added with sodium acetate (23.53 mg, 0.34 mmol) and N-cyanoacetylurethane (165.3 mg, 1.06 mmol). The obtained mixture was removed out of the ice bath, and warmed up to room temperature for 12 h of reaction. The resulting mixture was added with 15 mL of water, and a solid was generated. The solid was filtered out, and dried to obtain ethyl (E)-(2-cyano-2-(2-(3,5-dichloro-4-((1-cyclopentyl-6-oxo-1,6-dihydropyridine-3-oxy)phenyl)hydrazone)acetyl)carbamate 12b (189.0 mg, yellow solid) with a yield of 24.0%. MS m/z (ESI): 506.10 [M+1]⁺.

### The third step

### 2-(3,5-Dichloro-4-((1-cyclopentyl-6-oxo-1,6-dihydropyridine-3-oxy)phenyl)-3,5-dioxo-2,3,4,5-tetrahydro-1,2,4-triazine-6-carbonitrile

Ethyl (E)-(2-cyano-2-(2-(3,5-dichloro-4-((1-cyclopentyl-6-oxo-1,6-dihydropyridine-3-oxy)phenyl)hydrazone)acetyl)carbamate 12b (189.0 mg, 0.37 mmol) crude product was dissolved in N,N-dimethylacetamide (5.0 mL), and added with potassium acetate (109.0 mg, 1.12 mmol) for 3 h of reaction at 120°C. The solvent was removed. The residue was purified by preparative HPLC to obtain 2-(3,5-dichloro-4-((1-cyclopentyl-6-oxo-1,6-dihydropyridine-3-oxy)phenyl)-3,5-dioxo-2,3,4,5-tetrahydro-1,2,4-triazine-6-carbonitrile 12 (120.0 mg, white solid) with a yield of 70.1%. MS m/z (ESI): 460.00 [M+1]⁺. ¹H NMR (400 MHz, DMSO-d₆) δ 13.29 (s, 1H), 7.79 (s, 2H), 7.44 (d, *J* = 3.2 Hz, 1H), 7.27 (dd, *J* = 10.0, 3.2 Hz, 1H), 6.39 (d, *J* = 10.0 Hz, 1H), 5.04-4.98 (m, 1H), 2.00-1.90 (m, 2H), 1.81 - 1.53 (m, 6H).

### Example 13

### 2-(3,5-Dichloro-4-((1-(cyclohex-1-en-1-yl)-6-oxo-1,6-dihydropyridin-3-yl)oxy)phenyl)-3,5-dioxo-2,3,4,5-tetrahydro-1,2,4-triazine-6-carbonitrile

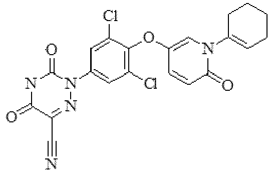

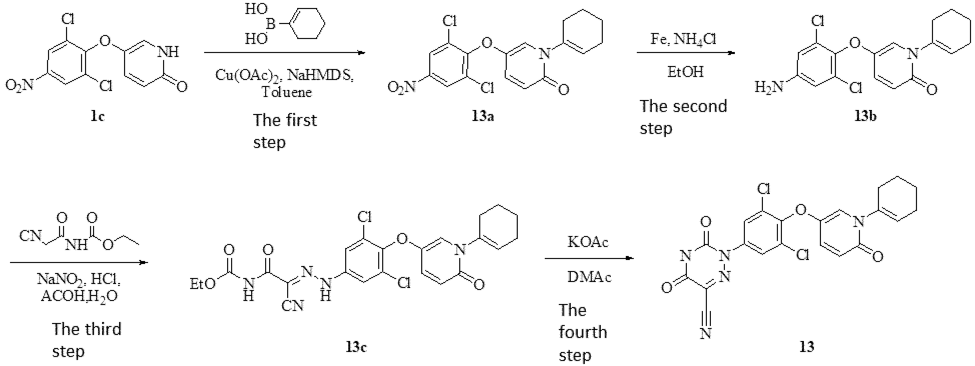

### The first step

### 1-(Cyclohex-1-en-1-yl)-5-(2,6-dichloro-4-nitrophenoxy)pyridin-2(1H)-one

5-(2,6-Dichloro-4-nitrophenoxy)-2-hydroxypyridine 1c (2.00 g, 6.6 mmol) was dissolved in 40 mL of toluene, and added with cyclohexenylboronic acid (3.60 g, 26.6 mmol), copper acetate (1.20 g, 6.6 mmol), sodium bis(trimethylsilyl)amide (1.20 g, 6.6 mmol), and pyridine (2.68 mL, 33.0 mmol) for 4 h of reaction at 80°C. The reaction solution was concentrated under reduced pressure to remove solvent. The residue was purified by flash column chromatography to obtain 1-(cyclohex-1-en-1-yl)-5-(2,6-dichloro-4-nitrophenoxy)pyridin-2(1H)-one 13a (720.0 m, yellow solid) with a yield of 28.5%. MS m/z (ESI): 381.00 [M+1]⁺.

### The second step

### 5-(4-Amino-2,6-dichlorophenoxy)-1-(cyclohex-1-en-1-yl)pyridin-2(1H)-one

1-(Cyclohex-1-en-1-yl)-5-(2,6-dichloro-4-nitrophenoxy)pyridin-2(1H)-one 13a (200.0 mg, 0.53 mmol) and reduced iron powder (147.0 mg) were dissolved in a mixed solution of 1 mL of ethanol and 1 mL of saturated ammonium chloride solution. The mixture was refluxed for 2 hours, concentrated under reduced pressure to remove ethanol, and extracted with dichloromethane (2 mL×3). The filtrate was dried over anhydrous sodium sulfate, filtered and concentrated. The residue was purified by column chromatography machine to obtain 5-(4-amino-2,6-dichlorophenoxy)-1-(cyclohex-1-en-1-yl)pyridin-2(1H)-one 13b (126.0 mg, yellow oil) with a yield of 68.4%. MS m/z (ESI): 351.00 [M+1]⁺.

### The third step

### (E)-Ethyl(2-cyano-2-(2-(3,5-dichloro-4-((1-(cyclohex-1-en-1-yl)-6-oxo-1,6-dihydropyri din-3-yl)oxy)phenyl)hydrazine)acetyl)carbamate

5-(4-Amino-2,6-dichlorophenoxy)-1-(cyclohex-1-en-1-yl)pyridin-2(1H)-one 13b (126.0 mg, 0.36 mmol) was dissolved in a mixed solvent consisting of 3.2 mL of acetic acid, 0.8 mL of water and 0.4 mL of concentrated hydrochloric acid. The solution was added with sodium nitrite (31.0 mg, 0.44 mmol) at 0°C for 10 min of reaction. Then the reaction solution was added with sodium acetate (90.0 mg, 1.10 mmol) for 10 min of reaction. The obtained mixture was added with N-cyanoacetylurethane (69.0 mg, 0.44 mmol), and warmed up to room temperature for 72 h of reaction. The reaction mixture was added with 5 mL of water, filtered, and dried to obtain a crude product (E)-ethyl(2-cyano-2-(2-(3,5-dichloro-4-((1-(cyclohex-1-en-1-yl)-6-oxo-1,6-dihydropyridin-3-yl)oxy)phenyl)hydrazine)acetyl)carbamate 13c (70.0 mg, yellow solid) with a yield of 37.0%. MS m/z (ESI): 518.09 [M+1]⁺.

### The fourth step

### 2-(3,5-Dichloro-4-((1-(cyclohex-1-en-1-yl)-6-oxo-1,6-dihydropyridin-3-yl)oxy)phenyl)-3,5-dioxo-2,3,4,5-tetrahydro-1,2,4-triazine-6-carbonitrile

(E)-Ethyl(2-cyano-2-(2-(3,5-dichloro-4-((1-(cyclohex-1-en-1-yl)-6-oxo-1,6-dihydropyri din-3-yl)oxy)phenyl)hydrazine)acetyl)carbamate 13c (55.0 mg, 0.11 mmol) was dissolved in 2.0 mL of N,N-dimethylacetamide, and added with potassium acetate (21.0 mg, 0.22 mmol) for 2 h of reaction at 120°C. The reaction solution was concentrated under reduced pressure to remove the solvent. The residue was purified by preparative chromatography to obtain 2-(3,5-dichloro-4-((1-(cyclohex-1-en-1-yl)-6-oxo-1,6-dihydropyridin-3-yl)oxy)phenyl)-3,5-dioxo -2,3,4,5-tetrahydro-1,2,4-triazine-6-carbonitrile 13 (15.0 mg, white solid) with a yield of 30.0%. MS m/z (ESI): 472.05 [M+1]⁺. ¹H NMR (400 MHz, DMSO-d*₆*) δ 7.77 (s, 2H), 7.43 (d, *J* = 4.0 Hz, 1H), 7.21 (dd, *J* = 8.0, 4.0 Hz, 1H), 6.41 (d, *J* = 8.0 Hz, 1H),5.72-5.62 (m, 1H), 2.24-2.18 (m, 2H), 2.15-2.08 (m, 2H), 1.72-1.64 (m, 2H), 1.60-1.52 (m, 2H).

### Example 14

### 2-(3,5-dichloro-4-((deuterated 1-cyclopentyl-6-oxo-1,6-dihydropyridine-3-oxy) phenyl)-3,5-dioxo-2,3,4,5-tetrahydro-1,2,4-triazine-6-carbonitrile

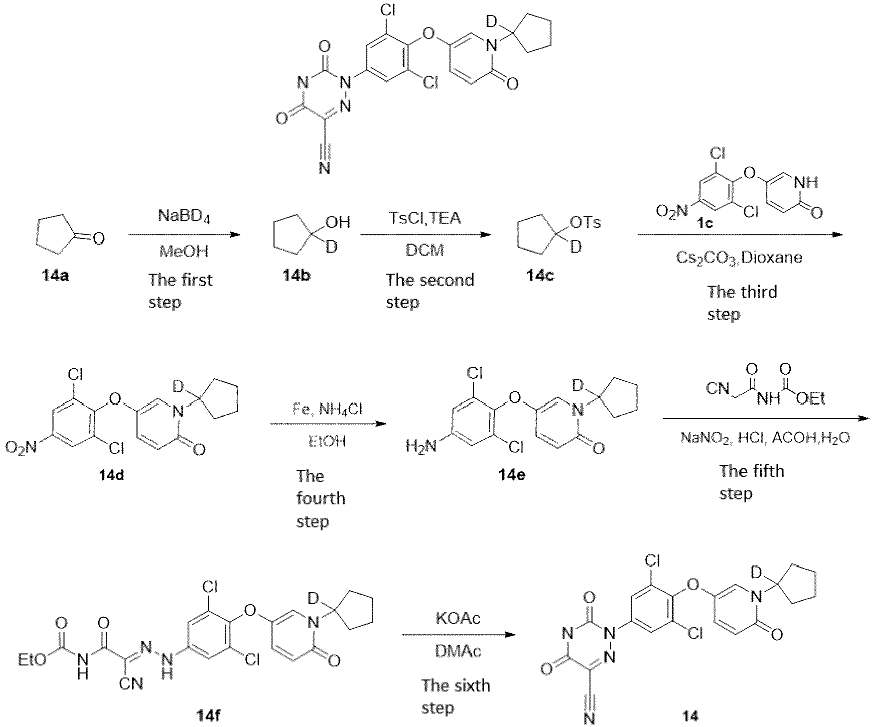

### The first step

### Deuterated cyclopentanol

Cyclopentanone 14a (5.00 g, 59.44 mmol) was dissolved in 100.0 mLof methanol, and added with sodium borodeuteride (6.20 g, 148.60 mmol) for 2 h of reaction at room temperature. The reaction was quenched with 2.0 mL of 1 N hydrochloric acid. The reaction solution was concentrated under reduced pressure to remove the solvent, and added with 100 mL of dichloromethane. The organic phase was dried over anhydrous sodium sulfate, and filtered. The filtrate was concentrated to obtain deuterated cyclopentanol 14b (4.86 g, colorless oil) with a yield of 93.9%.

### The second step

### Deuterated cyclopentyl 4-toluene-1-sulfonate

Deuterated cyclopentanol 14b (4.86 g, 55.8 mmol) was dissolved in 100.0 mL of dichloromethane, and added with p-toluenesulfonyl chloride (10.64 g, 55.8 mmol), 4-dimethylaminopyridine (0.68 g, 5.58 mmol) and triethylamine (16.94 g, 167.4 mmol). The mixture was stirred at room temperature for 12 hours. The solvent was removed. The residue was purified by column to obtain deuterated cyclopentyl 4-toluene-1-sulfonate 14c (3.20 g, colorless oil) with a yield of 23.5%. MS m/z (ESI): 240.31 [M+1]⁺.

### The third step

### Deuterated 1-cyclopentyl-5-(2,6-dichloro-4-nitrophenoxy)-1,2-dihydropyridin-2-one

5-(2,6-Dichloro-4-nitrophenoxy)-2-hydroxypyridine 1c (1.50 g, 4.98 mmol) was dissolved in 30.0 mL of 1,4-dioxane, and added with deuterated cyclopentyl 4-toluene-1-sulfonate 14c (2.4 g, 9.96 mmol) and cesium carbonate (4.87 g, 14.94 mmol) for 2 h of reaction at 100°C. The reaction solution was concentrated under reduced pressure to remove the solvent. The residue was purified by column chromatography machine to obtain deuterated 1-cyclopentyl-5-(2,6-dichloro-4-nitrophenoxy)-1,2-dihydropyridin-2-one 14d (1.0 g, yellow solid) with a yield of 52.4%. MS m/z (ESI): 370.21 [M+1]⁺.

### The fourth step

### Deuterated 1-cyclopentyl-5-(2,6-dichloro-4-aminophenoxy)-1,2-dihydropyridin-2-one

Deuterated 1-cyclopentyl-5-(2,6-dichloro-4-nitrophenoxy)-1,2-dihydropyridin-2-one 14d (985.0 mg, 2.67 mmol) was dissolved in a mixed solvent of 10.0 mL of saturated ammonium chloride and 10.0 mL of ethanol, and added with reduced iron powder (750.0 mg, 13.4 mmol) for 2 h of reaction at 100°C. The reaction solution was concentrated under reduced pressure to remove ethanol, and extracted with dichloromethane (20.0 mL×3). The organic phase was dried over anhydrous sodium sulfate. The filtrate was filtered and concentrated. The crude product was separated and purified by flash silica gel column chromatography to obtain deuterated 1-cyclopentyl-5-(2,6-dichloro-4-aminophenoxy)-1,2-dihydropyridin-2-one 14e (720.0 mg, pale yellow oil) with a yield of 73.0%. MS m/z (ESI): 340.07 [M+1]⁺.

### The fifth step

### (E)-Ethyl(2-cyano-2-(2-(3,5-dichloro-4-((deuterated 1-cyclopentyl-6-oxo-1,6-dihydropyridin-3-yl)oxy)phenyl)hydrazine)acetyl)carbamate

Deuterated 1-cyclopentyl-5-(2,6-dichloro-4-aminophenoxy)-1,2-dihydropyridin-2-one 14e (360.0 mg, 0.98 mmol) was dissolved in a mixed solvent consisting of 4.8 mL of acetic acid, 1.2 mL of water and 0.6 mL of concentrated hydrochloric acid, and added with sodium nitrite (81.1 mg, 1.18 mmol) at 0°C for 10 min of reaction. Then the reaction solution was added with sodium acetate (241.2 mg, 2.94 mmol) for 10 min of reaction. Then the reaction mixture was added with N-cyanoacetylurethane (183.6 mg, 1.18 mmol) and warmed up to room temperature for 72 h of reaction. The obtained mixture was added with 10.0 mL of water, filtered, and dried to obtain a crude product (E)-ethyl(2-cyano-2-(2-(3,5-dichloro-4-((deuterated 1-cyclopentyl-6-oxo-1,6- dihydropyridin-3-yl)oxy)phenyl)hydrazine)acetyl)carbamate 14f (620.0 mg, yellow solid) with a yield of 92.0 %. The crude product was used directly in the next step. MS m/z (ESI): 507.10 [M+1]⁺.

### The six step

### 2-(3,5-Dichloro-4-((deuterated 1-cyclopentyl-6-oxo-1,6-dihydropyridine-3-oxy) phenyl)-3,5-dioxo-2,3,4,5-tetrahydro-1,2,4-triazine-6-carbonitrile

(E)-ethyl(2-cyano-2-(2-(3,5-dichloro-4-((deuterated 1-cyclopentyl-6-oxo-1,6-dihydropyridin-3-yl)oxy)phenyl)hydrazine)acetyl)carbamate 14f (570.0 mg, 0.90 mmol) was dissolved in 10.0 mL of N,N-dimethylacetamide, and added with potassium acetate (176.7 mg, 1.80 mmol) for 2 h of reaction at 120°C. The reaction solution was concentrated under reduced pressure to remove the solvent. The crude product was purified by preparative HPLC chromatography to obtain 2-(3,5-dichloro-4-((deuterated 1-cyclopentyl-6-oxo-1,6-dihydropyridine-3-oxy)phenyl)-3,5-dioxo-2,3,4,5-tetrahydro-1,2,4-triazi ne-6-carbonitrile 14 (70.0 mg, white solid) with a yield of 17.0 %. MS m/z (ESI): 461.06 [M+1]⁺. ¹H NMR (400 MHz, DMSO-d*₆*) δ 7.79 (s, 2H), 7.43 (d, *J* = 4.0 Hz, 1H), 7.24 (dd, *J* = 4.0, 8.0 Hz, 1H), 6.40 (d, *J* = 8.0 Hz, 1H),1.97-1.95 (m, 2H), 1.76-1.59 (m, 6H).

### Example 15

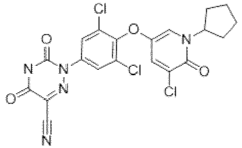

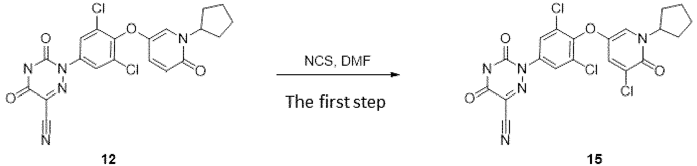

### The first step

### 2-(3,5-Dichloro-4-[(5-chloro-1-cyclopentyl-6-oxo-1,6-dihydropyridin-3-yl)oxy]phenyl) -3,5-dioxo-2,3,4,5-tetrahydro-1,2,4-triazine-6-carbonitrile

2-(3,5-Dichloro-4-((1-cyclopentyl-6-oxo-1,6-dihydropyridine-3-oxy)phenyl)-3,5-dioxo-2,3,4,5-tetrahydro-1,2,4-triazine-6-carbonitrile 12 (30.0 mg, 0.06 mmol) was dissolved in 3.0 mL of N,N-dimethylformamide, and then added with N-chlorosuccinimide (12.0 mg, 0.09 mmol) for 1 h of reaction at 70°C. The solvent was removed. The residue was purified by preparative HPLC to obtain 2-(3,5-dichloro-4-[(5-chloro-1-cyclopentyl-6-oxo-1,6-dihydropyridin-3-yl)oxy]phenyl)-3,5-dioxo-2,3,4,5-tetrahydro-1,2,4-triazine-6-carbonitrile 15 (13.0 mg, white solid) with a yield of 43.1%. MS m/z (ESI): 493.95 [M+1]⁺. ¹H NMR (400 MHz, DMSO-*d₆*) δ 13.30 (s, 1H), 7.82-7.76 (m, 3H), 7.52 (d, *J* = 2.8 Hz, 1H), 5.09-5.00 (m, 1H), 2.00-1.94 (m, 2H), 1.80-1.68 (m, 4H), 1.64-1.57 (m, 2H).

### Example 16

2-(3,5-Dichloro-4-((1-cyclopentyl-5-fluoro-6-oxo-1,6-dihydropyridin-3-yl)oxy)phenyl)-3,5-dioxo-2,3,4,5-tetrahydro-1,2,4-triazine-6-carbonitrile

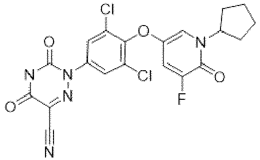

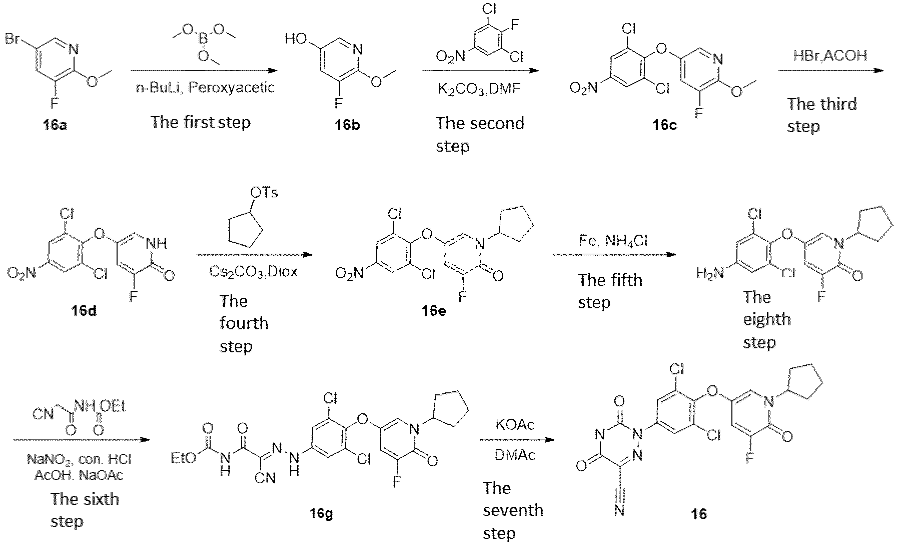

### The first step

### 5-Fluoro-6-methoxypyridin-3-ol

5-Bromo-3-fluoro-2-methoxypyridine 16a (2.00 g, 9.71 mmol) and trimethyl borate (2.20 g, 21.17 mmol) were dissolved in 60.0 mL of anhydrous tetrahydrofuran, replaced with nitrogen three times, and dropwise added with n-butyllithium (8.74 mL, 2.5 N, 21.86 mmol) at -78°C for 2 h of reaction at -78°C. The reaction solution was then added with 20% peracetic acid (8.10 g, 21.30 mmol) for 10 min of reaction. The reaction mixture was then warmed up to 0°C, stirred for 1 hour, and dropwise added with saturated sodium sulfite solution at 0°C until the temperature was no longer raised. Then the mixture was added with 50 mL of ethyl acetate, dried over anhydrous sodium sulfate, and filtered. The filtrate was concentrated. The residue was purified with a column chromatography machine to obtain 5-fluoro-6-methoxypyridin-3-ol 16b (755.0 mg, colorless oil) with a yield of 46.5 %. MS m/z (ESI): 144.11 [M+1]⁺.

### The second step

### 5-(2,6-Dichloro-4-nitrophenoxy)-3-fluoro-2-methoxypyridine

5-Fluoro-6-methoxypyridin-3-ol 16b (755.0 mg, 4.52 mmol) was dissolved in 10.0 mL of N,N-dimethylformamide, and added with 1,3-dichloro-2-fluoro-5-nitrobenzene (949.2 mg, 4.52 mmol) and potassium carbonate (1874.1 mg, 13.56 mmol) for 3 h of reaction at room temperature. The reaction solution was added with 80 mL of water, and filtered. The solid was concentrated under reduced pressure and dried by rotating to obtain 5-(2,6-dichloro-4-nitrophenoxy)-3-fluoro-2-methoxypyridine 16c (1.48 g, yellow solid) with a yield of 94.96%. MS m/z (ESI): 334.10 [M+1]⁺.

### The third step

### 5-(2,6-Dichloro-4-nitrophenoxy)-3-fluoropyridin-2-ol

5-(2,6-Dichloro-4-nitrophenoxy)-3-fluoro-2-methoxypyridine 16c (1.43 g, 4.15 mmol) was dissolved in a mixed solvent of 15.0 mL of acetic acid and 15.0 mL of hydrobromic acid for 2 h of reaction at 100°C. The reaction solution was concentrated under reduced pressure to remove the solvent. The residue was cooled to room temperature, filtered, and dried under reduced pressure to obtain 5-(2,6-dichloro-4-nitrophenoxy)-3-fluoropyridin-2-ol 16d (1.30 g, yellow solid) with a yield of 97.0 %. MS m/z (ESI): 320.07 [M+1]⁺.

### The fourth step

### 1-Cyclopentyl-5-(2,6-dichloro-4-nitrophenoxy)-3-fluoro-1,2-dihydropyridin-2-one

5-(2,6-Dichloro-4-nitrophenoxy)-3-fluoropyridin-2-ol 16d (1.25 g, 3.85 mmol) was dissolved in 25.0 mL of 1,4-dioxane, and added with cyclopentyl 4-toluene-1-sulfonate (2.78 g, 11.55 mmol) and cesium carbonate (3.76 g, 11.55 mmol) for 2 h of reaction at 100°C. The reaction solution was concentrated under reduced pressure to remove the solvent. The residue was purified by column chromatography machine to obtain 1-cyclopentyl-5-(2,6-dichloro-4-nitrophenoxy)-3-fluoro-1,2-dihydropyridin-2-one 16e (520.0 mg, yellow solid) with a yield of 34.3 %. MS m/z (ESI): 388.19 [M+1]⁺.

### The fifth step

### 5-(4-Amino-2,6-dichlorophenoxy)-1-cyclopentyl-3-fluoro-1,2-dihydropyridin-2-one

1-Cyclopentyl-5-(2,6-dichloro-4-nitrophenoxy)-3-fluoro-1,2-dihydropyridin-2-one 16e (470.0 mg, 1.19 mmol) was dissolved in a mixed solvent of 10.0 mL of saturated ammonium chloride and 10.0 mL of ethanol, and added with reduced iron powder (332.3 mg, 5.95 mmol) for 2 h of reaction at 100°C. The reaction solution was concentrated under reduced pressure to remove ethanol, and extracted with dichloromethane (20.0 mL×3). The organic phase was dried over anhydrous sodium sulfate, filtered and concentrated. The crude product was purified by flash silica gel column to obtain 5-(4-amino-2,6-dichlorophenoxy)-1-cyclopentyl-3-fluoro-1,2-dihydropyridin-2-one 16f (288.0 mg, light yellow oil) with a yield of 53.3%. MS m/z (ESI): 357.21 [M+1]⁺.

### The sixth step

### N-[(E)-cyanoethyl(2-(3,5-dichloro-4-[(1-cyclopentyl-5-fluoro-6-oxo-1,6-dihydropyridin -3-yl)oxy]phenyl)hydrazin-1-ylidene)carbonyl]carbamate

5-(4-Amino-2,6-dichlorophenoxy)-1-cyclopentyl-3-fluoro-1,2-dihydropyridin-2-one 16f (144.0 mg, 0.32 mmol) was dissolved in a mixed solvent consisting of 3.2 mL of acetic acid, 0.8 mL of water and 0.4 mL of concentrated hydrochloric acid, and added with sodium nitrite (26.5 mg, 0.38 mmol) at 0°C for 10 min of reaction. Then the reaction solution was added with sodium acetate (78.8 mg, 0.96 mmol) for 10 min of reaction. The obtained mixture was added with N-cyanoacetylurethane (59.9 mg, 0.38 mmol), and warmed up to room temperature for 72 h of reaction. The reaction mixture was added with 5.0 mL of water, filtered, and dried to obtain a crude product N-[(E)-cyanoethyl(2-(3,5-dichloro-4-[(1-cyclopentyl-5-fluoro-6-oxo-1,6-dihydropyridin-3-yl)oxylphenyl)hydrazin-1-ylidene)carbonyl]carbamate 16g (183.0 mg, yellow solid) with a yield of 79.0%. MS m/z (ESI): 524.33 [M+1]⁺.

### The seventh step

### 2-(3,5-Dichloro-4-[(1-cyclopentyl-5-fluoro-6-oxo-1,6-dihydropyridin-3-yl)oxy]phenyl)-3,5-dioxo-2,3,4,5-tetrahydro-1,2,4-triazine-6-carbonitrile

N-[(E)-cyanoethyl(2-(3,5-dichloro-4-[(1-cyclopentyl-5-fluoro-6-oxo-1,6-dihydropyridin -3-yl)oxy]phenyl)hydrazin-1-ylidene)carbonyl]carbamate 16g (183.0mg, 0.25mmol) was dissolved in 3.0mL of N,N-dimethylacetamide, and added with potassium acetate (73.61 mg, 0.75 mmol) for 2 h of reaction at 120°C. The reaction solution was concentrated under reduced pressure to remove the solvent. The residue was purified by preparative chromatography to obtain 2-(3,5-dichloro-4-[(1-cyclopentyl-5-fluoro-6-oxo-1,6-dihydropyridin-3-yl)oxy]phenyl)-3,5-dioxo -2,3,4,5-tetrahydro-1,2,4-triazine-6-carbonitrile 16 (70.0 mg, white solid) with a yield of 31.2 %. MS m/z (ESI): 478.26 [M+1]⁺. ¹H NMR (400 MHz, DMSO-d*₆*) δ 7.78 (s, 2H), 7.56 (d, *J* = 4.0 Hz, 1H),7.30 (d, *J* = 4.0 Hz, 1H), 5.08-5.04 (m, 2H),1.97-1.95 (m, 2H), 1.74-1.60 (m, 6H).

### Example 17

### 2-(3,5-Dichloro-4-[(1-cyclopentyl-5-methyl-6-oxo-1,6-dihydropyridin-3-yl)oxy]phenyl) -3,5-dioxo-2,3,4,5-tetrahydro-1,2,4-triazine-6-carbonitrile

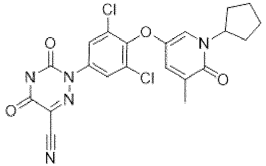

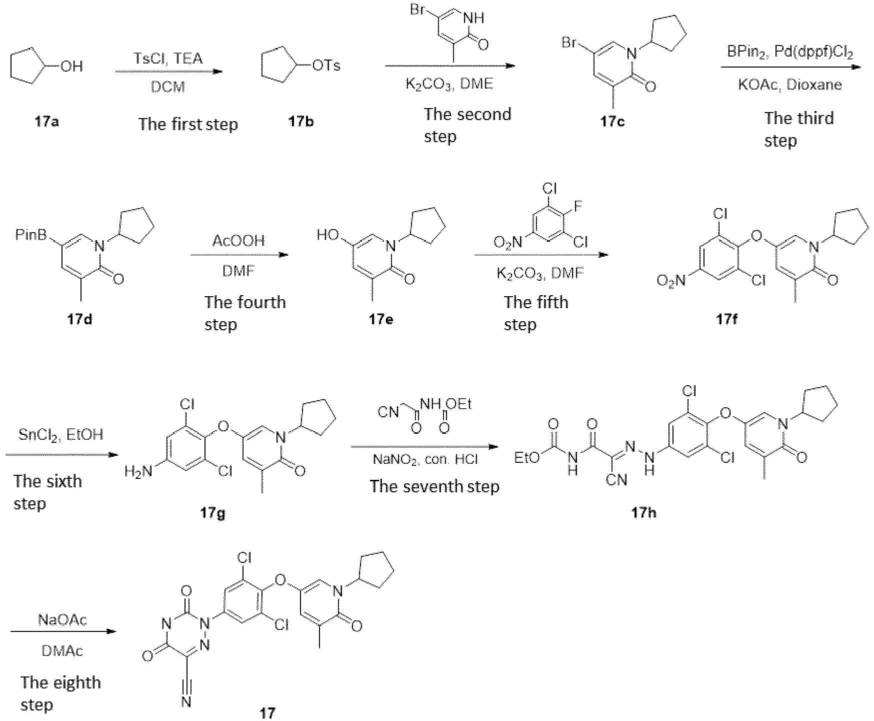

### The first step

### Cyclopentyl 4-toluene-1-sulfonate

Cyclopentanol 17a (10.00 g, 116.10 mmol) was dissolved in 100 mL of dichloromethane, and then added with p-toluenesulfonyl chloride (22.13 g, 116.10 mmol), 4-dimethylaminopyridine (1.42 g, 11.61 mmol), and triethylamine (29.37 g, 290.25 mmol). The mixture was stirred at room temperature for 12 hours. After the solvent was removed, the residue was subjected to flash silica gel column chromatography to obtain cyclopentyl 4-toluene-1-sulfonate 17b (13.12 g, colorless oil) with a yield of 42.8%.

### The second step

### 5-Bromo-1-cyclopentyl-3-methyl-1,2-dihydropyridin-2-one

Cyclopentyl 4-toluene-1-sulfonate 17b (3.77 g, 15.96 mmol) was dissolved in 10 mL of 1,2-dimethoxyethane, and then added with 5-bromo-3-methylpyridin-2-one (1.00 g, 5.32 mmol) and potassium carbonate (2.21 g, 15.96 mmol), and the mixture was heated up to 80°C for 12 h of reaction. The solvent was removed. The residue was purified by flash silica gel column chromatography to obtain 5-bromo-1-cyclopentyl-3-methyl-1,2-dihydropyridin-2-one 17c (0.92 g, white solid) with a yield of 62.1%. MS m/z (ESI): 256.05 [M+1]⁺. ¹H NMR (400 MHz, CDCl₃) δ 7.31 (d, *J* = 2.4 Hz, 1H), 7.22 (d, *J* = 1.2 Hz, 1H), 5.32-5.24 (m, 1H), 2.23 - 2.11 (m, 5H), 1.88-1.84 (m, 2H), 1.78 - 1.71 (m, 2H), 1.65-1.60 (m, 2H).

### The third step

### 1-Cyclopentyl-3-methyl-5-(tetramethyl-1,3,2-dioxin-2-yl)-1,2-dihydropyridin-2-one

5-Bromo-1-cyclopentyl-3-methyl-1,2-dihydropyridin-2-one 17c (300.0 mg, 1.08 mmol), bis(pinacolato)diboron (411.3 mg, 1.62 mmol), potassium acetate (317.9 mg, 3.24 mmol), and [1,1'-bis(diphenylphosphino)ferrocene]dichloropalladium (II) (79.0 mg, 0.11 mmol) were dissolved in 10 mL of 1,4-dioxane. The mixture was replaced with nitrogen and heated to 100°C for 6 h of reaction. The solvent was removed. The residue was purified by flash silica gel column chromatography to obtain 1-cyclopentyl-3-methyl-5-(tetramethyl-1,3,2-dioxin-2-yl)-1,2-dihydropyridin-2-one 17d (343.0 mg, colorless oil), with a yield of 71.2%. MS m/z (ESI): 304.15 [M+1]⁺.

### The fourth step

### 1-Cyclopentyl-5-hydroxy-3-methyl-1,2-dihydropyridin-2-one

1-Cyclopentyl-3-methyl-5-(tetramethyl-1,3,2-dioxin-2-yl)-1,2-dihydropyridin-2-one 17d (100.0 mg, 0.22 mmol) was dissolved in 5.0 mL of N,N-dimethylformamide. The mixture was cooled to 0°C, and dropwise added with peracetic acid (167.3 mg, 0.44 mmol). After the dropwise addition was completed, the resulting mixture was reacted at 0°C for 1 hour. Then the obtained mixture was added with 1.0 mL of saturated sodium sulfite aqueous solution to obtain a reaction solution of 1-cyclopentyl-5-hydroxy-3-methyl-1,2-dihydropyridin-2-one 17e, which was directly used in the next step without purification. MS m/z (ESI): 194.15 [M+1]⁺.

### The fifth step

### 1-Cyclopentyl-5-(2,6-dichloro-4-nitrophenoxy)-3-methyl-1,2-dihydropyridin-2-one

1,3-dichloro-2-fluoro-5-nitrobenzene (46.2 mg, 0.22 mmol) and potassium carbonate (304.0 mg, 2.20 mmol) were dissolved in the reaction solution of 1-cyclopentyl-5-hydroxy-3-methyl-1,2-dihydropyridin-2-one 17e for 3 h of reaction at room temperature. The solvent was removed. The residue was purified by flash silica gel column chromatography to obtain 1-cyclopentyl-5-(2,6-dichloro-4-nitrophenoxy)-3-methyl-1,2-dihydropyridin-2-one 17f (0.07 g, yellow oil) with a yield of 59.8%. MS m/z (ESI): 383.00 [M+1]⁺.

### The sixth step

### 5-(4-Amino-2,6-dichlorophenoxy)-1-cyclopentyl-3-methyl-1,2-dihydropyridin-2-one

1-Cyclopentyl-5-(2,6-dichloro-4-nitrophenoxy)-3-methyl-1,2-dihydropyridin-2-one 17f (70.0 mg, 0.13 mmol) was dissolved in 5.0 mL of ethanol, and then added with tin dichloride dihydrate (117.3 mg, 0.52 mmol), and the mixture was heated up to 80°C for 2 h of reaction. The pH value was adjusted to greater than 7 with aqueous solution of sodium hydroxide. The solvent was removed. The residue was purified by flash silica gel column chromatography to obtain 5-(4-amino-2,6-dichlorophenoxy)-1-cyclopentyl-3-methyl-1,2-dihydropyridin-2-one 17g (70.0 mg, white solid) with a yield of 98.0%. MS m/z (ESI): 353.10 [M+1]⁺.

### The seventh step

### Ethyl (E)-(2-cyano-2-(2-(3,5-dichloro-4-((1-cyclopentyl-5-methyl-6-oxo-1,6-dihydropyridin-3-1)oxy)phenyl)hydrazine)acetyl)carbamate

5-(4-Amino-2,6-dichlorophenoxy)-1-cyclopentyl-3-methyl-1,2-dihydropyridin-2-one 17g (70.0 mg, 0.18 mmol) was dissolved in 3 mL of acetic acid and 1 mL of water. The mixture was cooled to 0°C, then added with 0.5 mL of concentrated hydrochloric acid, and slowly dropwise added with 1 mL of aqueous solution of sodium nitrite (13.6 mg, 0.20 mmol). After the dropwise addition was completed, the obtained mixture was stirred for ten minutes, and then added with sodium acetate (44.3 mg, 0.54 mmol) and N-cyanoacetylurethane (28.1 mg, 0.18 mmol). The mixture was removed out of the ice bath, and warmed up to room temperature for 12 h of reaction. The reaction mixture was added with 10 mL of water. The solid was filtered out and dried to obtain ethyl (E)-(2-cyano-2-(2-(3,5-dichloro-4-((1-cyclopentyl-5-methyl-6-oxo-1,6-dihydropyridin-3-1)oxy)phenyl)hydrazine)acetyl)carbamate 17h (100.0 mg, yellow solid) with a yield of 51.0%. The crude product was used directly in the next step. MS m/z (ESI): 520.00 [M+1]⁺.

### The eighth step

### 2-(3,5-Dichloro-4-[(1-cyclopentyl-5-methyl-6-oxo-1,6-dihydropyridin-3-yl)oxy]phenyl) -3,5-dioxo-2,3,4,5-tetrahydro-1,2,4-triazine-6-carbonitrile

Ethyl (E)-(2-cyano-2-(2-(3,5-dichloro-4-((1-cyclopentyl-5-methyl-6-oxo-1,6-dihydropyridin-3-1)oxy)phenyl)hydrazine)acetyl)carbamate 17h (100.0 mg, 0.09 mmol) crude product was dissolved in N,N-dimethylacetamide (5.0 mL), and added with potassium acetate (26.5 mg, 0.27 mmol) for 3 h of reaction at 120°C. The solvent was removed. The residue was purified by preparative HPLC to obtain 2-(3,5-dichloro-4-[(1-cyclopentyl-5-methyl-6-oxo-1,6-dihydropyridin-3-yl)oxy]phenyl)-3,5-dioxo-2,3,4,5-tetrahydro-1,2,4-triazine-6-carbonitrile 17 (8.0 mg, white solid) with a yield of 17.6 %. MS m/z (ESI): 474.00 [M+1]⁺. ¹H NMR (400 MHz, DMSO-*d₆*) δ 13.30 (s, 1H), 7.78 (s, 2H), 7.22 (s, 2H), 5.05-5.15 (m, 1H), 2.01 (s, 3H), 1.98-1.94 (m,2H), 1.78-1.70 (s, 2H), 1.68-1.60 (m, 2H).

### Example 18

### 2-(3,5-Dichloro-4-((1-cyclopropyl-5-methyl-6-oxo-1,6-dihydropyridin-3-yl)oxy)phenyl )-3,5-dioxo-2,3,4,5-tetrahydro-1,2,4-triazine-6-carbonitrile

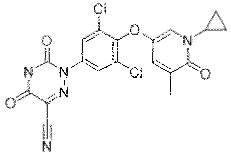

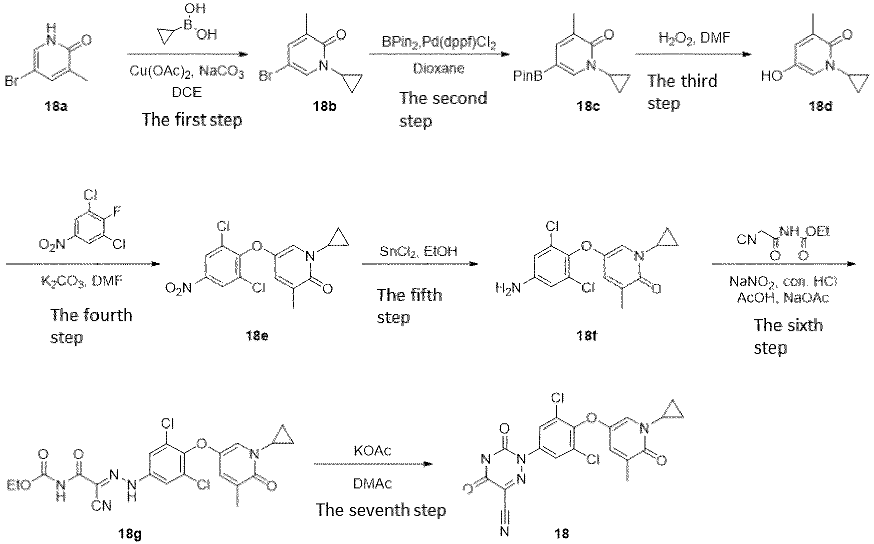

### The first step

### 5-Bromo-1-cyclopropyl-3-methylpyridin-2(1H)-one

5-Bromo-3-methylpyridin-2-one 18a (2.00 g, 10.6 mmol) was dissolved in 20 mL of 1,2-dichloroethane, and then added with 2,2'-bipyridine (8.31 g, 53.2 mmol), copper acetate (1.58 g, 12.7 mmol), cyclopropylboronic acid (1.37 g, 15.9 mmol), and sodium carbonate (2.26 g, 21.2 mmol) for 6 h of reaction at 70°C. The solvent was removed. The residue was purified by flash silica gel column chromatography to obtain 5-bromo-1-cyclopropyl-3-methyl-1,2-dihydropyridin-2-one 18b (2.10 g, white solid) with a yield of 84.9%. MS m/z (ESI): 228.05 [M+1]⁺.

### The second step

### 1-Cyclopropyl-3-methyl-5-(tetramethyl-1,3,2-dioxin-2-yl)-1,2-dihydropyridin-2-one

5-Bromo-1-cyclopropyl-3-methyl-1,2-dihydropyridin-2-one 18b (1.50 g, 6.5 mmol), 4,4,5,5-tetramethyl-2-(tetramethyl-1,3,2-dioxol-2-yl)-1,3,2-dioxol (1.64 g, 6.5 mmol), potassium acetate (0.63 g, 6.5 mmol), and [1,1'-bis(diphenylphosphino)ferrocene]dichloropalladium (II) (4.73 g, 6.5 mmol) were dissolved in 20 mL of 1,4-dioxane. The mixture was replaced with nitrogen, and heated to 100°C for 6 h of reaction. The solvent was removed. The residue was purified by flash silica gel column chromatography to obtain 1-cyclopropyl-3-methyl-5-(tetramethyl-1,3,2-dioxin-2-yl)-1,2-dihydropyridin-2-one 18c (1.78 g, colorless oil) with a yield of 82.4%. MS m/z (ESI): 276.15 [M+1]⁺.

### The third step

### 1-Cyclopropyl-5-hydroxy-3-methyl-1,2-dihydropyridin-2-one

1-Cyclopropyl-3-methyl-5-(tetramethyl-1,3,2-dioxin-2-yl)-1,2-dihydropyridin-2-one 18c (0.30 g, 0.90 mmol) was dissolved in 5.0 mL of N,N-dimethylformamide, cooled to 0°C, and dropwise added with hydrogen peroxide (0.09 g, 0.79 mmol). After the dropwise addition was completed, the mixture was warmed up to room temperature for 12 h of reaction to obtain a reaction solution of 1-cyclopropyl-5-hydroxy-3-methyl-1,2-dihydropyridin-2-one 18d, which was directly used in the next step without purification. MS m/z (ESI): 166.20 [M+1]⁺.

### The fourth step

### 1-Cyclopropyl-5-(2,6-dichloro-4-nitrophenoxy)-3-methyl-1,2-dihydropyridin-2-one

1,3-Dichloro-2-fluoro-5-nitrobenzene (0.20 g, 0.98 mmol) and potassium carbonate (0.27 g, 1.95 mmol) were dissolved in the reaction solution of 1-cyclopropyl-5-hydroxy-3-methyl-1,2-dihydropyridin-2-one 18d for 3 h of reaction at room temperature. The solvent was removed. The residue was purified by flash silica gel column chromatography to obtain 1-cyclopropyl-5-(2,6-dichloro-4-nitrophenoxy)-3-methyl-1,2-dihydropyridin-2-one 18e (0.24 g, colorless oil) with a yield of 88.0%. MS m/z (ESI): 355.00 [M+1]⁺.

### The fifth step

### 5-(4-Amino-2,6-dichlorophenoxy)-1-cyclopropyl-3-methyl-1,2-dihydropyridin-2-one

1-Cyclopropyl-5-(2,6-dichloro-4-nitrophenoxy)-3-methyl-1,2-dihydropyridin-2-one 18e (0.24 g, 0.57 mmol) and tin dichloride dihydrate (0.64 g, 2.85 mmol) were dissolved in 5.0 mL of ethanol for 3 h of reaction at 80°C. The pH value was adjusted to greater than 7 with aqueous solution of sodium hydroxide. The solvent was removed. The residue was purified by flash silica gel column chromatography to obtain 5-(4-amino-2,6-dichlorophenoxy)-1-cyclopropyl-3-methyl-1,2-dihydropyridin-2-one 18f (0.13 g, yellow solid) with a yield of 69.9%. MS m/z (ESI): 325.05 [M+1]⁺.

### The sixth step

### Ethyl (E)-(2-cyano-2-(2-(3,5-dichloro-4-((1-cyclopropyl-5-methyl-6-oxo-1,6-dihydropyridin-3-l)oxy)phenyl)hydrazone)acetyl)carbamate

5-(4-Amino-2,6-dichlorophenoxy)-1-cyclopropyl-3-methyl-1,2-dihydropyridin-2-one 18f (0.13 g, 0.40 mmol) was dissolved in 4 mL of acetic acid and 1.0 mL of water, and the mixture was cooled to 0°C. Then 0.5 mL of concentrated hydrochloric acid was added, and 1 mL of aqueous solution of sodium nitrite (0.03 g, 0.48 mmol) was slowly dropwise added. After the dropwise addition was completed, the mixture was stirred for ten minutes, and then added with sodium acetate (0.03 g, 0.40 mmol) and N-cyanoacetylurethane (0.06 g, 0.40 mmol). The obtained mixture was removed out of the ice bath and warmed up to room temperature for 12 h of reaction. The reaction mixture was added with 10 mL of water. The solid was filtered out, and dried to obtain ethyl (E)-(2-cyano-2-(2-(3,5-dichloro-4-((1-cyclopropyl-5-methyl-6-oxo-1,6-dihydropyridin-3-l)oxy)p henyl)hydrazone)acetyl)carbamate 18g (0.06 g, yellow solid) with a yield of 22.6%. The crude product was used directly in the next step. MS m/z (ESI): 492.00 [M+1]⁺.

### The seventh step

### 2-(3,5-Dichloro-4-[(1-cyclopropyl-5-methyl-6-oxo-1,6-dihydropyridin-3-yl)oxy]phenyl )-3, 5-dioxo-2,3,4,5-tetrahydro-1,2,4-triazine-6-carbonitrile

Ethyl (E)-(2-cyano-2-(2-(3,5-dichloro-4-((1-cyclopropyl-5-methyl-6-oxo-1,6-dihydropyridin-3-l)oxy)phenyl)hydrazone)acetyl)carbamate 18g (0.06 g, 0.09 mmol) crude product was dissolved in N,N-dimethylacetamide (5.0 mL), and added with potassium acetate (0.01 g, 0.12 mmol) for 3 h of reaction at 120°C. The solvent was removed. The residue was purified by preparative HPLC to obtain 2-(3,5-dichloro-4-[(1-cyclopropyl-5-methyl-6-oxo-1,6-dihydropyridin-3-yl)oxy]phenyl)-3,5-dioxo-2,3,4,5-tetrahydro-1,2,4-triazine-6-carbonitrile 18 (30.0 mg, white solid) with a yield of 74.3%. MS m/z (ESI): 446.00 [M+1]⁺. ¹H NMR (400 MHz, DMSO-d*₆*) δ 13.28 (s, 1H), 7.78 (s, 2H), 7.24 (d, *J* = 2.0 Hz, 1H), 7.09 (d, *J* = 3.2 Hz, 1H), 3.34 - 3.28 (m, 1H), 2.01 (s, 3H), 0.99-0.94 (m, 2H), 0.80-0.75 (m, 2H).

### Example 19

### 2-(3,5-Dichloro-4-((5-cyclopropyl-1-isopropyl-6-oxo-1,6-dihydropyridin-3-yl)oxy)phen yl)-3,5-dioxo-2,3,4,5-tetrahydro-1,2,4-triazine-6-carbonitrile

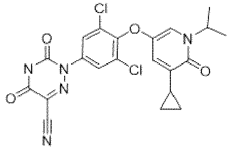

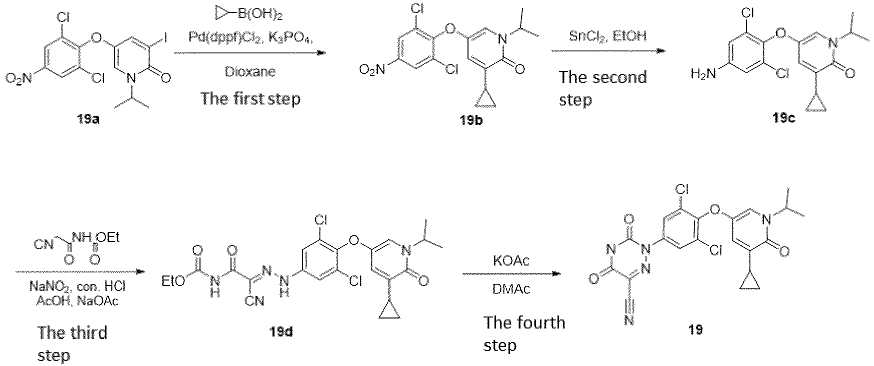

### The first step

### 3-Cyclopropyl-5-(2,6-dichloro-4-nitrophenoxy)-1-ethyl-1,2-dihydropyridin-2-one

5-(2,6-Dichloro-4-nitrophenoxy)-3-iodo-1-(propan-2-yl)-1,2-dihydropyridin-2-one 19a (200.0 mg, 0.39 mmol) was dissolved in 5.0 mL of 1,4-dioxane, and then added with cyclopropylboronic acid (100.5 mg, 1.17 mmol), tripotassium phosphate (248.3 mg, 1.17 mmol), and [1,1'-bis(diphenylphosphine)ferrocene]dichloropalladium (II) (28.5 mg, 0.04 mmol) for 8 h of reaction at 100°C under nitrogen protection. The solvent was removed. The residue was purified by flash silica gel column chromatography to obtain 3-cyclopropyl-5-(2,6-dichloro-4-nitrophenoxy)-1-ethyl-1,2-dihydropyridin-2-one 19b (150.0 mg, yellow solid) with a yield of 93.7 %. MS m/z (ESI): 383.00 [M+1]⁺.

### The second step

### 5-(4-Amino-2,6-dichlorophenoxy)-3-cyclopropyl-1-(propan-2-yl)-1,2-dihydropyridin-2-one

3-Cyclopropyl-5-(2,6-dichloro-4-nitrophenoxy)-1-ethyl-1,2-dihydropyridin-2-one 19b (150.0 mg, 0.35 mmol) and tin dichloride dihydrate (394.8 mg, 1.75 mmol) were dissolved in 10.0 mL of ethanol for 3 h of reaction at 80°C. The pH value was adjusted to greater than 7 with aqueous solution of sodium hydroxide. The solvent was removed. The residue was purified by flash silica gel column chromatography to obtain 5-(4-amino-2,6-dichlorophenoxy)-3-cyclopropyl-1-(propan-2-yl)-1,2-dihydropyridin-2-one 19c (122.0 mg, yellow solid) with a yield of 89.8%. MS m/z (ESI): 353.10 [M+1]⁺.

### The third step

### Ethyl N-[(Z)-cyano(2-(3,5-dichloro-4-[(5-cyclopropyl-6-oxo-1-(propan-2-yl)-1,6-dihydropyridin-3-yl)oxy]phenyl)hydrazin-1-ylidene)carbonyl] carbamate

5-(4-Amino-2,6-dichlorophenoxy)-3-cyclopropyl-1-(propan-2-yl)-1,2-dihydropyridin-2-one 19c (122.0 mg , 0.31mmol) was dissolved in 4.0 mL of acetic acid and 1.0 mL of water. The mixture was cooled to 0°C, then added with 0.5 mL of concentrated hydrochloric acid, and slowly dropwise added with 4.0 mL of aqueous solution of sodium nitrite (0.03 g, 0.48 mmol). After the dropwise addition was completed, the mixture was stirred for ten minutes, and added with sodium acetate (23.5 mg, 0.34 mmol) and N-cyanoacetylurethane (96.8 mg, 0.62 mmol). The obtained mixture was removed out of the ice bath and warmed up to room temperature for 12 h of reaction. The reaction mixture was added with 10 mL of water. The solid was filtered out, and dried to obtain ethyl N-[(Z)-cyano(2-(3,5-dichloro-4-[(5-cyclopropyl-6-oxo-1-(propan-2-yl)-1,6-dihydropyridin-3-yl)oxylphenyl)hydrazin-1-ylidene)carbonyl] carbamate 19d (300.0 mg, yellow solid). The crude product was used directly in the next step. MS m/z (ESI): 520.05 [M+1]⁺.

### The fourth step

### 2-(3,5-Dichloro-4-[(5-cyclopropyl-6-oxo-1-(propan-2-yl)-1,6-dihydropyridin-3-yl)oxy] phenyl)-3,5-dioxo-2,3,4,5-tetrahydro-1,2,4-triazine-6-carbonitrile

Ethyl N-[(Z)-cyano(2-(3,5-dichloro-4-[(5-cyclopropyl-6-oxo-1-(propan-2-yl)-1,6-dihydropyridin-3-yl)oxy]phenyl)hydrazin-1-ylidene)carbonyl] carbamate 19d (140.0 mg, 0.25 mmol) crude product was dissolved in N,N-dimethylacetamide (5.0 mL), and added with potassium acetate (73.6 mg, 0.75 mmol) for 3 h of reaction at 120°C. The solvent was removed. The residue was purified by preparative HPLC to obtain 2-(3,5-dichloro-4-[(5-cyclopropyl-6-oxo-1-(propan-2-yl)-1,6-dihydropyridin-3-yl)oxy]phenyl)-3, 5-dioxo-2,3,4,5-tetrahydro-1,2,4-triazine-6-carbonitrile 19 (19.0 mg, white solid) with a yield of 15.8%. MS m/z (ESI): 474.00 [M+1]⁺. ¹H NMR (400 MHz, DMSO-d*₆*) δ 13.36 (s, 1H), 7.85 (s, 2H), 7.21 (d, *J* = 3.2 Hz, 1H), 6.93 (d, *J* = 2.8 Hz, 1H), 5.18-5.11 (m, 1H), 2.18 - 2.10 (m, 1H), 1.31 (d, *J* = 6.8 Hz, 6H), 0.97-0.92 (m, 2H), 0.77-0.73 (m, 2H).

### Example 20

### 2-(3,5-dichloro-4-((6-oxo-1-phenyl-1,6-dihydropyridin-3-yl)oxy)phenyl)-3,5-dioxo-2,3, 4,5-tetrahydro-1,2,4-triazine-6-carbonitrile

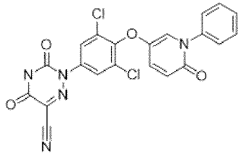

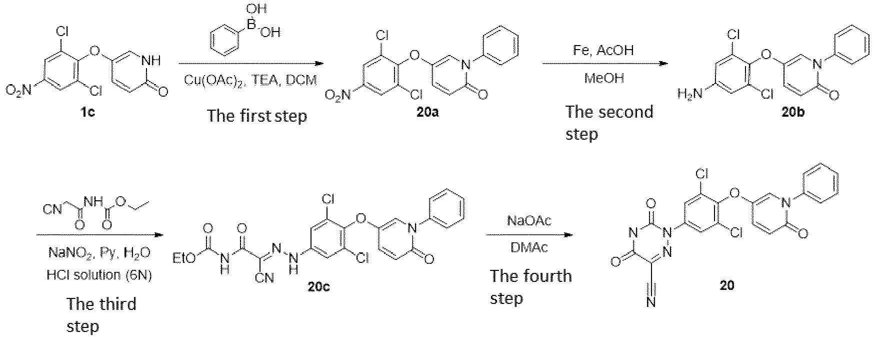

### The first step

### 5-(2,6-Dichloro-4-nitrophenoxy)-1-phenylpyridin-2(1H)-one

5-(4-Nitro-2,6-dichlorophenoxy)-2-carbonylpyridine 1c (2.00 g, 6.22 mmol) was dissolved in 200 mL of anhydrous dichloromethane, and then added with phenylboronic acid (2.28 g, 18.66 mmol), triethylamine (3.46 mL, 34.88 mmol), and copper acetate (0.77 g, 6.22 mmol) for 16 h of reaction at 20°C. The reaction solution was added with water, and extracted with ethyl acetate three times. The organic phase was dried over anhydrous sodium sulfate, filtered, and purified by flash column chromatography (PE:EA=1:2) to obtain 5-(2,6-dichloro-4-nitrophenoxy)-1-phenylpyridin-2(1H)-one 20a (2.30 g, yellow solid) with a yield of 83.6%. MS m/z (ESI): 377.00 [M+1]⁺.

### The second step

### 5-(2,6-Dichloro-4-aminophenoxy)-1-phenylpyridin-2(1H)-one

5-(2,6-Dichloro-4-nitrophenoxy)-1-phenylpyridin-2(1H)-one 20a (2.30 g, 5.20 mmol) was dissolved in 30 mL of methanol, and then added with iron powder (1.16 g, 20.80 mmol) and 1.5 mL of aqueous solution of ammonium chloride (0.56 g, 10.40 mmol) for 4 h of reaction at 85°C. The reaction solution was concentrated, then added with 20 mL of water, and extracted with 35 mL of ethyl acetate three times. The organic phase was dried over anhydrous sodium sulfate, filtered, and dried by rotating to obtain 5-(2,6-dichloro-4-aminophenoxy)-1-phenylpyridin-2(1H)-one 20b (2.10 g, brown solid) with a yield of 95.7%. MS m/z (ESI): 347.00 [M+1]⁺.

### The third step

### Ethyl (E)-(2-cyano-2-(2-(3,5-dichloro-4-((6-oxo-1-phenyl-1,6-dihydropyridin-3-yl)oxy)phenyl)hydrazono)acetyl carbamate

The compound 5-(2,6-dichloro-4-aminophenoxy)-1-phenylpyridin-2(1H)-one 20b (2.00 g, 4.74 mmol) was dissolved in 30 mL (6N) of aqueous solution of hydrochloric acid, cooled to 0°C, and then slowly dropwise added with 1 mL of aqueous solution of sodium nitrite (0.49 g, 7.11 mmol). The reaction solution was then stirred for 15 minutes. N-cyanoacetylurethane (1.11 g, 7.11 mmol) was dissolved in 20 mL of water, cooled to 0°C, and then added with 32 mL of pyridine. The mixture was stirred at 0°C for 10 minutes. Then, the latter reaction solution was slowly dropwise added to the former reaction solution for 30 min of reaction at 0°C. The obtained reaction solution was added with 20mL of water, and a large amount of solids were precipitated. The mixture was then filtered. The filter cake was dissolved with absolute ethanol, and then the solution was concentrated. The above operations were repeated 2-3 times to obtain ethyl (E)-(2-cyano-2-(2-(3,5-dichloro-4-((6-oxo-1-phenyl-1,6-dihydropyridin-3-yl)oxy)phenyl)hydrazo no)acetyl carbamate 20c (2.65 g, red solid) crude product, which was used directly in the next step. MS m/z (ESI): 514.05 [M+1]⁺.

### The fourth step

### 2-(3,5-Dichloro-4-((6-oxo-1-phenyl-1,6-dihydropyridin-3-yl)oxy)phenyl)-3,5-dioxo-2,3 ,4,5-tetrahydro-1,2,4-triazine-6-carbonitrile

Ethyl (E)-(2-cyano-2-(2-(3,5-dichloro-4-((6-oxo-1-phenyl-1,6-dihydropyridin-3-yl)oxy)phenyl)hydrazono)acetyl carbamate 20c (2.65 g, 4.59 mmol) was dissolved in 30 mL of N,N-dimethylacetamide, and added with sodium acetate (1.13 g, 13.77 mmol), and the mixture was heated up to 120°C and reacted at this temperature for 4 h. The reaction solution was added with 20 mL of water, and extracted with ethyl acetate (30 mL×3). The organic phases were combined, dried over anhydrous sodium sulfate, and filtered. The filtrate was concentrated under reduced pressure, and purified by a flash silica gel column to obtain a crude product. 450 mg of the crude product was subjected to Prep-HPLC to obtain 2-(3,5-dichloro-4-((6-oxo-1-phenyl-1,6-dihydropyridin-3-yl)oxy)phenyl)-3,5-dioxo-2,3,4,5-tetrah ydro-1,2,4-triazine-6-carbonitrile 20 (290.0 mg, white solid) with a yield of 66.4%. MS m/z (ESI): 467.95 [M+1]⁺. ¹H NMR (400 MHz, DMSO-d*₆*) *δ* 7.76 (s, 2H), 7.58 - 7.47 (m, 3H), 7.46 - 7.41 (m, 2H), 7.37-7.33 (m, 2H), 6.55 (d, *J =* 10.0 Hz, 1H).

### Example 21

### 2-(3,5-Dichloro-4-((1-(4-fluorophenyl)-6-oxo-1,6-dihydropyridin-3-yl)oxy)phenyl)-3,5-dioxo-2,3,4,5-tetrahydro-1,2,4-triazine-6-carbonitrile

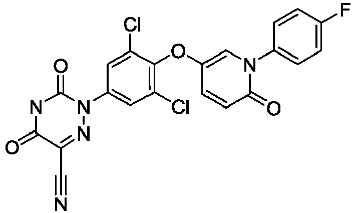

The title product 2-(3,5-dichloro-4-((1-(4-fluorophenyl)-6-oxo-1,6-dihydropyridin-3-yl)oxy)phenyl)-3,5-dioxo-2,3, 4,5-tetrahydro-1,2,4-triazine-6-carbonitrile 21 (273.0 mg, white solid) was prepared in a yield of 64.8% using the method of Example 20 except that phenylboronic acid was replaced with p-fluorophenylboronic acid. MS m/z (ESI): 485.95 [M+1]⁺. ¹H NMR (400 MHz, DMSO-d*₆*) *δ* 13.26 (s, 1H), 7.75 (s, 2H), 7.53 (dd, *J* = 10.0 Hz, 3.2 Hz, 1H), 7.47 (d, *J* = 2.8 Hz, 1H), 7.44 - 7.38 (m, 2H), 7.36 - 7.27 (m, 2H), 6.54 (d, *J* = 10.0 Hz, 1H).

### Example 22

### 2-(3,5-Dichloro-4-((1-(3-fluorophenyl)-6-oxo-1,6-dihydropyridin-3-yl)oxy)phenyl)-3,5-dioxo-2,3,4,5-tetrahydro-1,2,4-triazine-6-carbonitrile

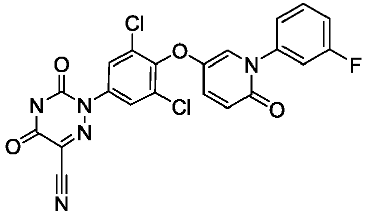

The title product 2-(3,5-dichloro-4-((1-(3-fluorophenyl)-6-oxo-1,6-dihydropyridin-3-yl)oxy)phenyl)-3,5-dioxo-2,3, 4,5-tetrahydro-1,2,4-triazine-6-carbonitrile 22 (30 mg, pale yellow solid, yield 34.18%) was prepared using the method of Example 20 except that phenylboronic acid was replaced with m-fluorophenylboronic acid. MS m/z (ESI): 485.95 [M+1]⁺. ¹H NMR (400 MHz, DMSO-d*₆*) *δ* 7.75 (s, 2H), 7.62-7.58 (m, 1H), 7.47 (d, *J* = 2.8 Hz, 3H), 7.35 - 7.20 (m, 3H), 6.55 (d, *J* = 8.0 Hz, 1H).

### Example 23

### 2-(3,5-Dichloro-4-((6-oxo-1-(3-(trifluoromethyl)phenyl)-1,6-dihydropyridin-3-yl)oxy)p henyl)-3,5-dioxo-2,3,4,5-tetrahydro-1,2,4-triazine-6-carbonitrile

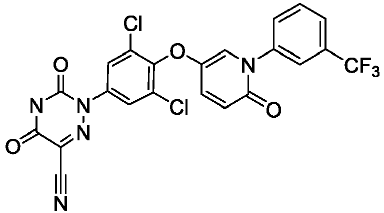

The title product 2-(3,5-dichloro-4-((6-oxo-1-(3-(trifluoromethyl)phenyl)-1,6-dihydropyridin-3-yl)oxy)phenyl)-3,5 -dioxo-2,3,4,5-tetrahydro-1,2,4-triazine-6-carbonitrile 23 (17.0 mg, white solid, yield: 99.8%) was prepared using the method of Example 20 except that phenylboronic acid was replaced with m-trifluoromethylbenzeneboronic acid. MS m/z (ESI): 536.01 [M+1]⁺. ¹H NMR (400 MHz, DMSO-d*₆*) δ 13.25(s, 1H), 7.81 (s, 2H), 7.78-7.72 (m, 3H), 7.70-7.65(m, 1H), 7.62 (d, *J* = 4.0 Hz, 1H), 7.55(dd, *J* = 4.0, 4.0 Hz , 1H), 6.57 (d, *J* = 12.0 Hz, 1H).

### Example 24

### 2-(3,5-Dichloro-4-((6-oxo-1-(3-(trifluoromethoxy)phenyl)-1,6-dihydropyridin-3-yl)oxy) phenyl)-3,5-dioxo-2,3,4,5-tetrahydro-1,2,4-triazine-6-carbonitrile

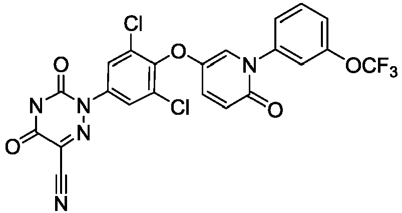

The title product 2-(3,5-dichloro-4-((6-oxo-1-(3-(trifluoromethoxy)phenyl)-1,6-dihydropyridin-3-yl)oxy)phenyl)-3 ,5-dioxo-2,3,4,5-tetrahydro-1,2,4-triazine-6-carbonitrile 24 (40.0 mg, white solid, yield: 90.1%) was prepared using the method of Example 20 except that phenylboronic acid was replaced with m-trifluoromethoxyphenylboronic acid. MS m/z (ESI): 551.95 [M+1]⁺. ¹H NMR (400 MHz, DMSO-d*₆*) δ 13.26(s, 1H), 7.76 (s, 2H), 7.63 (t, *J* = 8.0 Hz, 1H), 7.56-7.40 (m, 5H), 6.56 (d, *J =* 8.0 Hz, 1H).

### Example 25

### 2-(3,5-Dichloro-4-((6-oxo-1-(4-methylphenyl)-1,6-dihydropyridin-3-yl)oxy)phenyl)-3,5 -dioxo-2,3,4,5-tetrahydro-1,2,4-triazine-6-carbonitrile

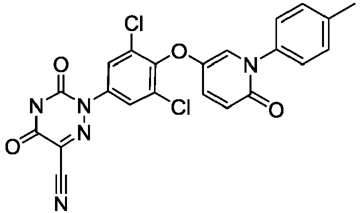

The title product 2-(3,5-dichloro-4-((6-oxo-1-(4-methylphenyl)-1,6-dihydropyridin-3-yl)oxy)phenyl)-3,5-dioxo-2, 3,4,5-tetrahydro-1,2,4-triazine-6-carbonitrile 25 (50.0 mg, pale yellow solid, yield 38.02%) was prepared using the method of Example 20 except that phenylboronic acid was replaced with p-methylphenylboronic acid. MS m/z (ESI): 482.00 [M+1]⁺. ¹H NMR (400 MHz, DMSO-d*₆*) δ 13.25(s, 1H), 7.75 (s, 2H), 7.50 (dd, *J* = 10.0, 3.2 Hz, 1H), 7.36 (d, J = 3.2 Hz, 1H), 7.30-7.19 (m, 4H), 6.52 (d, *J* = 10.0 Hz, 1H), 2.33 (s, 3H).

### Example 26

### 2-(3,5-Dichloro-4-((6-oxo-1-(3-methylphenyl)-1,6-dihydropyridin-3-yl)oxy)phenyl)-3,5 -dioxo-2,3,4,5-tetrahydro-1,2,4-triazine-6-carbonitrile

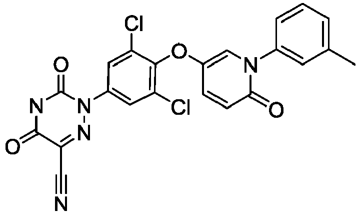

The title product 2-(3,5-dichloro-4-((6-oxo-1-(3-methylphenyl)-1,6-dihydropyridin-3-yl)oxy)phenyl)-3,5-dioxo-2, 3,4,5-tetrahydro-1,2,4-triazine-6-carbonitrile 26 (280.00 mg, white solid, yield: 22.86%) was prepared using the method of Example 20 except that phenylboronic acid was replaced with m-methylphenylboronic acid. MS m/z (ESI): 482.28 [M+1]⁺. ¹H NMR (400 MHz, DMSO-d*₆*) *δ* 7.76 (s, 2H), 7.51 (dd, *J* = 10.0, 3.2Hz, 1H), 7.40 (d, *J* = 3.2 Hz, 1H), 7.36 (d, *J* = 8.0 Hz, 1H), 7.24 (d, *J* = 8.0 Hz, 1H), 7.18 (s, 1H), 7.13 (d, *J* = 8.0 Hz, 1H), 6.53 (d, *J* = 10.0 Hz, 1H), 2.34 (s, 3H).

### Example 27

### 2-(3,5-Dichloro-4-((6-oxo-1-(o-methylphenyl)-1,6-dihydropyridin-3-yl)oxy)phenyl)-3,5 -dioxo-1,2,3,4-4,5-tetrahydro-1,2,4-triazine-6-carbonitrile

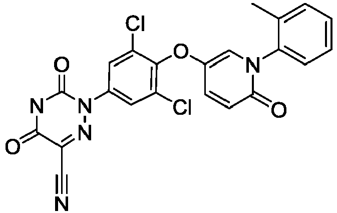

The title product 2-(3,5-dichloro-4-((6-oxo-1-(o-methylphenyl)-1,6-dihydropyridin-3-yl)oxy)phenyl)-3,5-dioxo-1, 2,3,4-4,5-tetrahydro-1,2,4-triazine-6-carbonitrile 27 (61.1 mg, white solid, yield: 40.45%) was prepared using the method of Example 20 except that phenylboronic acid was replaced with o-methylphenylboronic acid. MS m/z (ESI): 482.28[M+1]⁺. ¹H NMR (400 MHz, DMSO-d6) *δ* 13.25(s, 1H), 7.74 (s, 2H), 7.58 (dd, *J* = 10.0, 4.0 Hz, 1H), 7.35 (d, *J* = 4.0 Hz, 2H), 7.33 - 7.26 (m, 2H), 7.19 (d, *J* = 8.0 Hz, 1H), 6.56 (d, *J* = 10.0 Hz, 1H), 2.03 (s, 3H).

### Example 28

### 2-(3,5-Dichloro-4-((1-(3-cyanophenyl)-6-oxo-1,6-dihydropyridin-3-yl)oxy)phenyl)-3,5-dioxo-1,2,3,4-4,5-tetrahydro-1,2,4-triazine-6-carbonitrile

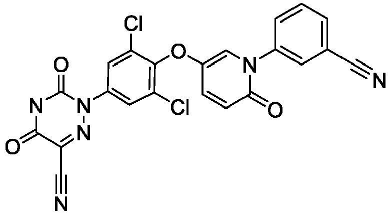

The title product 2-(3,5-dichloro-4-((1-(3-cyanophenyl)-6-oxo-1,6-dihydropyridin-3-yl)oxy)phenyl)-3,5-dioxo-1,2, 3,4-4,5-tetrahydro-1,2,4-triazine-6-carbonitrile 28 (125.1 mg, white solid, yield: 78.61%) was prepared using the method of Example 20 except that phenylboronic acid was replaced with m-cyanophenylboronic acid. MS m/z (ESI): 493.26 [M+1]⁺. ¹H NMR (400 MHz, DMSO-d6) *δ* 13.25(s, 1H),7.96 (s, 1H), 7.91 (d, *J* = 7.2 Hz, 1H), 7.76 (s, 2H), 7.74 - 7.66 (m, 2H), 7.62 - 7.51 (m, 2H), 6.58 (d, *J* = 10.0 Hz, 1H).

### Example 29

### 2-(3,5-Dichloro-4-((1-(3,5-difluorophenyl)-6-oxo-1,6-dihydropyridin-3-yl)oxy)phenyl)-3,5-dioxo-2,3,4,5-tetrahydro-1,2,4-triazine-6-carbonitrile

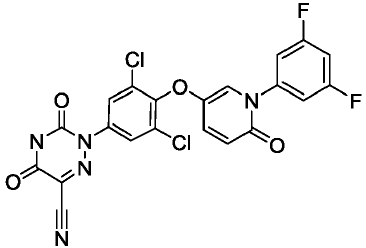

The title product 2-(3,5-dichloro-4-((1-(3,5-difluorophenyl)-6-oxo-1,6-dihydropyridin-3-yl)oxy)phenyl)-3,5-dioxo -2,3,4,5-tetrahydro-1,2,4-triazine-6-carbonitrile 29 (50.0 mg, white solid, yield: 98.84%) was prepared using the method of Example 20 except that phenylboronic acid was replaced with 3,5-difluorophenylboronic acid. MS m/z (ESI): 504.23[M+1]⁺. ¹H NMR (400 MHz, DMSO-d6) *δ* 13.24(s, 1H), 7.76(s, 2H), 7.58-7.50 (m, 2H), 7.41-7.33 (m, 1H), 7.30-7.23 (m, 2H), 6.60-6.51 (m, 1H).

### Example 30

### 2-(3,5-Dichloro-4-((1-(3,5-dimethylphenyl)-6-oxo-1,6-dihydropyridin-3-yl)oxy)phenyl) -3,5-dioxo-2,3,4,5-tetrahydro-1,2,4-triazine-6-carbonitrile

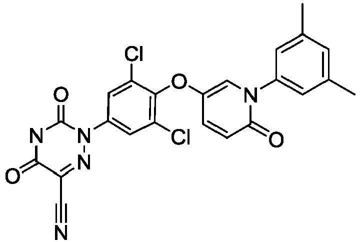

The title product 2-(3,5-dichloro-4-((1-(3,5-dimethylphenyl)-6-oxo-1,6-dihydropyridin-3-yl)oxy)phenyl)-3,5-diox o-2,3,4,5-tetrahydro-1,2,4-triazine-6-carbonitrile 30 (262.1 mg, white solid, yield: 24.68%) was prepared using the method of Example 20 except that phenylboronic acid was replaced with 3,5-dimethylphenylboronic acid. MS m/z (ESI): 496.3 [M+1]⁺. ¹H NMR (400 MHz, DMSO-d6) *δ*13.25(s, 1H), 7.76 (s, 2H), 7.49 (dd, *J* = 10.0, 4.0 Hz, 1H), 7.36 (d, *J* = 4.0 Hz, 1H), 7.06 (s, 1H), 6.95 (s, 2H), 6.51 (d, *J* = 10.0 Hz, 1H), 2.30 (s, 6H).

### Example 31

### 2-(3,5-Dichloro-4-((1-(3-cyclopropylphenyl)-6-oxo-1,6-dihydropyridin-3-yl)oxy)phenyl )-3,5-dioxo-2,3,4,5-tetrahydro-1,2,4-triazine-6-carbonitrile

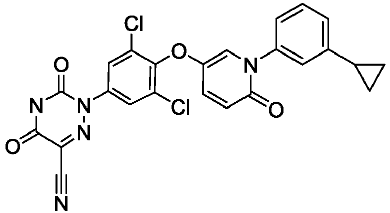

The title product 2-(3,5-dichloro-4-((1-(3-cyclopropylphenyl)-6-oxo-1,6-dihydropyridin-3-yl)oxy)phenyl)-3,5-diox o-2,3,4,5-tetrahydro-1,2,4-triazine-6-carbonitrile 31 (50.0 mg, white solid, yield: 97.47%) was prepared using the method of Example 20 except that phenylboronic acid was replaced with 3-cyclopropylphenylboronic acid. MS m/z (ESI): 508.05[M+1]⁺. ¹H NMR (400 MHz, DMSO-d6) *δ* 13.24(s, 1H), 7.75 (s, 2H), 7.52-7.47 (m, 1H), 7.45-7.40 (m, 1H), 7.38-7.31 (m, 1H), 7.19-7.02 (m, 3H), 6.61-6.47 (m, 1H), 2.02-1.91(m, 1H), 1.01-0.93(m,2H), 0.75-0.67(m, 2H).

### Example 32

### 2-(3,5-Dichloro-4-((6-oxo-1-(thiophen-3-yl)-1,6-dihydropyridin-3-yl)oxy)phenyl)-3,5-d ioxo-2,3,4,5-tetrahydro-1,2,4-triazine-6-carbonitrile

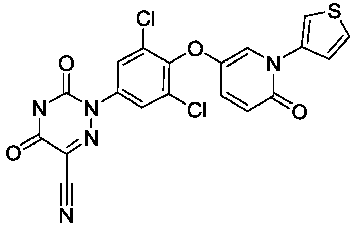

The title product 2-(3,5-dichloro-4-((6-oxo-1-(thiophen-3-yl)-1,6-dihydropyridin-3-yl)oxy)phenyl)-3,5-dioxo-2,3,4 ,5-tetrahydro-1,2,4-triazine-6-carbonitrile KPC00134 (50.0 mg, white solid, yield: 97.83%) was prepared using the method of Example 20 except that phenylboronic acid was replaced with 3-thiopheneboronic acid. MS m/z (ESI): 473.98[M+1]⁺. ¹H NMR (400 MHz, DMSO-d6) *δ* 13.23(s, 1H), 7.85-7.70 (m, 3H), 7.65-7.57 (m, 1H), 7.54-7.43 (m, 2H), 7.27 (d, *J* = 4.0 Hz, 2H), 6.54 (d, *J* = 12.0 Hz 1H).

### Example 33

### 2-(3,5-Dichloro-4-((6-oxo-1-(thien-2-yl)-1,6-dihydropyridin-3-yl)oxy)phenyl)-3,5-diox o-2,3,4,5-tetrahydro-1,2,4-triazine-6-carbonitrile

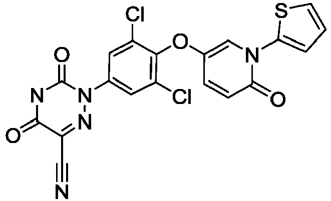

The title product 2-(3,5-dichloro-4-((6-oxo-1-(thien-2-yl)-1,6-dihydropyridin-3-yl)oxy)phenyl)-3,5-dioxo-2,3,4,5-t etrahydro-1,2,4-triazine-6-carbonitrile 33 (130.0 mg, white solid, yield 19.71%) was prepared using the method of Example 1 except that 2-iodopropane was replaced with 2-bromothiophene. MS m/z (ESI): 473.90 [M+1]⁺.

### Example 34

### 2-(3,5-Dichloro-4-((6-oxo-1-(thiazol-2-yl)-1,6-dihydropyridin-3-yl)oxy)phenyl)-3,5-dio xo-2,3,4,5-tetrahydro-1,2,4-triazine-6-carbonitrile

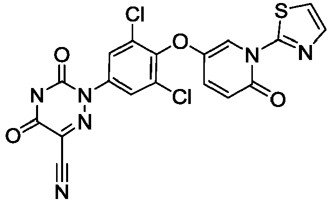

The title product 2-(3,5-dichloro-4-((6-oxo-1-(thiazol-2-yl)-1,6-dihydropyridin-3-yl)oxy)phenyl)-3,5-dioxo-2,3,4,5 -tetrahydro-1,2,4-triazine-6-carbonitrile 34 (13.0 mg, white solid, yield 22.48%) was prepared using the method of Example 1 except that 2-iodopropane was replaced with 2-bromothiazole. MS m/z (ESI): 474.90 [M+1]⁺. ¹H NMR (400 MHz, DMSO-d6), 8.13 (d, *J* = 3.2 Hz, 1 H), 7.88 (s, 2 H), 7.84 (dd, *J* = 10.0, 4.0 Hz, 1 H), 7.74 (d, *J* = 4.0 Hz, 1 H), 7.62 (d, *J* = 4.0 Hz, 1 H), 6.91 (d, *J* = 10.0 Hz, 1 H).

### Example 35/36

### 2-(3,5-Dichloro-4-((6-oxo-1-phenyl-1,6-dihydropyridin-3-yl)oxy)phenyl)-6-(fluorometh yl)-1,2,4-triazine-3,5(2H,4H)-dione

### 2-(3,5-Dichloro-4-((6-oxo-1-phenyl-1,6-dihydropyridin-3-yl)oxy)phenyl)-6-methyl-1,2, 4-triazine-3,5(2H,4H)-dione

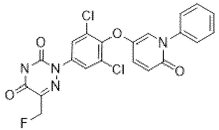

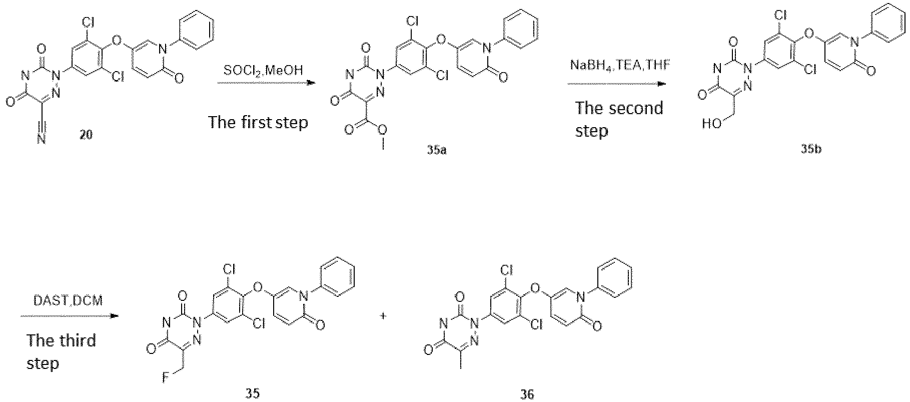

### The first step

### Methyl 2-(3,5-dichloro-4-((6-oxo-1-phenyl-1,6-dihydropyridyl-3-yl)oxy)phenyl)-3,5-dioxo-2,3,4,5-tetrahydro-1,2,4-triazine-6-carboxylate

2-(3,5-Dichloro-4-((6-oxo-1-phenyl-1,6-dihydropyridin-3-yl)oxy)phenyl)-3,5-dioxo-2,3 ,4,5-tetrahydro-1,2,4-triazine-6-carbonitrile 20 (1.50 g, 1.60 mmol) was dissolved in 15 mL of anhydrous methanol, and then slowly dropwise added with sulfoxide chloride (2.50ml, 34.46 mmol) at room temperature. The mixture was reacted at 100°C for 4 h. After cooling to room temperature, the reaction solution was concentrated to remove the solvent, added with water, and extracted with ethyl acetate three times. The organic phase was dried over anhydrous sodium sulfate, filtered, and added with silica gel to prepare a sample. The sample was purified by column chromatography (PE:EA=0-100%) to obtain methyl 2-(3,5-dichloro-4-((6-oxo-1-phenyl-1,6-dihydropyridyl-3-yl)oxy)phenyl)-3,5-dioxo-2,3,4,5-tetrah ydro-1,2,4-triazine-6-carboxylate 35a (1.45 g, red solid) with a yield of 66.62%. MS m/z (ESI): 501.00 [M+1]⁺.

### The second step

### 2-(3,5-Dichloro-4-((6-oxo-1-phenyl-1,6-dihydropyridin-3-yl)oxy)phenyl)-6-(hydroxym ethyl)-1,2,4-triazine-3,5(2H,4H)-dione

Methyl 2-(3,5-dichloro-4-((6-oxo-1-phenyl-1,6-dihydropyridyl-3-yl)oxy)phenyl)-3,5-dioxo-2,3,4,5-tetrahydro-1,2,4-triazine-6-carboxylate 35a (1.45 g, 1.93 mmol) was dissolved in 15 mL of anhydrous tetrahydrofuran, and then added with triethylamine (0.20 g, 1.93 mmol) at 20°C. The mixture was stirred for 10 minutes, then added with sodium borohydride (0.07 g, 1.93 mmol), and slowly dropwise added with 5.0 mL of anhydrous methanol. After the dropwise addition was completed, the mixture was reacted at 20°C for 20 min. The reaction was quenched by water. The solvent was dried by rotating. Then the reaction solution was added with water, and extracted with ethyl acetate three times. The organic phase was dried over anhydrous sodium sulfate, filtered, and added with silica gel to prepare a sample. The sample was purified by column chromatography (PE:EA=0-100%) to obtain 2-(3,5-dichloro-4-((6-oxo-1-phenyl-1,6-dihydropyridin-3-yl)oxy)phenyl)-6-(hydroxymethyl)-1,2, 4-triazine-3,5(2H,4H)-dione 35b (0.83 g, red solid) with a yield of 74.83%. MS m/z (ESI): 472.95 [M+1]⁺.

### The third step

### 2-(3,5-Dichloro-4-((6-oxo-1-phenyl-1,6-dihydropyridin-3-yl)oxy)phenyl)-6-(fluorometh yl)-1,2,4-triazine-3,5(2H,4H)-dione

### 2-(3,5-Dichloro-4-((6-oxo-1-phenyl-1,6-dihydropyridin-3-yl)oxy)phenyl)-6-methyl-1,2, 4-triazine-3,5(2H,4H)-dione

2-(3,5-Dichloro-4-((6-oxo-1-phenyl-1,6-dihydropyridin-3-yl)oxy)phenyl)-6-(hydroxym ethyl)-1,2,4-triazine-3,5(2H,4H)-dione 35b (680 mg, 1.18 mmol) was added to 10 mL of dichloromethane, and then slowly dropwise added with DAST (380.4 mg, 2.36 mmol). After the dropwise addition was completed, the mixture was reacted at 20°C for 10 min. The reaction was quenched with water. Then the reaction solution was extracted three times. The organic phase was dried over anhydrous sodium sulfate, filtered, dried by rotating and subjected to Prep-HPLC (column model: Welch Ultimate XB-C18, 21.2*250mm, 10um, mobile phase: A: 0.05% TFA aqueous solution, B: Acetonitrile) to obtain 2-(3,5-dichloro-4-((6-oxo-1-phenyl-1,6-dihydropyridin-3-yl)oxy)phenyl)-6-(fluoromethyl)-1,2,4-triazine-3,5(2H,4H)-dione 35 (142.4 mg, white solid, yield: 25.19%) MS m /z(ESI): 474.95[M+1]⁺. ¹H NMR (400 MHz, DMSO-d6) *δ* 12.66 (s, 1H), 7.81 (s, 2H), 7.55 - 7.46 (m, 3H), 7.45 - 7.40 (m, 1H), 7.39 - 7.33 (m, 3H), 6.54 (d, *J* = 10.0 Hz, 1H), 5.28 (d, *J* = 46.8 Hz, 2H).

Meanwhile, 2-(3,5-dichloro-4-((6-oxo-1-phenyl-1,6-dihydropyridin-3-yl)oxy)phenyl)-6-methyl-1,2,4-triazine-3,5(2H,4H)-dione 36 (26.8 mg, white solid) was also obtained. MS m/z (ESI): 457.27 [M+1]⁺. ¹H NMR (400 MHz, DMSO-d6) *δ* 7.80 (s, 2H), 7.62 - 7.23 (m, 7H), 6.54 (d, *J* = 10.0 Hz, 1H), 2.15 (s, 3H).

### Example 37

### 2-(3,5-Dichloro-4-((1-(4-fluorophenyl)-6-oxo-1,6-dihydropyridin-3-yl)oxy)phenyl)-6-(f luoromethyl)-1,2,4-triazine-3,5(2H,4H)-dione

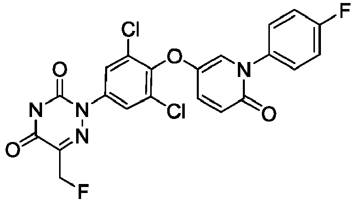

The title product 2-(3,5-dichloro-4-((1-(4-fluorophenyl)-6-oxo-1,6-dihydropyridin-3-yl)oxy)phenyl)-6-(fluoromethyl)-1,2,4-triazine-3,5(2H,4H)-dione 37 (164.0 mg, white solid, yield: 34.82%) was prepared using the method of Example 35 except that 21 was used as the starting material. MS m/z (ESI): 493.25 [M+1]⁺. ¹H NMR (400 MHz, DMSO-d6) *δ* 12.67 (s, 1H), 7.80 (s, 2H), 7.51 (dd, *J* = 10.0, 4.0 Hz, 1H), 7.46 - 7.38 (m, 3H), 7.36 - 7.26 (m, 2H), 6.54 (d, *J* = 10.0 Hz, 1H), 5.28 (d, *J* = 46.8 Hz, 2H).

### Example 38

### 2-(3,5-Dichloro-4-((1-(3-fluorophenyl)-6-oxo-1,6-dihydropyridin-3-yl)oxy)phenyl)-6-(f luoromethyl)-1,2,4-triazine-3,5(2H,4H)-dione

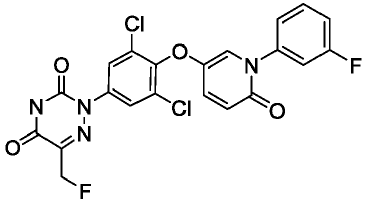

The title product 2-(3,5-dichloro-4-((1-(3-fluorophenyl)-6-oxo-1,6-dihydropyridin-3-yl)oxy)phenyl)-6-(fluoromethyl)-1,2,4-triazine-3,5(2H,4H)-dione 38 (20.0 mg, white solid, yield 26.6%) was prepared using the method of Example 35 except that 21 was used as the starting material. MS m/z (ESI): 493.05 [M+1]⁺. ¹H NMR (400 MHz, DMSO-d6) *δ* 12.67 (s, 1H), 7.80 (s, 2H), 7.68-7.45 (m, 3H), 7.36-7.28 (m, 2H), 7.25-7.19 (m, 1H), 6.55 (d, *J =* 8.0 Hz, 1H), 5.26 (d, *J* = 48.0 Hz, 2H).

### Example 39

### 2-(3,5-Dichloro-4-((6-oxo-1-(3-(trifluoromethyl)phenyl)-1,6-dihydropyridin-3-yl)oxy)p henyl)-6-(fluoromethyl)-1,2,4-triazine-3,5(2H,4H)-dione

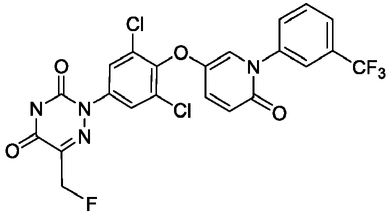

The title product 2-(3,5-dchloro-4-((6-oxo-1-(3-(trifluoromethyl)phenyl)-1,6-dihydropyridin-3-yl)oxy)phenyl)-6-(f luoromethyl)-1,2,4-triazine-3,5(2H,4H)-dione 39 (4.0 mg, white solid, yield: 96.6%) was prepared using the method of Example 35 except that 23 was used as the starting material. MS m/z (ESI): 543.02[M+1]⁺. ¹H NMR (400 MHz, DMSO-d6) *δ* 12.72(s, 1H), 7.89-7.79 (m, 4H), 7.77-7.71 (m, 1H), 7.70-7.66 (m, 1H), 7.59 (d, *J* = 4.0 Hz, 1H), 7.52(dd, *J* = 4.0,4.0 Hz ,1H), 6.56 (d, *J* = 12.0 Hz, 1H), 5.28 (d, *J* = 48.0 Hz,2H).

### Example 40

### 2-(3,5-Dichloro-4-((6-oxo-1-(3-(trifluoromethoxy)phenyl)-1,6-dihydropyridin-3-yl)oxy) phenyl)-6-(fluoromethyl)-1,2,4-triazine-3,5(2H,4H)-dione

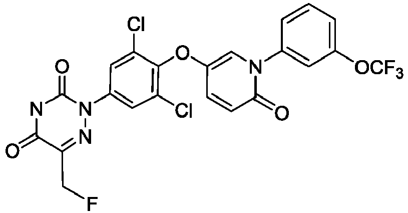

The title product 2-(3,5-dichloro-4-((6-oxo-1-(3-(trifluoromethoxy)phenyl)-1,6-dihydropyridin-3-yl)oxy)phenyl)-6 -(fluoromethyl)-1,2,4-triazine-3,5(2H,4H)-dione 40 (8.0 mg, white solid, yield: 50.6%) was prepared using the method of Example 35 except that 24 was used as the starting material. MS m/z (ESI): 559.00[M+1]⁺. ¹H NMR (400 MHz, DMSO-d6) *δ* 7.81 (s, 2H), 7.52-7.47 (m, 1H), 7.45-7.36 (m, 5H), 6.55 (d, *J* = 8.8 Hz, 1H), 5.27 (d, *J* = 48.0 Hz, 2H).

### Example 41

### 2-(3,5-Dichloro-4-((6-oxo-1-(p-tolyl)-1,6-dihydropyridin-3-yl)oxy)phenyl)-6-(fluorome thyl)-1,2,4-triazine-3,5(2H,4H)-dione

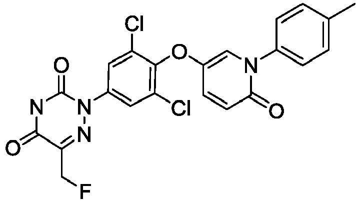

The title product 2-(3,5-dichloro-4-((6-oxo-1-(p-tolyl)-1,6-dihydropyridin-3-yl)oxy)phenyl)-6-(fluoromethyl)-1,2,4-triazine-3,5(2H,4H)-dione 41 (8.5 mg, pale yellow solid , yield 3.0%) was prepared using the method of Example 35 except that 25 was used as the starting material. MS m/z (ESI): 489.00 [M+1]⁺. ¹H NMR (400 MHz, DMSO-d6) *δ* 7.81 (s, 2H), 7.51-7.45 (m, 1H), 7.41-7.17 (m, 5H), 6.53 (d, *J* = 8.0 Hz, 1H), 5.27 (d, *J* = 48.0 Hz, 2H), 2.34 (s, 3H).

### Example 42

### 2-(3,5-Dichloro-4-((1-(3,5-dimethylphenyl)-6-oxo-1,6-dihydropyridin-3-yl)oxy)phenyl) -6-(fluoromethyl)-1,2,4-triazine-3,5(2H,4H)-dione

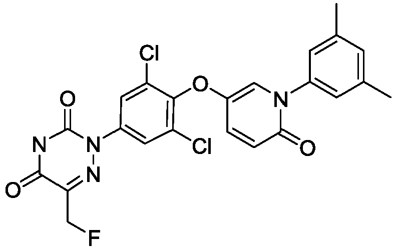

The title product 2-(3,5-dichloro-4-((1-(3,5-dimethylphenyl)-6-oxo-1,6-dihydropyridin-3-yl)oxy)phenyl)-6-(fluoromethyl)-1,2,4-triazine-3,5(2H,4H)-dione 42 (280.00 mg, white solid, yield: 44.9%) was prepared using the method of Example 35 except that 30 was used as the starting material. MS m/z (ESI): 503.31 [M+1]⁺. ¹H NMR (400 MHz, DMSO-d6) *δ* 12.68 (s, 1H), 7.81 (s, 2H), 7.47 (dd, *J =* 10.0, 3.6 Hz, 1H), 7.32 (d, *J* = 3.6 Hz, 1H), 7.06 (s, 1H), 6.95 (s, 2H), 6.52 (d, *J* = 10.0 Hz, 1H), 5.29 (d, *J* = 46.8 Hz, 2H), 2.30 (s, 6H).

### Example 43

### 2-(3,5-Dichloro-4-((6-oxo-1-(thiophen-2-yl)-1,6-dihydropyridin-3-yl)oxy)phenyl)-6-(fl uoromethyl)-1,2,4-triazine-3,5(2H,4H)-dione

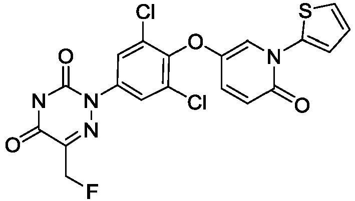

The title product 2-(3,5-dichloro-4-((6-oxo-1-(thiophen-2-yl)-1,6-dihydropyridin-3-yl)oxy)phenyl)-6-(fluoromethyl)-1,2,4-triazine-3,5(2H,4H)-dione 43 (1.98 mg, white solid, yield 3.9%) was prepared using the method of Example 35 except that 33 was used as the starting material. MS m/z (ESI): 480.95 [M+1]⁺. ¹H NMR (400 MHz, DMSO-d6) *δ* 12.69 (s, 1H), 7.93 (d, *J* = 4.0 Hz, 1H), 7.83 (s, 2H), 7.49-7.35 (m, 3H), 7.03 (s, 1H), 6.64 (d, *J =* 12.0 Hz, 1H), 5.29 (d, *J* = 36.0 Hz, 2H).

### Example 44

### 2-(3,5-Dichloro-4-((1-isopropyl-6-oxo-1,6-dihydropyridin-3-yl)oxy)phenyl)-6-(fluorom ethyl)-1,2,4-triazine-3,5(2H,4H)-dione

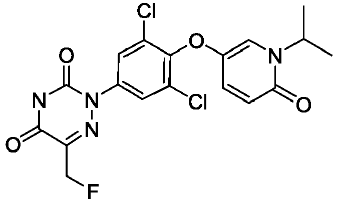

The title product 2-(3,5-dichloro-4-((1-isopropyl-6-oxo-1,6-dihydropyridin-3-yl)oxy)phenyl)-6-(fluoromethyl)-1,2,4-triazine-3,5(2H,4H)-dione 44 (150.0 mg, white solid, yield 35.0%) was prepared using the method of Example 35 except that 1 was used as the starting material. MS m/z (ESI): 441.00 [M+1]⁺. ¹H NMR (400 MHz, DMSO-d6) *δ* 12.68 (s, 1H), 8.10 (s, 2H), 7.51 (d, *J* = 4.0 Hz, 1H), 7.24-7.17 (m, 1H), 6.41-6.32 (m, 1H), 5.99 (d, *J* = 40.0 Hz, 2H), 5.04-4.98 (m, 1H), 1.25 (d, *J* = 4.0 Hz, 6H).

### Example 45

### 2-(3,5-Dichloro-4-((1-(2-methoxyethyl)-6-oxo-1,6-dihydropyridin-3-yl)oxy)phenyl)-6-( fluoromethyl)-1,2,4-triazine-3,5(2H,4H)-dione

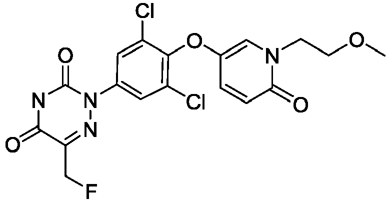

The title product 2-(3,5-dichloro-4-((1-(2-methoxyethyl)-6-oxo-1,6-dihydropyridin-3-yl)oxy)phenyl)-6-(fluoromet hyl)-1,2,4-triazine-3,5(2H,4H)-dione 45 (114.0 mg , white solid, yield 46.0%) was prepared using the method of Example 35 except that 8 was used as the starting material. MS m/z (ESI): 467.00 [M+1]⁺. ¹H NMR (400 MHz, DMSO-d6) *δ* 12.68 (s, 1H), 7.82 (s, 2H), 7.42-7.40 (m, 1H), 7.30 (s, 1H), 6.43 (d, *J* = 8.0 Hz, 1H), 5.29 (d, *J* = 44.0 Hz, 2H), 4.00 (t, *J* = 8.0 Hz, 2H), 3.49 (t, *J* = 8.0 Hz, 2H), 3.19(s, 3H).

### Example 46

### 2-(3,5-Dichloro-4-((3-methyl-5-oxo-1,2,3,5-tetrahydroindoline-8-yl)oxy)phenyl)-3,5-di oxo-2,3,4,5-tetrahydro-1,2,4-triazine-6-carbonitrile

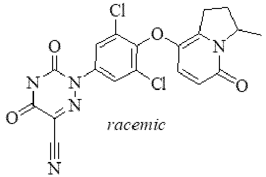

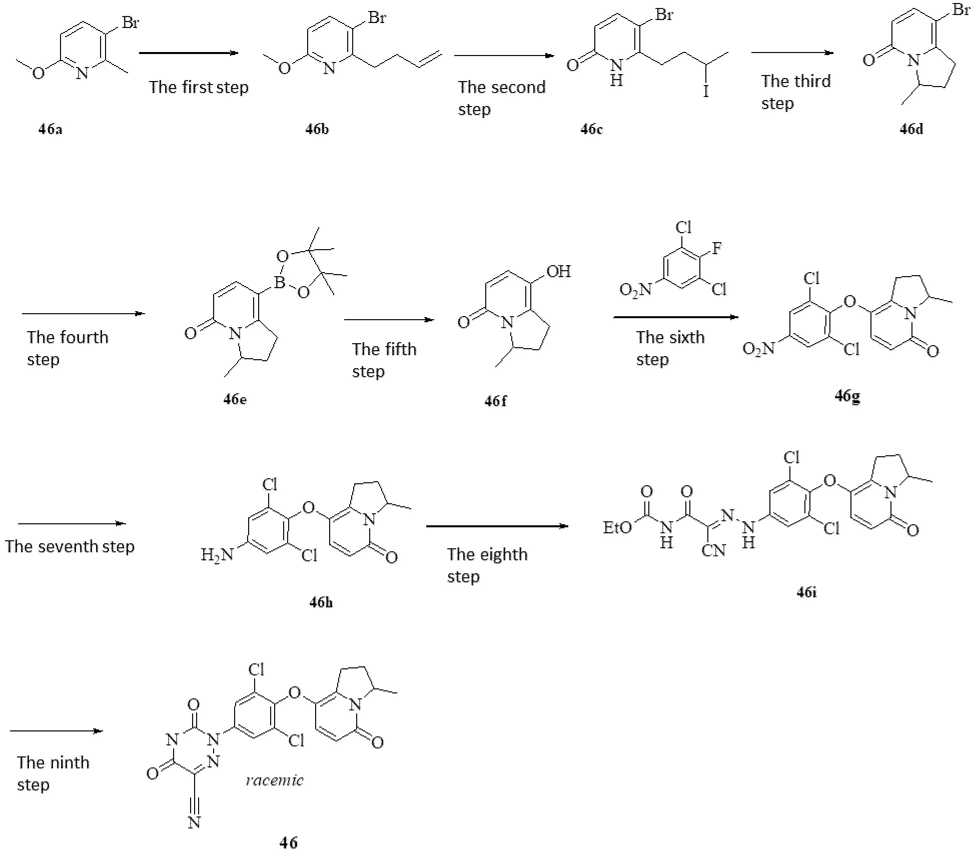

### The first step

### 3-Bromo-2-(3-buten-1-yl)-6-methoxypyridine

3-Bromo-6-methoxy-2-methylpyridine 46a (5.0 g, 24.75 mmol) was dissolved in anhydrous tetrahydrofuran (100 mL), and dropwise added with diisopropylamide lithium (27.23 mL, 54.45 mmol) under nitrogen protection at -78°C. The mixture was reacted at -50°C for 40 minutes. Then the reaction mixture was added with 3-bromopropene (6.587 g, 54.45 mmol) for 30 min of reaction at -50°C, then naturally warmed up to room temperature for 16 h of reaction. The reaction was quenched with 3 mL of saturated aqueous solution of ammonium chloride. The reaction solution was dried over anhydrous sodium sulfate, filtered, concentrated, and purified by column chromatography to obtain 3-bromo-2-(3-buten-1-yl)-6-methoxypyridine 46b (3.79 g, colorless oil) with a yield of 53.43%. MS m/z (ESI): 242.01 [M+1]⁺.

### The second step

### 5-Bromo-6-(3-iodobutyl)pyridin-2(1H)-one

3-Bromo-2-(3-buten-1-yl)-6-methoxypyridine 46b (2.38 g, 9.83 mmol) was dissolved in hydroiodic acid (20.0 mL) for 2 h of reaction at 65°C. Then the reaction solution was added with 100 mL of water, extracted with ethyl acetate (100 mL*3), and washed once with saturated brine. The organic phase was dried over anhydrous sodium sulfate, filtered, and concentrated to obtain 5-bromo-6-(3-iodobutyl)pyridin-2(1H)-one 46c (3.33 g, black oil) with a yield of 95.14 %. MS m/z (ESI): 355.91 [M+1]⁺.

### The third step

### 8-Bromo-3-methyl-2,3-dihydroindoline-5(1H)-one

5-Bromo-6-(3-iodobutyl)pyridin-2(1H)-one 46c (3.33 g, 9.35 mmol) was dissolved in 60.0 mL of ethanol, and added with potassium hydroxide (1.57 g, 28.05 mmol) for 30 min of reaction at 25°C. The reaction solution was concentrated under reduced pressure to remove the solvent. The residue was purified by column chromatography to obtain 8-bromo-3-methyl-2,3-dihydroindoline-5(1H)-one 46d (1.40 g, white solid) with a yield of 65.65%. MS m/z (ESI): 227.99 [M+1]⁺.

### The fourth step

### 3-Methyl-8-(4,4,5,5-tetramethyl-1,3,2-dioxapentan-2-yl)-2,3-dihydroindoline-5(1H)-on e

Dihydroindoline-5(1H)-one 46d (1.18 g, 5.17 mmol) was dissolved in 1,4-dioxane (24.0 mL), and added with pinacol borate (2.63 g, 10.34 mmol), potassium acetate (1.01 g, 10.34 mmol), and 1,1'-bisdiphenylphosphinoferrocene palladium dichloride (378 .0 mg, 0.52 mmol). The mixture was replaced with nitrogen three times, refluxed and reacted for 16 hours, and concentrated under reduced pressure to remove the solvent. The residue was purified by column chromatography to obtain 3-methyl-8-(4,4,5,5-tetramethyl-1,3,2-dioxapentan-2-yl)-2,3-dihydroindoline-5(1H)-one 46e (550.0 mg, white solid) with a yield of 38.6%. MS m/z (ESI): 276.17 [M+1]⁺.

### The fifth step

### 8-Hydroxy-3-methyl-2,3-dihydroindoline-5(1H)-one

3-Methyl-8-(4,4,5,5-tetramethyl-1,3,2-dioxapentan-2-yl)-2,3-dihydroindoline-5(1H)-on e 46e (550.0 mg, 2.0 mmol) was dissolved in a mixed solvent of 12.0 mL of tetrahydrofuran and 2.0 mL of water, and added with 30% hydrogen peroxide (680.0 mg, 6.0 mmol) for 16 h of reaction at 25°C. The reaction mixture was added with ethyl acetate (30 mL), dried over anhydrous sodium sulfate, filtered, concentrated, and purified by column chromatography to obtain 8-hydroxy-3-methyl-2,3-dihydroindoline-5(1H)-one 46f (280.0 mg, white solid) with a yield of 84.75%. MS m/z (ESI): 166.08 [M+1]⁺.

### The sixth step

### 8-(2,6-Dichloro-4-nitrophenoxy)-3-methyl-2,3-dihydroindoline-5(1H)-one

8-Hydroxy-3-methyl-2,3-dihydroindoline-5(1H)-one 46f (92.5 mg, 0.56 mmol) was dissolved in 2.0 mL of N,N-dimethylformamide, and added with 1,3-dichloro-2-fluoro-5-nitrobenzene (117.6 mg, 0.56 mmol) and potassium carbonate (232.2 mg, 1.68 mmol) for 2 h of reaction at 25°C. The reaction solution was concentrated under reduced pressure to remove solvent, and purified by column chromatography to obtain 8-(2,6-dichloro-4-nitrophenoxy)-3-methyl-2,3-dihydroindoline-5(1H)-one 46g (100.0 mg, white solid) with a yield of 50.28%. MS m/z (ESI): 355.02 [M+1]⁺.

### The seventh step

### 8-(4-Amino-2,6-dichlorophenoxy)-3-methyl-2,3-dihydroindoline-5(1H)-one

8-(2,6-Dichloro-4-nitrophenoxy)-3-methyl-2,3-dihydroindoline-5(1H)-one 46g (480 mg, 1.35 mmol) was dissolved in a mixed solvent of 10 mL of ethanol and 10 mL of saturated ammonium chloride, and added with reduced iron powder (377 mg, 6.75 mmol). The mixture was refluxed and reacted for 2 h. The reaction mixture was then filtered, concentrated to remove ethanol, and extracted with dichloromethane (30 mL*3). The organic phase was dried over anhydrous sodium sulfate, filtered, concentrated, and purified by column chromatography to obtain 8-(4-amino-2,6-dichlorophenoxy)-3-methyl-2,3-dihydroindoline-5(1H)-one 46h (220.0 mg, colorless oil) with a yield of 50.0%. MS m/z (ESI): 325.04 [M+1]⁺.

### The eighth step

### (E)-Ethyl(2-cyano-2-(2-(3,5-dichloro-4-((3-methyl-5-oxo-1,2,3,5-tetrahydroindoline-8-yl)oxy)phenyl)hydrazone)acetyl)carbamate

8-(4-Amino-2,6-dichlorophenoxy)-3-methyl-2,3-dihydroindoline-5(1H)-one 46h (150.0 mg, 0.46 mmol) was dissolved in a mixed solvent consisting of 3.2 mL of acetic acid, 0.8 mL of water and 0.4 mL of concentrated hydrochloric acid, and added with sodium nitrite (33.3 mg, 0.48 mmol) at 0°C for 10 min of reaction. The reaction solution was added with sodium acetate (113.2.0 mg, 1.38 mmol) for 10 min of reaction. Then the mixture was added with N-cyanoacetylurethane (143.6 mg, 0.92 mmol), and warmed up to room temperature for 72 h of reaction. The reaction mixture was added with 5 mL of water, filtered, and dried to obtain a crude product (E)-ethyl(2-cyano-2-(2-(3,5-dichloro-4-((3-methyl-5-oxo-1,2,3,5-tetrahydroindoline -8-yl)oxy)phenyl)hydrazone)acetyl)carbamate 46i (180.0 mg, yellow solid) with a yield of 79.5%. MS m/z (ESI): 492.08 [M+1]⁺.

### The ninth step

### 2-(3,5-Dichloro-4-((3-methyl-5-oxo-1,2,3,5-tetrahydroindoline-8-yl)oxy)phenyl)-3,5-di oxo-2,3,4,5-tetrahydro-1,2,4-triazine-6-carbonitrile

(E)-Ethyl(2-cyano-2-(2-(3,5-dichloro-4-((3-methyl-5-oxo-1,2,3,5-tetrahydroindoline-8-yl)oxy)phenyl)hydrazone)acetyl)carbamate 46i (180.0 mg, 0.37 mmol) was dissolved in 3.0 mL of N,N-dimethylacetamide, and added with potassium acetate (108.9 mg, 1.11 mmol) for 2 h of reaction at 120°C. The reaction solution was concentrated under reduced pressure to remove the solvent, and purified by preparative chromatography to obtain 2-(3,5-dichloro-4-((3-methyl-5-oxo-1,2,3,5-tetrahydroindoline-8-yl)oxy)phenyl)-3,5-dioxo-2,3,4, 5-tetrahydro-1,2,4-triazine-6-carbonitrile 46 (60.0 mg, white solid) with a yield of 35.3%. MS m/z (ESI): 446.03 [M+1]⁺. ¹H NMR (400 MHz, DMSO-d6) *δ* 13.26(s, 1H), 7.77 (s, 2H), 7.02 (d, *J* = 12.0 Hz,1H), 6.13 (d, *J* = 8.0 Hz,1H), 4.72-4.68 (m, 1H), 3.26-3.11(m, 2H), 2.37-2.32 (m, 1H), 1.91-1.88(m, 1H), 1.32(d, *J* = 8.0 Hz,2H).

### Example 47 THPβ binding assay

Experimental methods: *In vitro* analysis of the agonistic effect of compounds on THPβ was performed using time-resolved fluorescence resonance energy transfer coactivator peptide recruitment assays. The assay used biotin-SRC2-2 coactivator peptide, streptavidin-d2, RXRα and THRβ-LBD Eu-anti-GST antibody with GST tag. Eu-anti-GST antibody indirectly labeled THRβ-LBD by binding to the GST tag. Streptavidin-d2 indirectly labeled the SRC2-2 coactivator peptide by binding to the biotin tag. In the presence of RXRα, THRβ-LBD can form heterodimer THRβ-LBD/RXRα with it. The binding of agonists with THRβ-LBD/RXRα resulted in a conformational change in THRβ-LBD, thereby increasing the recruitment capacity of the heterodimer to the SRC2-2 coactivator peptide. Meanwhile, it resulted in decreased distance between the d2-labeled SRC2-2 coactivator peptide and the Eu-anti-GST antibody, which increased the THR-FRET signal. According to the effect of different concentrations of the compound on the activity of THRβ, the agonistic ability of the compound can be evaluated.

The detailed procedure is as follows:
a. 100X reference compound or compound was prepared with DMSO, and equally diluted by 1:3.
b. 100X gradiently diluted reference compound or compound was diluted with 1X reaction buffer to 4X, which was then added to an assay plate.
c. A mixed solution of 4X THRβ-LBD and 4X RXRα was prepared with 1X reaction buffer, and added to the assay plate.
d. A mixed solution of 2X biotin-SRC2-2, 2X Eu-anti-GST, and 2X streptavidin-d2 was prepared with 1X reaction buffer, and added to the assay plate.
e. The assay plate was centrifuged at 1000 rpm for 1 min, and incubated at room temperature in the dark for 4 hours.
f. The fluorescence signal values at 665 nm and 615 nm were read on EnVision 2104 plate reader, and Ratio_{665 nm/615 nm} was calculated.

Experimental results: see Table 1.

**Table 1: Results of THRβ binding assay**

| Number of example | EC₅₀(nM) | Number of example | EC₅₀(nM) |
|---|---|---|---|
| 1 | 11 | 25 | NA |
| 2 | 3647 | 26 | 60 |
| 3 | 23 | 27 | 136 |
| 4 | 561 | 28 | 23 |
| 5 | 20 | 29 | 297 |
| 6 | 100 | 30 | 150 |
| 7 | 40 | 31 | 81 |
| 8 | 431 | 32 | 100 |
| 9 | 12 | 33 | 119 |
| 10 | 107 | 34 | 435 |
| 11 | 17 | 35 | 6 |
| 12 | 18 | 36 | 18 |
| 13 | 22 | 37 | 92 |
| 14 | 30 | 38 | 3 |
| 15 | 793 | 39 | 5 |
| 16 | 370 | 40 | 4 |
| 17 | 450 | 41 | 117 |
| 18 | 1430 | 42 | 9 |
| 19 | 1340 | 43 | 8 |
| 20 | 88 | 44 | 3 |
| 21 | NA | 45 | 32 |
| 22 | 20 | 46 | 102 |
| 23 | 26 | | |
| 24 | 23 | | |
| *Control compound 1 | 0.6 | | |
| * Control compound 2 | 204 | | |

| | | | |
|---|---|---|---|
| *The control compound 1 is T3; the control compound 2 is Example 8 (compound 31) in WO2007009913, NA represents not detected. | | | |

### Example 48 THRα binding assay

Experimental method: *In vitro* analysis of the agonistic effect of the compound on THRα was conducted using a method similar to that of the THPβ binding assay in Example 49, except that THPβ was replaced with THRα.

Experimental results: see Table 2.

**Table 2: Results of THRα binding assay**

| Number of example | EC₅₀(nM) | THRα/β selectivity (fold) |
|---|---|---|
| 1 | 49 | 4.5 |
| 3 | 290 | 12.6 |
| 6 | 2030 | 20.3 |
| 11 | 236 | 13.9 |
| 14 | 600 | 20.0 |
| 20 | 4180 | 47.5 |
| 22 | 669 | 33.5 |
| 23 | 2686 | 103.3 |
| 24 | 810 | 35.2 |
| 28 | 1138 | 49.5 |
| 35 | 220 | 36.7 |
| 36 | 351 | 19.5 |
| 37 | 9255 | 100.6 |
| 38 | 85 | 28.3 |
| 39 | 117 | 23.3 |
| 40 | 95 | 23.7 |
| 41 | 7628 | 53.7 |
| 42 | 696 | 72.5 |
| 46 | 930 | 9.1 |
| * Control compound 1 | 0.2 | / |
| * Control compound 2 | 2690 | 13.2 |

| | | |
|---|---|---|
| *The control compound 1 is T3; the control compound 2 is Example 8 (compound 31) in WO2007009913. | | |

Conclusion: Compared with the disclosed control compound 2, some compounds of the present invention unexpectedly showed high activity on THKβ, and some compounds showed higher selectivity for THKβ than the control compound 2.

### Example 49 In vitro liver microsome stability assay

### Experimental method:

### (1) Preparation of solution

The test substance and the positive control substance verapamil were respectively dissolved with DMSO to 10 mM as a stock solution. The above 10 mM stock solution was diluted with 70% acetonitrile aqueous solution to a concentration of 0.25 mM.

An NADPH regeneration system finally containing concentrations of 6.5 mM NADP, 16.5 mM G-6-P, 3 U/mL G-6-PDH, and 3.3 mM MgCl was prepared.

Stopping solution was an acetonitrile solution containing tolbutamide and propranolol (both internal standards).

Phosphate buffer was a 100 mM K₃PO₄ (pH=7.4) buffer containing 3.3 mM MgCl₂.

Liver microsomal incubation system was a 100 mM phosphate buffer containing 0.2 mg/mL liver microsomal protein and 1 µM test substance/positive control.

### (2) Incubation process

80 µL of the mixed solution from the incubation system was taken. 400 µL of the stopping solution was added to precipitate protein. The mixture was vortexed and added with 20 µL of NADPH regeneration system as the sample at 0 min.

130 µL of NADPH regeneration system was added to the remaining 520 µL of protein-drug mixed solution. The mixture was mixed well, and incubated. The final incubation system was 650 µL, containing 0.2 mg/mL liver microsomal protein, 1 µM test substance/positive control, 1.3 mM NADP, 3.3 mM G-6-P, and 0.6 U/mL G-6-PDH.

The mixed system was slowly shaken and incubated in a water bath at 37°C. 100 µL of the incubation solution was taken at 5, 10, 30, and 60 min, respectively, and added into a new 96-well plate with 400 µL of stopping solution in each well. The mixture in each well was mixed well, and the protein was precipitated by centrifuging at 4000 × g for 15 minutes at 4°C.

100 µL of the supernatant was taken, and diluted with water at a ratio of 1:2. The samples were analyzed by LC-MS/MS.

Experimental results: see Table 3.

**Table 3: Results of in vitro liver microsome stability assay**

| Number of example | Liver microsome stability (species: human): T1/2, min |
|---|---|
| 3 | 62.8 |
| 4 | 110.4 |
| 5 | 2.1 |
| 9 | 7.0 |
| 10 | >120 |
| 11 | 41.6 |
| 12 | 4.4 |
| 14 | 4.6 |
| 15 | 11.5 |
| 16 | 5.1 |
| 17 | 3.2 |
| 20 | >120 |
| 22 | >120 |
| 35 | >120 |

From the above results, it can be seen that most aromatic-substituted pyridone compounds had better stability in liver microsome.

### Example 50 Evaluation on in vivo pharmacokinetics in mice

Mice were used as test animals, and intragastrically administered with the compounds of Example 1 and Example 46, and then the drug concentrations in plasma at different time points were measured. The pharmacokinetic performance of the compounds of the present invention in mice was studied, and the drug metabolism characteristics were evaluated. In the example, healthy adult male ICR mice with similar body weight were selected, and orally administered with drugs once at a dose of 3.0 mg/kg. Whole blood was collected before the administration and 1h, 2h, 4h, 6h, 8h, 12h, 24h and 48h after the administration, respectively. LC/MS/MS method was established to determine the concentrations of prototype drugs in plasma samples. The main pharmacokinetic parameters were calculated using WinNonlin 6.3 NCA model.

### Experimental method:

Experimental drug: the compounds obtained in Example 1 and Example 46.

Experimental animals: 9 healthy adult male ICR mice, equally divided into 3 groups, 3 mice in each group, purchased from Shanghai Xipu'er-Bikai Experimental Animal Co., Ltd., with an animal certificate number: SCXK (Shanghai) 2018-0006 2018 0006 010467.

Preparation of drug: A certain amount of the compounds of Example 1 and Example 46 were accurately weighed, put into a 15 ml sample tube, wetted by 5 µL of Tween 80, and then added with 5.2 mL of 2% hydroxypropyl methylcellulose (HPMC) solution. The mixture was sonicated and mixed by vortex to obtain a homogeneous suspension. The drugs were freshly prepared just before use.

Administration: After fasting overnight, ICR mice were intragastrically administered with the drugs at a dose of 3.0 mg/kg.

Operation: The mice were intragastrically administered with the compounds of Example 1 and Example 46. Whole blood was collected before the administration and 1h, 2h, 4h, 6h, 8h, 12h, 24h and 48h after the administration, then anticoagulated with heparin sodium, and centrifuged at 4°C for 5 min. Plasma was separated, and stored at -80°C for determination.

Determination of the contents of the compounds to be tested in the mice plasma after intragastric administration of drugs: 12.5 µL of the mice plasma to be tested at each time point after the administration of the compounds of Example 1 and Example 46 were taken respectively, mixed in pairs, and added with 100 µL of the internal standard solution and 125 µL of methanol. The mixture was mixed by vortex for 2 min, and centrifuged at 13000 rpm for 10 min at 4°C. The supernatant was taken for LC-MS/MS analysis.

The results of pharmacokinetic parameters are shown in Table 4.

**Table 4: Data of metabolism of drug in mice**

| Compound | Dose (mg/Kg) | Peak plasma concentration (ng/mL) | Area of curve (ng*h/ml) | Half life (h) |
|---|---|---|---|---|
| 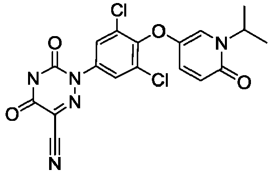 Compound of Example 1 | 3 | 54.0 | 535.2 | 5.6 |
| 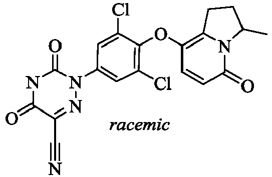 Compound of Example 46 | 3 | 92.37 | 1774.03 | 11.15 |

Conclusion: The compounds of the present invention have good pharmacological activity, especially the compounds with cyclic structure have more superior pharmacological activity, and good pharmacokinetic absorption, showing obvious pharmacokinetic advantages. Under the same dosage and formulation, some compounds of the present invention unexpectedly showed higher Cmax value and exposure amount, and longer half-life. All the above PK results show that the compounds provided by the present invention have good PK properties and can be used as therapeutic drugs for metabolic related diseases. As understood in the art, the above compounds can exert their pharmacological and pharmacokinetic advantages in the preparation of pharmaceutical compositions.

The descriptions of the above examples are only used to help understand the method and core idea of the present invention. It should be noted that for those of ordinary skill in the art, without departing from the principle of the present invention, several improvements and modifications can be made to the present invention, and these improvements and modifications also fall within the protection scope of the claims of the present invention.

## Claims

1. A 2-pyridone derivative having a structure represented by formula I or a pharmaceutically acceptable salt thereof: 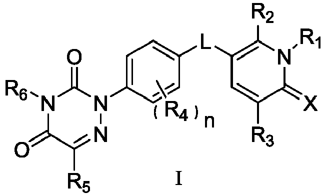
wherein, R₁ is selected from the group consisting of C₁₋₆ alkyl, saturated or unsaturated C₃₋₆ cycloalkyl, C₅₋₁₀ aryl and C₅₋₁₀ heteroaryl; wherein, the C₁₋₆ alkyl can be optionally substituted with one or more of halogen, deuterium, hydroxyl, alkoxy, oxo, amino, C₃₋₆ cycloalkyl, 5-10 membered aryl or 5-10 membered heteroaryl; the saturated or unsaturated C₃₋₆ cycloalkyl can be optionally substituted with one or more of halogen, deuterium, hydroxyl, alkoxy, oxo or amino; the C₅₋₁₀ aryl, C₅₋₁₀ heteroaryl, 5-10 membered aryl or 5-10 membered heteroaryl can be optionally substituted with one or more of halogen, substituted or unsubstituted C₁₋₆ alkyl, substituted or unsubstituted C₃₋₆ cycloalkyl, substituted or unsubstituted C₁₋₆ alkoxy, cyano, hydroxyl, or amino;
R₂ is selected from the group consisting of hydrogen and C₁₋₆ alkyl;
R₃ is selected from the group consisting of hydrogen, halogen, C₁₋₆ alkyl and C₃₋₆ cycloalkyl;
R₄ is independently selected from the group consisting of hydrogen, hydroxyl, C₁₋₆ alkyl, halogen, C₁₋₆ alkoxy, and C₃₋₆ cycloalkyl, or two adjacent R₄ form C₃₋₆ cycloalkyl, wherein the C₁₋₆ alkyl, C₃₋₆ cycloalkyl or C₁₋₆ alkoxy can be optionally further substituted with one or more of hydroxyl or halogen;
n is 1, 2 or 3;
R₅ is selected from the group consisting of hydrogen, cyano, carboxyl, C₁₋₆ alkyl, and C₃₋₆ cycloalkyl, wherein the C₁₋₆ alkyl or C₃₋₆ cycloalkyl can be optionally substituted with one or more of halogen, hydroxyl, or C₁₋₆ alkoxy;
R₆ is selected from the group consisting of hydrogen and C₁₋₆ alkyl;
L is selected from the group consisting of -CH₂-, -O-, -CF₂- and -S-;
X is selected from the group consisting of O and S;
or alternatively, R₁ and R₂ together with the atoms to which they are attached form a 4-10 membered heterocycloalkyl, wherein the heterocycloalkyl can further contains 0, 1 or 2 optional oxygen, sulfur and nitrogen atoms in addition to the original nitrogen atom connected to R₁, and the heterocycloalkyl can be optionally further substituted with one or more of C₁₋C₆ alkyl, hydroxyl, halogen or cycloalkyl;
when R₃ is C₁₋₆ alkyl and/or C₃₋C₆ cycloalkyl, R₁ is not C₁₋₆ alkyl, saturated or unsaturated C₃₋₆ cycloalkyl.

2. The 2-pyridone derivative according to claim 1, wherein R₁ is selected from the group consisting of methyl, ethyl, n-propyl, isopropyl, n-butyl, isobutyl, tert-butyl, n-pentyl, isopentyl, neopentyl, -CH(CH₂CH₃)₂, cyclopropyl, cyclobutyl, cyclopentyl, cyclohexyl, cyclopentenyl, cyclohexenyl, phenyl, thienyl, and thiazolyl;
the methyl, ethyl, n-propyl, isopropyl, n-butyl, isobutyl, tert-butyl, n-pentyl, isopentyl, neopentyl, or -CH(CH₂CH₃) can be optionally substituted with one or more of halogen, deuterium, hydroxyl, C₁₋₆ alkoxy, oxo, amino, C₃₋₆ cycloalkyl, phenyl, naphthyl, pyridyl or pyrrolyl;
the cyclopropyl, cyclobutyl, cyclopentyl, cyclohexyl, cyclopentenyl, or cyclohexenyl can be optionally substituted with one or more of halogen, deuterium, hydroxyl, C₁₋₆ alkoxy, oxo or amino;
the phenyl, thienyl, thiazolyl, naphthyl, pyridyl or pyrrolyl can be optionally substituted with one or more of halogen, substituted or unsubstituted C₁₋₆ alkyl, substituted or unsubstituted C₃₋₆ cycloalkyl, substituted or unsubstituted C₁₋₆ alkoxy, cyano, hydroxyl, or amino.

3. The 2-pyridone derivative according to claim 1, wherein the C₅₋₁₀ aryl, C₅₋₁₀ heteroaryl, 5-10 membered aryl or 5-10 membered heteroaryl can be optionally substituted with one or more of halogen, substituted or unsubstituted C₁₋₆ alkyl, substituted or unsubstituted C₃₋₆ cycloalkyl, substituted or unsubstituted C₁₋₆ alkoxy, or cyano;
the C₁₋₆ alkyl, C₃₋₆ cycloalkyl, or C₁₋₆ alkoxy can be optionally substituted with one or more F atoms.

4. The 2-pyridone derivative according to claim 1, having any one of the structures represented by formula IIa to formula IIc: 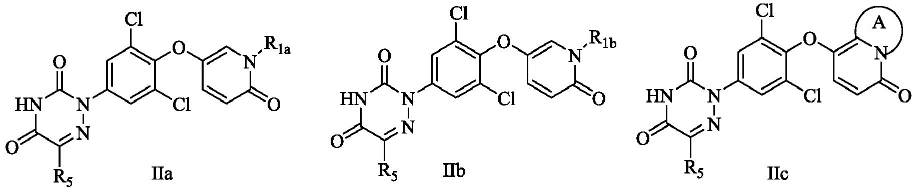
wherein, R₁ₐ is selected from the group consisting of C₁₋₆ alkyl and saturated or unsaturated C₃₋₆ cycloalkyl; wherein the C₁₋₆ alkyl can be optionally further substituted with one or more of halogen, deuterium, hydroxyl, alkoxy, oxo, amino, C₃₋₆ cycloalkyl, 5-10 membered aryl or 5-10 membered heteroaryl; the saturated or unsaturated C₃₋₆ cycloalkyl can be optionally further substituted with one or more of halogen, deuterium, hydroxy, alkoxy, oxo or amino;
the 5-10 membered aryl or 5-10-membered heteroaryl can be optionally substituted with one or more of halogen, substituted or unsubstituted C₁₋₆ alkyl, substituted or unsubstituted C₃₋₆ cycloalkyl, substituted or unsubstituted C₁₋₆ alkoxy, cyano, hydroxyl, or amino;
R_{1b} is selected from the group consisting of C₅₋₁₀ aryl and C₅₋₁₀ heteroaryl, wherein the C₅₋₁₀ aryl or C₅₋₁₀ heteroaryl can be optionally further substituted with one or more of halogen, substituted or unsubstituted C₁₋₆ alkyl, substituted or unsubstituted C₃₋₆ cycloalkyl, substituted or unsubstituted C₁₋₆ alkoxy, cyano, hydroxyl, or amino;
ring A in formula IIc is a 4-10 membered heterocycloalkyl containing attached nitrogen, wherein the 4-10 membered heterocycloalkyl can further contains 0, 1 or 2 optional oxygen, sulfur and nitrogen atoms in addition to the attached nitrogen atom, and the heterocycloalkyl can be optionally further substituted with one or more of C₁-C₆ alkyl, hydroxyl, halogen, or cycloalkyl;
R₅ is selected from the group consisting of hydrogen, cyano, carboxyl, C₁₋₆ alkyl, and C₃₋₆ cycloalkyl, wherein the C₁₋₆ alkyl or C₃₋₆ cycloalkyl can be optionally substituted with one or more of halogen, hydroxyl, or C₁₋₆ alkoxy.

5. The 2-pyridone derivative according to claim 4, wherein the C₅₋₁₀ aryl, C₅₋₁₀ heteroaryl, 5-10 membered aryl or 5-10 membered heteroaryl can be optionally substituted with one or more of halogen, substituted or unsubstituted C₁₋₆ alkyl, substituted or unsubstituted C₃₋₆ cycloalkyl, substituted or unsubstituted C₁₋₆ alkoxy, or cyano;
the C₁₋₆ alkyl, C₃₋₆ cycloalkyl, or C₁₋₆ alkoxy can be optionally substituted with one or more F atoms.

6. The 2-pyridone derivative according to claim 4, wherein in the formula IIc, ring A is selected from the group consisting of a 5-6 membered monocyclic heterocyclyl and a 7-10 membered spiro heterocyclyl.

7. The 2-pyridone derivative according to claim 6, wherein in the formula IIc, ring A is a five-membered or six-membered monocyclic heterocyclyl;
the five-membered or six-membered monocyclic heterocyclyl can be optionally substituted with one or more of halogen, C₁₋₃ alkyl or C₃₋₆ cycloalkyl.

8. The 2-pyridone derivative according to claim 4, wherein R₁ₐ is selected from the group consisting of methyl, ethyl, n-propyl, isopropyl, n-butyl, isobutyl, tert-butyl, n-pentyl, isopentyl, neopentyl, -CH(CH₂CH₃)₂, cyclopropyl, cyclobutyl, cyclopentyl, cyclohexyl, cyclopentenyl, and cyclohexenyl;
the methyl, ethyl, n-propyl, isopropyl, n-butyl, isobutyl, tert-butyl, n-pentyl, isopentyl, neopentyl, or -CH(CH₂CH₃)₂ can be optionally substituted with one or more of halogen, deuterium, hydroxyl, C₁₋₆ alkoxy, oxo, amino, C₃₋₆ cycloalkyl, phenyl, naphthyl, pyridyl, or pyrrolyl;
the cyclopropyl, cyclobutyl, cyclopentyl, cyclohexyl, cyclopentenyl, or cyclohexenyl can be optionally substituted with one or more of halogen, deuterium, hydroxyl, C₁₋₆ alkoxy, oxo or amino;
R_{1b} is selected from the group consisting of phenyl, thienyl and thiazolyl;
the phenyl, thienyl, thiazolyl, naphthyl, pyridyl, or pyrrolyl can be optionally substituted with one or more of halogen, substituted or unsubstituted C₁₋₆ alkyl, substituted or unsubstituted C₃₋₆ cycloalkyl, substituted or unsubstituted C₁₋₆ alkoxy, cyano, hydroxyl, or amino.

9. The 2-pyridone derivative according to claim 1 or 4, wherein R₅ is selected from the group consisting of hydrogen, cyano and C₁₋₆ alkyl;
the C₁₋₆ alkyl can be optionally substituted with one or more of halogen, hydroxyl or amino.

10. The 2-pyridone derivative according to claim 9, wherein R₅ is selected from the group consisting of hydrogen, cyano, methyl, ethyl, n-propyl, isopropyl, n-butyl, isobutyl, tert-butyl, n-pentyl, isopentyl, neopentyl and -CH(CH₂CH₃)₂;
the methyl, ethyl, n-propyl, isopropyl, n-butyl, isobutyl, tert-butyl, n-pentyl, isopentyl, neopentyl or -CH(CH₂CH₃)₂ can be optionally substituted with 1-3 fluorine atoms.

11. The 2-pyridone derivative according to claim 1, wherein R₂ is H;
R₃ is H;
R₄ is selected from the group consisting of ortho-difluoro, ortho-dichloro and ortho-dibromo at the L position;
R₆ is H;
L is -O-;
Xis O.

12. The 2-pyridone derivative according to claim 1, having any one of the following structures:

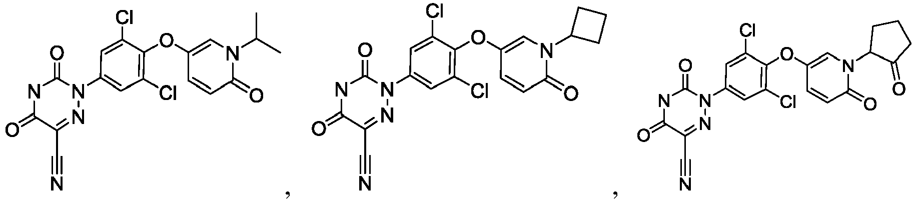

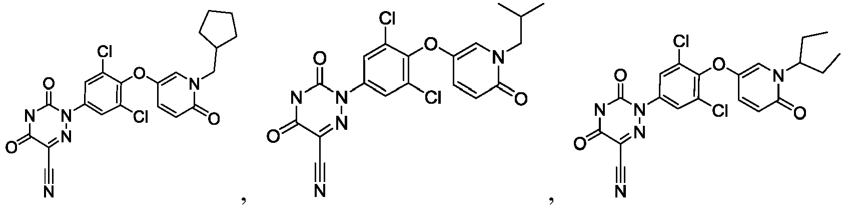

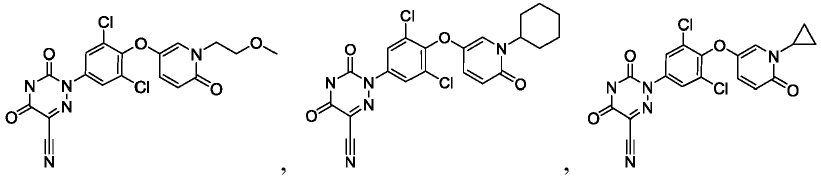

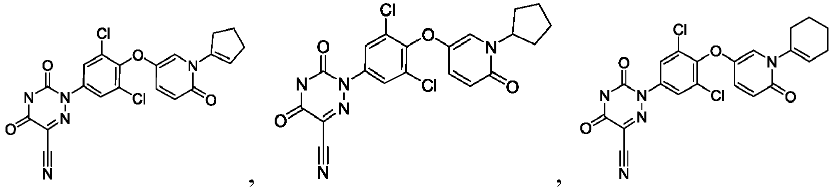

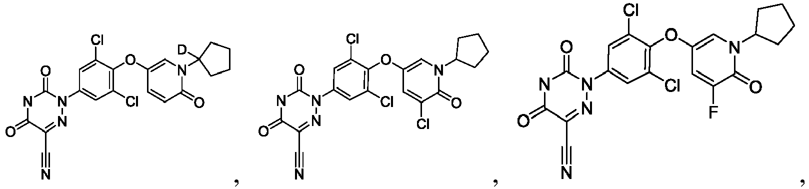

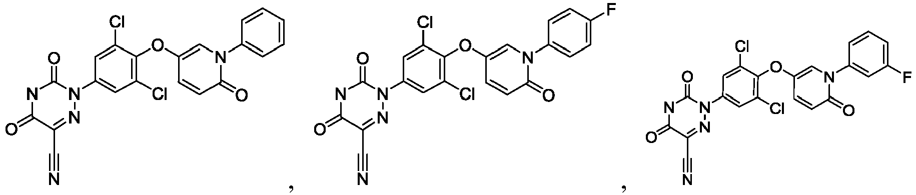

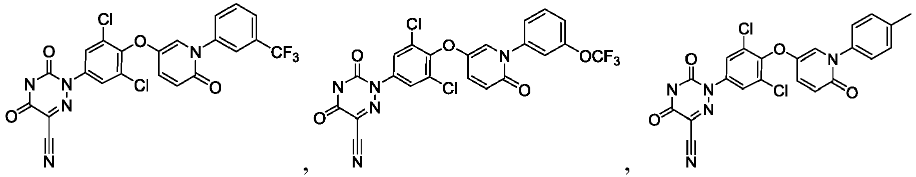

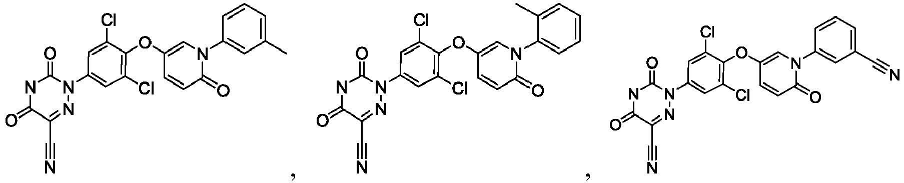

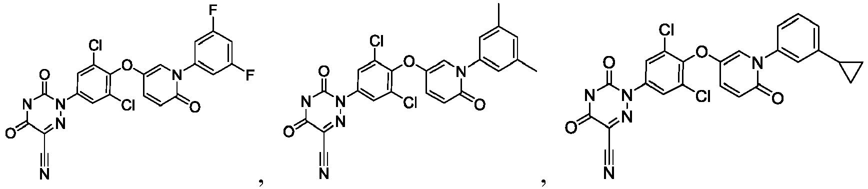

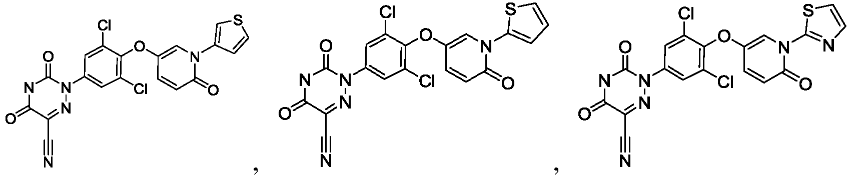

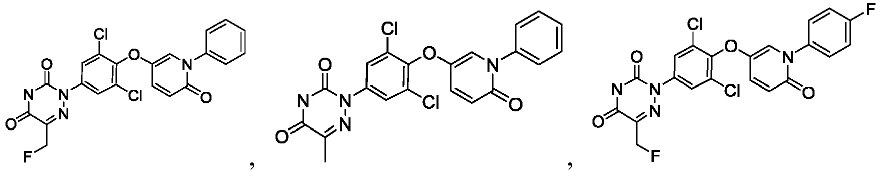

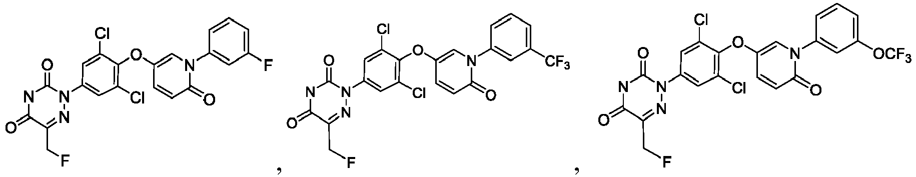

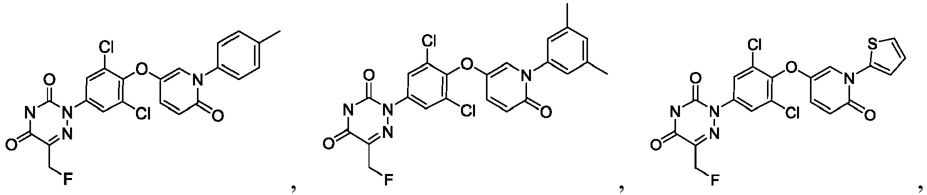

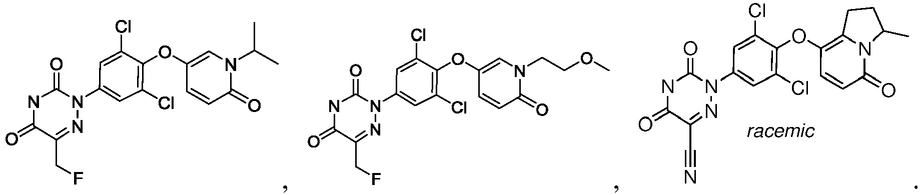

13. Use of the 2-pyridone derivative according to any one of claims 1-12 in the manufacture of a medicament for preventing, treating and/or alleviating a disease caused by the regulation of thyroid hormone analogs.

14. The use according to claim 13, wherein the disease caused by the regulation of thyroid hormone analogs is selected from the group consisting of obesity, hyperlipidemia, hypercholesterolemia, diabetes, non-alcoholic steatohepatitis, atherosclerosis, cardiovascular disease, hypothyroidism, thyroid cancer and a combination thereof.

15. A pharmaceutical preparation, comprising the 2-pyridone derivative according to any one of claims 1-12, and a pharmaceutically acceptable excipient.
